(19) Europäisches Patentamt / European Patent Office / Office européen des brevets

(11) **EP 4 763 867 A1**

(12) **EUROPEAN PATENT APPLICATION**
published in accordance with Art. 153(4) EPC

(43) Date of publication:
**24.06.2026 Bulletin 2026/26**

(21) Application number: **24853855.5**

(22) Date of filing: **15.08.2024**

(51) International Patent Classification (IPC):
**C07K 19/00** (2006.01)   **C07K 16/28** (2006.01)
**C07K 14/55** (2006.01)   **C12N 15/62** (2006.01)
**C12N 15/63** (2006.01)   **A61P 35/00** (2006.01)
**A61K 38/20** (2006.01)   **A61K 39/00** (2006.01)
**A61K 39/395** (2006.01)   **A61K 47/68** (2017.01)

(52) Cooperative Patent Classification (CPC):
**A61K 38/20; A61K 39/00; A61K 39/395;**
**A61K 47/68; A61P 35/00; C07K 14/55;**
**C07K 16/28; C07K 19/00; C12N 15/62; C12N 15/63**

(86) International application number:
**PCT/CN2024/112330**

(87) International publication number:
**WO 2025/036449 (20.02.2025 Gazette 2025/08)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **16.08.2023  CN 202311036103**

(71) Applicant: **Shanghai Mabgen Biotech Ltd.
Shanghai 201206 (CN)**

(72) Inventors:
• **WU, Hui
  Shanghai 201206 (CN)**
• **ZHAO, Zhilong
  Shanghai 201206 (CN)**
• **LI, Lei
  Shanghai 201206 (CN)**

(74) Representative: **Dragotti & Associati S.P.A.
Via Nino Bixio, 7
20129 Milano (MI) (IT)**

Remarks:
The complete document including Reference Table(s) and the Sequence Listing(s) can be downloaded from the EPO website

(54) **PHARMACEUTICAL COMPOSITION CONTAINING IMMUNOCONJUGATE AND USE THEREOF**

(57)    The present invention relates to a pharmaceutical composition containing an immunoconjugate, and the use thereof. Specifically, the present invention relates to a pharmaceutical composition, which contains an immunoconjugate and a buffer.

**EP 4 763 867 A1**

**Description**

The present disclosure claims priority to Chinese Patent Application No. 2023110361039, titled "PHARMACEUTICAL COMPOSITION CONTAINING IMMUNOCONJUGATE AND USE THEREOF" and filed on August 16, 2023, which is incorporated herein by reference in its entirety.

**TECHNICAL FIELD**

The present disclosure pertains to the field of pharmaceutical formulations and particularly relates to a pharmaceutical composition comprising an immunoconjugate and use thereof as a medicament.

**BACKGROUND**

Human interleukin-2 (IL-2), also known as T cell growth factor (TCGF), has a gene located on chromosome 4 (4q27) and comprising a sequence of 7 kb in total, and consists of 133 amino acids with a molecular weight of about 15 kD. Early *in vitro* cell experiments showed that T cells could secrete IL-2 and express the IL-2 receptor (IL-2R) on the cell surface after being activated by TCR and CD28. The binding of IL-2 to its receptor can trigger T cell proliferation and enable T cell response. This model makes IL-2 a molecule that plays a central role in T cell immune responses. However, it was found in subsequent *in vivo* experiments that animals developed autoimmunity after IL-2 or its receptor was knocked out. Subsequent experiments showed that IL-2 could activate not only effector cells such as T cells and NK cells, but also regulatory T cells (Tregs), thereby suppressing excess self-directed autoimmunity.

IL-2 acts through IL-2R on the cell surface. IL-2R includes three subunits: IL-2R$\alpha$ (i.e., CD25), IL-2R$\beta$ (i.e., CD122), and IL-2R$\gamma$ (i.e., CD132). Those three subunits can form three forms of receptors: the high-binding avidity receptor comprising all of the three subunits IL-2R$\alpha/\beta/\gamma$, the medium-binding avidity receptor comprising IL-2R$\beta/\gamma$, and the low-binding avidity receptor being IL-2R$\alpha$. Among them, IL-2R$\beta$ and IL-2R$\gamma$ are necessary for IL-2 to activate downstream signaling pathways, and when IL-2 binds to both IL-2R$\beta$ and IL-2R$\gamma$, those two receptor subunits form a heterodimer that phosphorylates STATS in cells, and enters the cell nucleus to cause corresponding gene transcription and expression; and IL-2R$\alpha$ is not necessary for signaling, but can enhance the binding of IL-2 to IL-2R$\beta$ and IL-2R$\gamma$. Tregs continuously express the high-binding avidity receptor IL-2R$\alpha/\beta/\gamma$, while unactivated CD8$^+$ T cells, NK cells, etc., only express the medium-binding avidity receptor IL-2R$\beta/\gamma$. Therefore, low-dose IL-2 preferentially binds to and activates Tregs, while high-dose IL-2 can activate immune effector cells such as CD8$^+$ T cells and NK cells. Due to its ability to activate immune effector cells, high-dose IL-2 can be used for tumor immunotherapy. However, high-dose IL-2 treatment is accompanied by severe toxic and side effects, including fever, diarrhea, capillary leak syndrome, etc. These side effects are associated with the excessive activation of peripheral T cells and NK cells. By reducing or eliminating the activity of IL-2 on peripheral T cells and NK cells and specifically targeting the tumor microenvironment or tumor-associated immune cells, the anti-tumor effect of IL-2 can be exerted without causing systemic toxicity.

PD-1 (programmed cell death-1), an immunosuppressive receptor, belongs to the CD28 receptor family (Riley et al., 2009, Immunol. Rev. 29:114-25). This family also includes CD28, CTLA-4, ICOS, PD-1, and BTLA. PD-1 is expressed mainly by activated B cells, T cells, and bone marrow cells (Chen et al., 2013, Nat. Rev. Immunol. 13:227-42). There are two cell surface glycoprotein ligands: PD-L1 and PD-L2. PD-L1 is widely expressed by lymphocytes (e.g., CD4$^+$ T cells, CD8$^+$ T cells, and macrophages), peripheral tissues, various tumor cells, virus-infected cells, etc. PD-L2 is expressed mainly by activated dendritic cells and macrophages (Dong et al., 1999, Nat. Med. 5:1365-9). Upon binding to its ligand PD-L1 or PD-L2, PD-1 down-regulates T cell function, including reducing T cell activation, differentiation, proliferation, cytokine secretion, etc.

The present disclosure provides an immunoconjugate comprising a PD-1 antibody and an IL-2 or a variant thereof, wherein the IL-2 variant has a reduced avidity for the high-avidity receptors (IL-2R$\alpha/\beta/\gamma$) and the moderate-avidity receptors (IL-2R$\beta/\gamma$) as compared to wild-type IL-2 and has an eliminated avidity for the low-avidity receptor (IL-2R$\alpha$) as compared to wild-type IL-2, and the avidity for the high-avidity receptors is reduced more than the avidity for the moderate-avidity receptors. Due to its reduced avidity for IL-2R$\alpha/\beta/\gamma$ and IL-2R$\beta/\gamma$ as compared to wild-type IL-2, the immunoconjugate is inactive on immune cells that lowly express or do not express PD-1 but can activate immune cells that highly express PD-1 in two ways. First, the PD-1 antibody blocks the interactions of PD-1/PD-L1 and PD-1/PD-L2, eliminating the immunosuppression of T cells by tumors. Second, the immunoconjugate binds to PD-1 on the T cell surface, and only upon binding to PD-1 can its IL-2 variant bind to IL-2 receptors on the cell surface in an in-cis manner, thereby activating PD-1-positive T cells. This design utilizes the IL-2 variant to enhance the activation and proliferation of T cells by the PD-1 antibody and the anti-tumor effect and also increases the specificity of IL-2 for PD-1-positive immune cells, avoiding the systemic toxicity commonly associated with wild-type IL-2. This provides a more effective solution for clinical cancer treatment.

However, due to its large molecular weight and complex structure, the immunoconjugate is prone to degradation,

EP 4 763 867 A1

aggregation, etc., leading to instability and a reduction in activity or even a loss of effectiveness. It is particularly important to develop stable formulations of the drug that make the fusion protein suitable for administration, keep it stable during storage and subsequent use, and make it produce better effects.

**SUMMARY**

The present disclosure provides a pharmaceutical composition comprising an immunoconjugate (e.g., a fusion protein of a PD-1-binding domain and a cytokine), a method for preparing the pharmaceutical composition, and a method for treating or preventing a disease using same, or related pharmaceutical use thereof.

The present disclosure provides a pharmaceutical composition comprising an immunoconjugate (e.g., a fusion protein of a PD-1-binding domain and a cytokine) and a buffer. In some embodiments, the buffer is a histidine salt buffer. In some embodiments, the buffer is one or more selected from the group consisting of acetic acid-histidine, succinic acid-histidine, and histidine-histidine hydrochloride. The term "one or more" in the present disclosure includes one, two, or more.

In some embodiments, the buffer is at a concentration of about 1 mM to about 100 mM, e.g., about 1 mM to about 50 mM, about 1 mM to about 55 mM, about 1 mM to about 60 mM, about 1 mM to about 70 mM, about 1 mM to about 80 mM, about 5 mM to about 100 mM, about 1 mM to about 80 mM, about 5 mM to about 70 mM, about 5 mM to about 50 mM, about 5 mM to about 45 mM, about 5 mM to about 40 mM, about 5 mM to about 35 mM, about 5 mM to about 30 mM, about 5 mM to about 25 mM, about 5 mM to about 20 mM, about 5 mM to about 10 mM, about 10 mM to about 20 mM, about 10 mM to about 30 mM, about 10 mM to about 35 mM, about 10 mM to about 40 mM, about 10 mM to about 50 mM, about 15 mM to about 20 mM, about 15 mM to about 25 mM, about 15 mM to about 35 mM, about 15 mM to about 50 mM, about 20 mM to about 35 mM, about 1 mM to about 10 mM, about 1 mM to about 20 mM, or any range between these point values. In some embodiments, the buffer is at a concentration of about 5 mM to about 35 mM. In some embodiments, the buffer is at a concentration of about 5 mM to about 20 mM. In some embodiments, the buffer is at a concentration of about 10 mM to about 20 mM.

In some embodiments, the buffer is at a concentration of about 1 mM, about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 22 mM, about 25 mM, about 26 mM, about 28 mM, about 30 mM, about 35 mM, about 40 mM, about 43 mM, about 45 mM, about 48 mM, about 50 mM, about 55 mM, about 57 mM, about 58 mM, about 60 mM, about 62 mM, about 65 mM, about 68 mM, about 70 mM, about 72 mM, about 75 mM, about 78 mM, about 80 mM, about 82 mM, about 85 mM, about 88 mM, about 90 mM, about 92 mM, about 95 mM, or about 100 mM.

In some embodiments, the buffer is a histidine salt at a concentration of about 1 mM to about 100 mM, e.g., about 1 mM to about 50 mM, about 1 mM to about 55 mM, about 1 mM to about 60 mM, about 1 mM to about 70 mM, about 1 mM to about 80 mM, about 1 mM to about 85 mM, about 1 mM to about 90 mM, about 5 mM to about 100 mM, about 1 mM to about 80 mM, about 5 mM to about 70 mM, about 5 mM to about 60 mM, about 5 mM to about 50 mM, about 5 mM to about 35 mM, about 5 mM to about 20 mM, about 5 mM to about 10 mM, or about 10 mM to about 20 mM. In some embodiments, the histidine salt is at a concentration of about 1 mM, about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 22 mM, about 25 mM, about 26 mM, about 28 mM, about 30 mM, about 35 mM, about 40 mM, about 43 mM, about 45 mM, about 48 mM, about 50 mM, about 55 mM, about 57 mM, about 58 mM, about 60 mM, about 62 mM, about 65 mM, about 68 mM, about 70 mM, about 72 mM, about 75 mM, about 78 mM, about 80 mM, about 82 mM, about 85 mM, about 88 mM, about 90 mM, about 92 mM, about 95 mM, or about 100 mM.

In some specific embodiments, the buffer is acetic acid-histidine at a concentration of about 1 mM to about 100 mM, e.g., about 1 mM to about 50 mM, about 1 mM to about 55 mM, about 1 mM to about 60 mM, about 1 mM to about 70 mM, about 1 mM to about 80 mM, about 1 mM to about 85 mM, about 1 mM to about 90 mM, about 5 mM to about 100 mM, about 1 mM to about 80 mM, about 5 mM to about 70 mM, about 5 mM to about 60 mM, about 5 mM to about 50 mM, about 5 mM to about 35 mM, about 5 mM to about 20 mM, about 5 mM to about 10 mM, or about 10 mM to about 20 mM. In some embodiments, the acetic acid-histidine is at a concentration of about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 25 mM, about 30 mM, about 35 mM, about 40 mM, about 50 mM, about 55 mM, about 57 mM, about 58 mM, about 60 mM, about 62 mM, about 65 mM, about 68 mM, about 70 mM, about 72 mM, about 75 mM, about 78 mM, about 80 mM, about 82 mM, about 85 mM, about 88 mM, about 90 mM, about 92 mM, about 95 mM, or about 100 mM.

In some specific embodiments, the buffer is histidine-histidine hydrochloride at a concentration of about 1 mM to about 100 mM, e.g., about 1 mM to about 50 mM, about 1 mM to about 55 mM, about 1 mM to about 60 mM, about 1 mM to about 70 mM, about 1 mM to about 80 mM, about 1 mM to about 85 mM, about 1 mM to about 90 mM, about 5 mM to about 100 mM, about 1 mM to about 80 mM, about 5 mM to about 70 mM, about 5 mM to about 60 mM, about 5 mM to about 50 mM, about 5 mM to about 35 mM, about 5 mM to about 20 mM, about 5 mM to about 10 mM, about 10 mM to about 20 mM, about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 25 mM, about 30 mM, about 35 mM, about 40 mM, about 50 mM, about 55 mM, about 57 mM, about 58 mM, about 60 mM, about 62 mM, about 65 mM, about 68 mM, about 70 mM, about 72 mM, about 75 mM, about 78 mM, about 80 mM, about 82 mM, about 85 mM, about 88 mM, about 90 mM, about 92 mM, about 95 mM, or about 100 mM.

In some specific embodiments, the buffer is succinic acid-histidine at a concentration of about 1 mM to about 100 mM, e.g., about 1 mM to about 50 mM, about 1 mM to about 55 mM, about 1 mM to about 60 mM, about 1 mM to about 70 mM, about 1 mM to about 80 mM, about 1 mM to about 85 mM, about 1 mM to about 90 mM, about 5 mM to about 100 mM, about 1 mM to about 80 mM, about 5 mM to about 70 mM, about 5 mM to about 60 mM, about 5 mM to about 50 mM, about 5 mM to about 35 mM, about 5 mM to about 20 mM, about 5 mM to about 10 mM, or about 10 mM to about 20 mM. about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 25 mM, about 30 mM, about 35 mM, about 40 mM, about 50 mM, about 55 mM, about 57 mM, about 58 mM, about 60 mM, about 62 mM, about 65 mM, about 68 mM, about 70 mM, about 72 mM, about 75 mM, about 78 mM, about 80 mM, about 82 mM, about 85 mM, about 88 mM, about 90 mM, about 92 mM, about 95 mM, or about 100 mM.

The present disclosure provides a pharmaceutical composition comprising an immunoconjugate, and the pharmaceutical composition has a pH of about 4.0 to about 6.5. In some embodiments, the pharmaceutical composition comprises an immunoconjugate and a buffer.

In some embodiments, the buffer or the pharmaceutical composition has a pH of about 4.0 to about 6.5, e.g., about 4.0 to about 6.0, about 4.0 to about 5.5, about 4.0 to about 5.0, about 4.0 to about 4.8, 4.0 to about 4.5, about 4.8 to about 5.0, about 5.0 to about 5.5, about 4.5 to about 4.8, or any range between these point values. In some embodiments, the buffer or the pharmaceutical composition has a pH of about 4.0 to about 5.0. In some embodiments, the buffer or the pharmaceutical composition has a pH of about 4.0 to about 4.5.

In some embodiments, the buffer or the pharmaceutical composition has a pH of about 4.0, about 4.1, about 4.2, about 4.3, about 4.4, about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, or about 6.5.

Generally, the pH of a pharmaceutical composition obtained by buffer exchange is almost consistent with the pH of the buffer. In addition, it is well known to those skilled in the art that in the process of preparing a pharmaceutical formulation, there may sometimes be a pH drift, but the pH drift of the pharmaceutical formulation is generally small (e.g., within a range of $\pm 0.8$). In some embodiments, the pH drift of the pharmaceutical formulation is within a range of $\pm 0.5$.

In some embodiments, the immunoconjugate is at a concentration of about 0.01 mg/mL to about 200 mg/mL, e.g., about 0.1 mg/mL to about 200 mg/mL, about 0.5 mg/mL to about 200 mg/mL, about 1 mg/mL to about 200 mg/mL, about 5 mg/mL to about 200 mg/mL, about 10 mg/mL to about 200 mg/mL, about 10 mg/mL to about 180 mg/mL, about 10 mg/mL to about 150 mg/mL, about 10 mg/mL to about 120 mg/mL, about 10 mg/mL to about 110 mg/mL, about 10 mg/mL to about 100 mg/mL, about 10 mg/mL to about 80 mg/mL, about 10 mg/mL to about 60 mg/mL, about 10 mg/mL to about 40 mg/mL, about 20 mg/mL to about 200 mg/mL, about 20 mg/mL to about 180 mg/mL, about 20 mg/mL to about 150 mg/mL, about 20 mg/mL to about 120 mg/mL, about 20 mg/mL to about 110 mg/mL, about 20 mg/mL to about 100 mg/mL, about 20 mg/mL to about 80 mg/mL, about 20 mg/mL to about 60 mg/mL, about 20 mg/mL to about 40 mg/mL, about 40 mg/mL to about 120 mg/mL, about 40 mg/mL to about 110 mg/mL, about 40 mg/mL to about 100 mg/mL, about 40 mg/mL to about 80 mg/mL, 40 mg/mL to about 60 mg/mL, about 60 mg/mL to about 80 mg/mL, about 60 mg/mL to about 120 mg/mL, about 80 mg/mL to about 120 mg/mL, or any range between these point values. In some embodiments, the immunoconjugate is at a concentration of about 0.01 mg/mL, about 0.05 mg/mL, about 0.1 mg/mL, about 0.5 mg/mL, about 1 mg/mL, about 5 mg/mL, about 10 mg/mL, about 15 mg/mL, about 20 mg/mL, about 25 mg/mL, about 30 mg/mL, about 35 mg/mL, about 40 mg/mL, about 45 mg/mL, about 50 mg/mL, about 55 mg/mL, about 60 mg/mL, about 61 mg/mL, about 62 mg/mL, about 63 mg/mL, about 64 mg/mL, about 65 mg/mL, about 66 mg/mL, about 67 mg/mL, about 68 mg/mL, about 69 mg/mL, about 70 mg/mL, about 71 mg/mL, about 72 mg/mL, about 73 mg/mL, about 74 mg/mL, about 75 mg/mL, about 76 mg/mL, about 77 mg/mL, about 78 mg/mL, about 79 mg/mL, about 80 mg/mL, about 81 mg/mL, about 82 mg/mL, about 83 mg/mL, about 84 mg/mL, about 85 mg/mL, about 86 mg/mL, about 87 mg/mL, about 88 mg/mL, about 89 mg/mL, about 90 mg/mL, about 91 mg/mL, about 92 mg/mL, about 93 mg/mL, about 94 mg/mL, about 95 mg/mL, about 96 mg/mL, about 97 mg/mL, about 98 mg/mL, about 99 mg/mL, about 100 mg/mL, about 101 mg/mL, about 102 mg/mL, about 103 mg/mL, about 104 mg/mL, about 105 mg/mL, about 106 mg/mL, about 107 mg/mL, about 108 mg/mL, about 109 mg/mL, about 110 mg/mL, about 111 mg/mL, about 112 mg/mL, about 113 mg/mL, about 114 mg/mL, about 115 mg/mL, about 116 mg/mL, about 117 mg/mL, about 118 mg/mL, about 119 mg/mL, about 120 mg/mL, about 121 mg/mL, about 122 mg/mL, about 123 mg/mL, about 124 mg/mL, about 125 mg/mL, about 126 mg/mL, about 127 mg/mL, about 128 mg/mL, about 129 mg/mL, about 130 mg/mL, about 131 mg/mL, about 132 mg/mL, about 133 mg/mL, about 134 mg/mL, about 135 mg/mL, about 140 mg/mL, about 145 mg/mL, about 150 mg/mL, about 155 mg/mL, about 160 mg/mL, about 165 mg/mL, about 170 mg/mL, about 175 mg/mL, about 180 mg/mL, about 185 mg/mL, about 190 mg/mL, about 195 mg/mL, or about 200 mg/mL.

In some embodiments, the pharmaceutical composition according to any one of the above comprises a surfactant. In some embodiments, the surfactant is a nonionic surfactant. In some embodiments, the surfactant is selected from the group consisting of poloxamer (e.g., poloxamer 188), polysorbate (e.g., polysorbate 20 (i.e., PS20) or polysorbate 80 (i.e., PS80)), poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramidopropyl-betaine, cocamidopropyl-betaine, linoleamidopropyl-betaine, myristamidopropyl-betaine, palmitamidopropyl-betaine, isostearamidopropyl-betaine, myristamidopropyl-dimethylamine, palmitamidopropyl-dimethylamine, isostearamidopropyl-dimethylamine, sodium methyl cocoyl, sodium methyl oleyl taurate, polyethylene glycol, polypropylene glycol, copolymers of ethylene and propylene glycol, etc., and any combination thereof. In some embodiments, the surfactant is sodium dodecyl sulfate (SDS) or polysorbate. In some embodiments, the surfactant is polysorbate 80.

In some embodiments, the surfactant is at a concentration of about 0.001% (w/v) to about 1.0% (w/v), e.g., about 0.001% (w/v) to about 0.8% (w/v), about 0.001% (w/v) to about 0.5% (w/v), about 0.001% (w/v) to about 0.2% (w/v), about 0.001% (w/v) to about 0.1% (w/v), about 0.01% (w/v) to about 1.0% (w/v), about 0.01% (w/v) to about 0.8% (w/v), about 0.01% (w/v) to about 0.5% (w/v), about 0.01% (w/v) to about 0.2% (w/v), about 0.01% (w/v) to about 0.1% (w/v), about 0.01% (w/v) to about 0.18% (w/v), about 0.01% (w/v) to about 0.18% (w/v), about 0.01% (w/v) to about 0.15% (w/v), about 0.01% (w/v) to about 0.1% (w/v), about 0.01% (w/v) to about 0.08% (w/v), about 0.01% (w/v) to about 0.06% (w/v), about 0.01% (w/v) to about 0.04% (w/v), about 0.02% (w/v) to about 0.2% (w/v), about 0.02% (w/v) to about 0.15% (w/v), about 0.02% (w/v) to about 0.1% (w/v), about 0.02% (w/v) to about 0.08% (w/v), about 0.02% (w/v) to about 0.04% (w/v), about 0.04% (w/v) to about 0.2% (w/v), about 0.04% (w/v) to about 0.1% (w/v), about 0.04% (w/v) to about 0.08% (w/v), and any range between these point values. In some embodiments, the surfactant is at a concentration of about 0.001% (w/v), about 0.05% (w/v), about 0.01% (w/v), about 0.02% (w/v), about 0.03% (w/v), about 0.04% (w/v), about 0.05% (w/v), about 0.06% (w/v), about 0.07% (w/v), about 0.08% (w/v), about 0.09% (w/v), about 0.1% (w/v), about 0.11% (w/v), about 0.12% (w/v), about 0.13% (w/v), about 0.14% (w/v), about 0.15% (w/v), about 0.16% (w/v), about 0.17% (w/v), about 0.18% (w/v), about 0.19% (w/v), about 0.2% (w/v), about 0.3% (w/v), about 0.4% (w/v), about 0.5% (w/v), about 0.6% (w/v), about 0.7% (w/v), or about 0.8% (w/v).

In some embodiments, the pharmaceutical composition according to any one of the above comprises an osmotic pressure regulator. In some embodiments, the osmotic pressure regulator is a sugar (including monosaccharides, disaccharides, trisaccharides, polysaccharides, sugar alcohols, reducing sugars, non-reducing sugars, etc.), an amino acid (including arginine, glycine, cysteine, histidine, etc.), or a salt (sodium chloride, potassium chloride, calcium chloride, etc.). In some embodiments, the osmotic pressure regulator is a sugar selected from the group consisting of glucose, sucrose, trehalose, lactose, fructose, maltose, dextran, glycerin, erythritol, glycerol, arabitol, xylitol, sorbitol (also known as sorbol), mannitol, melibiose, melezitose, raffinose, mannotriose, stachyose, maltose, lactulose, maltulose, maltitol, lactitol, and iso-maltulose. In some embodiments, the osmotic pressure regulator is one or more selected from the group consisting of sucrose, trehalose, sorbitol, arginine, proline, glycine, and sodium chloride. In some embodiments, the osmotic pressure regulator is a non-reducing disaccharide. In some embodiments, the osmotic pressure regulator is trehalose and/or sucrose. In some embodiments, the osmotic pressure regulator is trehalose. In some embodiments, the trehalose is trehalose.

In some embodiments, the osmotic pressure regulator is at a concentration of about 1% (w/v) to about 20% (w/v), e.g., about 1% (w/v) to about 18% (w/v), about 1% (w/v) to about 15% (w/v), about 1% (w/v) to about 12 (w/v), about 1% (w/v) to about 8% (w/v), about 1% (w/v) to about 8.8% (w/v), about 2% (w/v) to about 20% (w/v), about 2% (w/v) to about 15% (w/v), about 2% (w/v) to about 10 (w/v), about 2% (w/v) to about 8% (w/v), about 2% (w/v) to about 8.8% (w/v), about 3% (w/v) to about 20% (w/v), about 3% (w/v) to about 15% (w/v), about 3% (w/v) to about 10% (w/v), about 3% (w/v) to about 8% (w/v), about 3% (w/v) to about 8.8% (w/v), about 4% (w/v) to about 20% (w/v), about 4% (w/v) to about 15% (w/v), about 4% (w/v) to about 10 (w/v), about 4% (w/v) to about 8% (w/v), about 4% (w/v) to about 8.8% (w/v), about 5% (w/v) to about 20% (w/v), about 5% (w/v) to about 15% (w/v), about 5% (w/v) to about 10 (w/v), about 5% (w/v) to about 8% (w/v), about 5% (w/v) to about 8.8% (w/v), about 4.4% (w/v) to about 20% (w/v), about 4.4% (w/v) to about 15% (w/v), about 4.4% (w/v) to about 10% (w/v), about 4.4% (w/v) to about 8% (w/v), about 4.4% (w/v) to about 8.8% (w/v), or any range between these point values. In some embodiments, the osmotic pressure regulator is at a concentration of about 1% (w/v), 1.2% (w/v), 1.4% (w/v), about 2% (w/v), about 3% (w/v), about 4% (w/v), about 4.2% (w/v), about 4.4% (w/v), about 4.8% (w/v), about 5% (w/v), about 6% (w/v), about 7% (w/v), about 7.8% (w/v), about 8% (w/v), about 8.2% (w/v), about 8.4% (w/v), about 8.8% (w/v), about 9% (w/v), about 10% (w/v), about 12% (w/v), about 14% (w/v), about 16% (w/v), about 18% (w/v), or about 20% (w/v).

In some embodiments, the pharmaceutical composition according to any one of the above further comprises a stabilizer. In some embodiments, the stabilizer is arginine or a salt thereof. In some embodiments, the pharmaceutical composition comprises arginine hydrochloride. In some embodiments, the pharmaceutical composition does not comprise arginine.

In some embodiments, the stabilizer is at a concentration of about 5 mM to about 200 mM, e.g., about 10 mM to about 150 mM, about 10 mM to about 180 mM, about 10 mM to about 120 mM, about 10 mM to about 100 mM, about 10 mM to about 80 mM, about 10 mM to about 70 mM, about 10 mM to about 50 mM, about 10 mM to about 30 mM, about 30 mM to about 50 mM, about 30 mM to about 80 mM, about 30 mM to about 100 mM, about 50 mM to about 70 mM, about 50 mM to about 100 mM, or any range between these point values. In some embodiments, the stabilizer is at a concentration of about 10 mM, about 20 mM, about 30 mM, about 40 mM, about 50 mM, about 60 mM, about 70 mM, about 80 mM, about 90 mM, about 100 mM, about 110 mM, about 120 mM, or about 150 mM.

In some embodiments, the pharmaceutical composition comprises the following:

(a) about 10 mg/mL to about 200 mg/mL immunoconjugate,

about 1 mM to about 50 mM buffer,
about 0.01% (w/v) to about 0.2% (w/v) surfactant,
about 1% (w/v) to about 20% (w/v) osmotic pressure regulator, and
about 10 mM to about 100 mM stabilizer, which is optionally present,
the pharmaceutical composition having a pH of about 4.0 to about 5.5; or

(b) about 10 mg/mL to about 120 mg/mL immunoconjugate,

about 5 mM to about 35 mM acetic acid or histidine salt buffer,
about 0.04% (w/v) to about 0.2% (w/v) polysorbate 80,
about 4% (w/v) to about 10% (w/v) sucrose or trehalose, and
about 30 mM to about 100 mM arginine or arginine hydrochloride, which is optionally present,
the pharmaceutical composition having a pH of about 4.0 to about 5.5; or

(c) about 10 mg/mL to about 120 mg/mL immunoconjugate,

about 5 mM to about 35 mM acetic acid, histidine-histidine hydrochloride, acetic acid-histidine, or succinic acid-histidine,
about 0.04% (w/v) to about 0.2% (w/v) polysorbate 80, and
about 4% (w/v) to about 10% (w/v) sucrose or trehalose,
the pharmaceutical composition having a pH of about 4.0 to about 5.0; or

(d) about 10 mg/mL to about 120 mg/mL immunoconjugate,

about 5 mM to about 20 mM acetic acid-histidine or succinic acid-histidine,
about 0.04% (w/v) to about 0.2% (w/v) polysorbate 80, and
about 4% (w/v) to about 8.8% (w/v) sucrose or trehalose,
the pharmaceutical composition having a pH of about 4.0 to about 5.0; or

(e) about 10 mg/mL to about 120 mg/mL immunoconjugate,

about 5 mM to about 20 mM succinic acid-histidine,
about 0.04% (w/v) to about 0.08% (w/v) polysorbate 80,
about 1% (w/v) to about 10% (w/v) trehalose, and
about 10 mM to about 100 mM arginine hydrochloride, which is optionally present,
the pharmaceutical composition having a pH of about 4.0 to about 4.5; or

(e-1) about 10 mg/mL to about 80 mg/mL immunoconjugate,

about 5 mM to about 10 mM succinic acid-histidine,
about 0.04% (w/v) to about 0.08% (w/v) polysorbate 80,
about 4% (w/v) to about 10% (w/v) trehalose, and
about 10 mM to about 100 mM arginine hydrochloride, which is optionally present,
the pharmaceutical composition having a pH of about 4.0 to about 4.5; or

(e-2) about 10 mg/mL to about 40 mg/mL immunoconjugate,

about 10 mM succinic acid-histidine,

about 0.04% (w/v) to about 0.08% (w/v) polysorbate 80, and
about 4% (w/v) to about 8.8% (w/v) trehalose,
the pharmaceutical composition having a pH of about 4.0 to about 4.5; or

(e-3) about 40 mg/mL to about 120 mg/mL immunoconjugate,

about 10 mM to about 20 mM succinic acid-histidine,
about 0.04% (w/v) to about 0.08% (w/v) polysorbate 80, and
about 4% (w/v) to about 10% (w/v) trehalose,
the pharmaceutical composition having a pH of about 4.0 to about 4.5; or

(e-4) about 40 mg/mL to about 80 mg/mL immunoconjugate,

about 20 mM succinic acid-histidine,
about 0.04% (w/v) to about 0.08% (w/v) polysorbate 80, and
about 4.4% (w/v) to about 8.8% (w/v) trehalose,
the pharmaceutical composition having a pH of about 4.0 to about 4.5; or

(f) about 10 mg/mL to about 120 mg/mL immunoconjugate,

about 5 mM to about 20 mM succinic acid-histidine,
about 0.1% (w/v) to about 0.2% (w/v) polysorbate 80,
about 4% (w/v) to about 8% (w/v) sucrose, and
about 10 mM to about 100 mM arginine hydrochloride, which is optionally present,
the pharmaceutical composition having a pH of about 4.5 to about 5.0; or

(f-1) about 10 mg/mL to about 80 mg/mL immunoconjugate,

about 5 mM to about 10 mM succinic acid-histidine,
about 0.1% (w/v) to about 0.2% (w/v) polysorbate 80,
about 4% (w/v) to about 8% (w/v) sucrose, and
about 10 mM to about 100 mM arginine hydrochloride, which is optionally present,
the pharmaceutical composition having a pH of about 4.5 to about 5.0; or

(f-2) about 40 mg/mL to about 80 mg/mL immunoconjugate,

about 5 mM succinic acid-histidine,
about 0.1% (w/v) to about 0.2% (w/v) polysorbate 80, and
about 4% (w/v) to about 8% (w/v) sucrose,
the pharmaceutical composition having a pH of about 4.5 to about 5.0; or

(f-3) about 40 mg/mL to about 120 mg/mL immunoconjugate,

about 10 mM to about 20 mM succinic acid-histidine,
about 0.1% (w/v) to about 0.2% (w/v) polysorbate 80, and
about 4% (w/v) to about 8% (w/v) sucrose,
the pharmaceutical composition having a pH of about 4.5 to about 5.0; or

(f-4) about 80 mg/mL to about 120 mg/mL immunoconjugate,

about 10 mM succinic acid-histidine,
about 0.1% (w/v) to about 0.2% (w/v) polysorbate 80, and
about 4% (w/v) to about 8% (w/v) sucrose,
the pharmaceutical composition having a pH of about 4.5 to about 5.0; or

(f-5) about 80 mg/mL to about 120 mg/mL immunoconjugate,

about 10 mM succinic acid-histidine,

about 0.2% (w/v) polysorbate 80, and
about 8% (w/v) sucrose,
the pharmaceutical composition having a pH of about 4.8; or

(f-6) about 80 mg/mL to about 120 mg/mL immunoconjugate,

about 10 mM succinic acid-histidine,
about 0.2% (w/v) polysorbate 80, and
about 8% (w/v) sucrose,
the pharmaceutical composition having a pH of about 5.0; or

(g) about 10 mg/mL to about 120 mg/mL immunoconjugate,

about 5 mM to about 20 mM acetic acid-histidine or histidine-histidine hydrochloride,
about 0.04% (w/v) to about 0.08% (w/v) polysorbate 80, and
about 4% (w/v) to about 8% (w/v) sucrose,
the pharmaceutical composition having a pH of about 4.5 to about 5.0.

In some embodiments, the pharmaceutical composition shown in any one of (a), (b), (e), (e-1), (f), and (f-1) comprises about 10 mM to about 100 mM arginine hydrochloride. In some embodiments, the pharmaceutical composition shown in any one of (a), (b), (e), (e-1), (f), and (f-1) does not comprise about 10 mM to about 100 mM arginine hydrochloride.

In some embodiments, the pharmaceutical composition comprises any one of the following:

(1) about 10 mg/mL to about 120 mg/mL immunoconjugate,

about 10 mM succinic acid-histidine,
about 0.04% (w/v) polysorbate 80, and
about 4.4% (w/v) trehalose,
the pharmaceutical composition having a pH of about 4.0 to about 4.5;

(2) about 10 mg/mL to about 80 mg/mL immunoconjugate,

about 10 mM succinic acid-histidine,
about 0.04% (w/v) polysorbate 80, and
about 4.4% (w/v) trehalose,
the pharmaceutical composition having a pH of about 4.0 to about 4.5;

(3) about 10 mg/mL to about 40 mg/mL immunoconjugate,

about 10 mM succinic acid-histidine,
about 0.04% (w/v) polysorbate 80, and
about 4.4% (w/v) trehalose,
the pharmaceutical composition having a pH of about 4.5;

(7) about 40 mg/mL immunoconjugate,

about 10 mM succinic acid-histidine,
about 0.04% (w/v) polysorbate 80, and
about 4.4% (w/v) trehalose,
the pharmaceutical composition having a pH of about 4.5;

(8) about 40 mg/mL immunoconjugate,

about 10 mM succinic acid-histidine,
about 0.08% (w/v) polysorbate 80, and
about 8% (w/v) trehalose,
the pharmaceutical composition having a pH of about 4.5;

(9) about 40 mg/mL immunoconjugate,

about 10 mM succinic acid-histidine,
about 0.04% (w/v) polysorbate 80, and
about 8% (w/v) trehalose,
the pharmaceutical composition having a pH of about 4.0;

(10) about 40 mg/mL immunoconjugate,

about 10 mM succinic acid-histidine,
about 0.08% (w/v) polysorbate 80, and
about 5% (w/v) trehalose,
the pharmaceutical composition having a pH of about 4.0;

(11) about 40 mg/mL immunoconjugate,

about 10 mM succinic acid-histidine,
about 0.08% (w/v) polysorbate 80, and
about 8% (w/v) trehalose,
the pharmaceutical composition having a pH of about 4.0; and

(12) about 40 mg/mL immunoconjugate,

about 10 mM succinic acid-histidine,
about 0.08% (w/v) polysorbate 80,
about 8% (w/v) trehalose, and
about 50 mM arginine hydrochloride,
the pharmaceutical composition having a pH of about 4.0;

or
(4) about 10 mg/mL to about 120 mg/mL immunoconjugate,

about 20 mM succinic acid-histidine,
about 0.08% (w/v) polysorbate 80, and
about 8.8% (w/v) trehalose,
the pharmaceutical composition having a pH of about 4.0 to about 4.5;

(5) about 40 mg/mL to about 120 mg/mL immunoconjugate,

about 20 mM succinic acid-histidine,
about 0.08% (w/v) polysorbate 80, and
about 8.8% (w/v) trehalose,
the pharmaceutical composition having a pH of about 4.0 to about 4.5;

(6) about 40 mg/mL to about 80 mg/mL immunoconjugate,

about 20 mM succinic acid-histidine,
about 0.08% (w/v) polysorbate 80, and
about 8.8% (w/v) trehalose,
the pharmaceutical composition having a pH of about 4.5; and

(13) about 80 mg/mL immunoconjugate,

about 20 mM succinic acid-histidine,
about 0.08% (w/v) polysorbate 80, and
about 8.8% (w/v) trehalose,
the pharmaceutical composition having a pH of about 4.5.

In some embodiments, the immunoconjugate in the pharmaceutical composition of the present disclosure is a fusion protein of an antigen-binding domain and a cytokine. In some embodiments, the immunoconjugate is a fusion protein of a PD-1-binding domain and a cytokine.

In some embodiments, the PD-1-binding domain comprises at least one immunoglobulin single variable domain comprising a CDR1, a CDR2, and a CDR3 from the amino acid sequence set forth in any one of SEQ ID NOs: 2 and 8-15.

The CDR1, the CDR2, and the CDR3 are defined according to the Kabat, IMGT, Chothia, AbM, or Contact numbering scheme. The immunoglobulin single variable structure specifically binds to a PD-1 antigen or a fragment thereof.

PD-1-Binding Domain

In some embodiments, in the aforementioned PD-1-binding domain, according to the Kabat numbering scheme, the amino acid sequences of the CDR1, the CDR2, and the CDR3 of the immunoglobulin single variable domain are set forth in SEQ ID NOs: 3, 16, and 17, respectively. In some embodiments, the amino acid sequence of the CDR1 of the immunoglobulin single variable domain in the aforementioned PD-1-binding domain is set forth in SEQ ID NO: 3, the amino acid sequence of the CDR2 is set forth in SEQ ID NO: 4 or 18, and the amino acid sequence of the CDR3 is set forth in any one of SEQ ID NOs: 5 and 19-20.

In some embodiments, the amino acid sequences of the CDR1, the CDR2, and the CDR3 of the immunoglobulin single variable domain in the aforementioned PD-1-binding domain are set forth in:

set forth in SEQ ID NOs: 3, 18, and 19, respectively,
set forth in SEQ ID NOs: 3, 4, and 5, respectively, or
SEQ ID NOs: 3, 4, and 20, respectively.

In some embodiments, the immunoglobulin single variable domain in the aforementioned PD-1-binding domain is engineered by humanization, avidity maturation, T cell epitope removal, reduction of antibody deamidation, and/or reduction of antibody isomerization. In some embodiments, the immunoglobulin single variable domain is obtained by avidity maturation and has one or more changes in one or more CDRs that result in an increase in avidity for PD-1 as compared to the parent molecule.

In some specific embodiments, the humanization engineering process uses the heavy chain framework region IGHV3-23*01 or IGHV3-23*04 of a human germline template.

In some embodiments, the amino acid sequence of the immunoglobulin single variable domain in the aforementioned PD-1-binding domain is set forth in any one of SEQ ID NOs: 2 and 8-15, or has at least 80%, at least 85%, or at least 90% sequence identity to any one of the aforementioned sequences.

In some embodiments, the amino acid sequence of the immunoglobulin single variable domain in the aforementioned PD-1-binding domain is set forth in SEQ ID NO: 13, or has at least 80%, at least 85%, or at least 90% sequence identity to any one of the aforementioned sequences.

In some embodiments, the at least one immunoglobulin single variable domain in the aforementioned PD-1-binding domain is a VHH.

In some embodiments, the aforementioned PD-1-binding domain comprises one or more (e.g., 2, 3, 4, 5, or 6) of the aforementioned immunoglobulin single variable domains, and the immunoglobulin single variable domains may be identical or different and may form a dimeric or multimeric molecule.

In some embodiments, the aforementioned PD-1-binding domain comprises or is an anti-PD-1 antibody or an antigen-binding fragment thereof that specifically binds to PD-1 or a fragment thereof; the antibody or the antigen-binding fragment thereof is, for example, a camelid antibody, a chimeric antibody, a humanized antibody, a fully human antibody, or an antigen-binding fragment thereof; the antibody or the antigen-binding fragment thereof is, for example, a recombinant antibody or a fragment thereof. In some specific embodiments, the antigen-binding fragment is a linear antibody, a single-chain antibody, a nanobody, a peptibody, a domain antibody, or a multispecific antibody (a bispecific antibody, a diabody, a triabody, a tetrabody, a tandem di-scFv, or a tandem tri-scFv).

In some embodiments, the aforementioned anti-PD-1 antibody or the antigen-binding fragment thereof further comprises a human immunoglobulin Fc region; for example, the Fc region is an Fc region of human IgG1, IgG2, or IgG4. The Fc region may comprise mutations. Exemplary mutations are L234A/L235A on IgG1, V234A/G237A/P238S/H268A/V309L/A330S/P331S on IgG2, F234A/L235A on IgG4, S228P/F234A/L235A on IgG4, N297A on IgG1, IgG2, IgG3, or IgG4, V234A/G237A on IgG2, K214T/E233P/L234V/L235A/G236 deletion/A327G/P331A/D365E/L358M on IgG1, H268Q/V309L/A330S/P331S on IgG2, L234F/L235E/D265A on IgG1, L234A/L235A/G237A/P238S/H268A/A330S/P331S on IgG1, S228P/F234A/L235A/G237A/P238S on IgG4, and S228P/F234A/L235A/G236 deletion/G237A/P238S on IgG4. A hybrid IgG2/4 Fc domain may also be used, such as an Fc with residues 117-260 from IgG2 and residues 261-447 from IgG4. In some specific embodiments, the Fc region of human IgG4 comprises mutations S228P, F234A, L235A, and/or K447A. In some specific embodiments, the Fc region of human IgG1 comprises mutations L234A/L235A or L234A/L235A/P329G.

In some embodiments, the Fc region that the aforementioned PD-1 antibody or the antigen-binding fragment thereof comprises can cause the binding protein to form a dimeric molecule and extend the *in vivo* half-life of the binding protein.

In some embodiments, in the aforementioned PD-1 antibody or the antigen-binding fragment thereof, the immunoglobulin single variable domain is linked to the Fc region by a linker. The linker may be a non-functional amino acid sequence of 1-20 or more amino acids having no secondary or higher structure. For example, the linker is a flexible linker, such as $G_4S$, GS, GAP, $(G_4S)_2$, $(G_4S)_3$, $(G_4S)_4$, $(G_4S)_5$, or ASGS.

In some embodiments, the aforementioned PD-1 antibody or the antigen-binding fragment thereof has activity selected from the group consisting of at least one of the following:

(a) binding to human PD-1 or an epitope thereof with a $K_D$ of $\leq 10^{-7}$;
(b) inhibiting the binding of PD-1 to PD-L1;
(c) inhibiting the binding of PD-1 to PD-L2;
(d) inducing CD4$^+$ T cells to secrete IFN-y;
(e) enhancing PBMC activation;
(f) enhancing T cell activation; and
(g) inhibiting tumor growth.

In some embodiments, the aforementioned PD-1-binding domain of the present disclosure may bind to PD-1 with a $K_D$ of $\leq 1 \times 10^{-7}$ M, e.g., $\leq 1 \times 10^{-8}$ M, or $\leq 1 \times 10^{-9}$ M, or $\leq 1 \times 10^{-10}$ M.

In some embodiments, the PD-1-binding domain of the present disclosure is capable of specifically binding to human PD-1 and blocking the interaction between PD-1 and PD-L1, and/or the interaction between PD-1 and PD-L2.

In some embodiments, the aforementioned PD-1-binding domain of the present disclosure is capable of inhibiting tumor growth by at least about 10%, e.g., at least about 20%, about 30%, about 40%, about 50%, about 60%, about 70%, or about 80%.

In some embodiments, the aforementioned PD-1-binding domain of the present disclosure encompasses variants; for example, the domain comprises one or more amino acid substitutions, preferably conservative amino acid substitutions, e.g., 1, 2, 3, 4, 5, 6, 7, 8, 9, or 10 conservative amino acid substitutions, as compared to any one of SEQ ID NOs: 2 and 8-15, and the substitutions may occur in CDRs and/or FRs.

In some embodiments, the aforementioned PD-1-binding domain of the present disclosure is resistant to heat treatment or has relatively high stability. For example, no significant aggregation or degradation is observed after up to 30 days of treatment at 40 °C, and the domain is stable at least at 60 °C.

In some embodiments, provided is a PD-1 antibody or an antigen-binding fragment thereof that binds to or competes for binding to the same epitope with the immunoglobulin single variable domain of the aforementioned PD-1-binding domain of the present disclosure.

In some embodiments, provided is a PD-1 antibody or an antigen-binding fragment thereof that blocks the binding of the immunoglobulin single variable domain of the aforementioned PD-1-binding domain of the present disclosure to PD-1 (e.g., human PD-1).

In some embodiments, provided is a PD-1 antibody or an antigen-binding fragment thereof whose binding to PD-1 (e.g., human PD-1) is blocked by the immunoglobulin single variable domain in the aforementioned PD-1 antibody or the antigen-binding fragment thereof of the present disclosure.

In some embodiments, the aforementioned PD-1-binding domain of the present disclosure reduces the binding of PD-1 to PD-L1 and/or PD-L2 by at least 60%, at least 70%, at least 80%, at least 90%, at least 95%, or at least 98%.

In some embodiments, the present disclosure provides a PD-1-binding protein comprising at least one of the aforementioned immunoglobulin single variable domains that bind to PD-1 (such as a VHH).

As previously described, and in the present disclosure, "at least 90% identity" includes at least 90%, at least 91%, at least 92%, at least 93%, at least 94%, at least 95%, at least 96%, at least 97%, at least 98%, and at least 99% identity.

IL-2 or Variant Thereof

In some embodiments, the cytokine is IL-2 or a variant thereof. The IL-2 is preferably human IL-2, and the amino acid sequence of wild-type human IL-2 is set forth in, for example, SEQ ID NO: 50. The position of the IL-2 variant of the present disclosure is numbered according to the wild-type IL-2 amino acid sequence set forth in SEQ ID NO: 50.

In some embodiments, the present disclosure provides an IL-2 variant having a reduced avidity for the high-avidity receptors (IL-2R$\alpha$/$\beta$/$\gamma$) and the moderate-avidity receptors (IL-2R$\beta$/$\gamma$) as compared to wild-type IL-2 and/or having an eliminated avidity for the low-avidity receptor (IL-2R$\alpha$) as compared to wild-type IL-2. In some specific embodiments, the avidity of the IL-2 variant for IL-2Rα/β/γ is reduced more than the avidity for IL-2Rβ/γ. The avidity can be measured by conventional methods, such as ELISA, ForteBio, and SPR, such as the SPR measuring method provided in Example 13 of the present disclosure.

In some embodiments, the present disclosure provides an IL-2 variant comprising or consisting of mutation (i), mutation (ii), mutation (iii), or mutation (iv) below, or any combination thereof.

Regarding mutation (i):

The present disclosure provides a mutation that makes the IL-2 variant have an altered (e.g., increased or reduced) avidity for IL-2Rβ/γ as compared to wild-type IL-2.

In some embodiments, the IL-2 variant comprises a mutation (i) that makes the variant have a reduced avidity for IL-2Rβ/γ as compared to wild-type IL-2. In some specific embodiments, the IL-2 variant has a 1-1000 fold, 2-800 fold, 5-500 fold, 5-200 fold, 10-200 fold, 10-100 fold, 10-50 fold, 20-200 fold, 20-100 fold, 5-20 fold, 2-20 fold, 50-100 fold, or 50-70 fold, e.g., about 5, about 10, about 13, about 15, about 20, about 30, about 40, about 50, about 60, about 63, about 70, about 80, about 90, about 100, about 110, about 120, about 130, about 140, about 150, about 180, about 200, about 250, about 300, about 400, or about 500 fold reduction in avidity for IL-2Rβ/γ as compared to wild-type IL-2. In some specific embodiments, the IL-2 variant has a 10-100 fold reduction in avidity for IL-2Rβ/γ as compared to wild-type IL-2. The avidity can be measured by conventional methods, such as the SPR measuring method provided in Example 13 of the present disclosure.

In some embodiments, the aforementioned mutation (i) is located at positions 12-20, positions 84-95, and positions 126-130 of IL-2.

In some embodiments, the aforementioned mutation (i) is selected from the group consisting of positions 12, 15, 16, 18, 19, 20, 23, 43, 69, 84, 87, 88, 91, 92, 95, 123, 126, 127, 129, and 130, and any combination thereof; in some embodiments, the mutation is selected from the group consisting of positions L12, E15, H16, L18, L19, D20, M23, Q22, K43, V69, D84, S87, N88, V91, I92, E95, T123, Q126, S127, I129, and S130, and any combination thereof, e.g., N88/D20; in some specific embodiments, the mutation is selected from the group consisting of L12R, L12K, L12E, L12Q, E15Q, E15R, E15A, E15S, H16N, H16A, H16E, H16D, H16G, H16S, H16T, H16V, H16P, D20A, D20H, D20Y, D20N, D20Q, D20E, D20R, D20S, M23A, M23R, M23K, M23G, M23S, M23T, M23V, M23P, S87K, S87A, D84S, D84L, D84N, D84V, D84H, D84Y, D84R, D84K, D84G, D84A, D84T, D84P, N88D, N88A, N88S, N88E, N88T, N88R, N88I, V91A, V91T, V91E, V91I, V91L, V91D, V91N, V91Q, V91S, V91H, I92E, I92T, I92K, I92R, I92L, E95S, E95A, E95R, E95Q, E95G, E95T, E95V, E95P, E95H, E95N, T123A, T123E, T123K, T123Q, Q126D, Q126L, Q126A, Q126S, Q126T, Q126E, S127A, S127E, S127K, S127Q, D84N/E95Q, D84E/E95Q, D84T/E95Q, D84Q/E95Q, D84T/H16T, D84N/V91I, D84T/Q126E, D84N/Q126E, H16T/D84Q, and H16T/V91I; in some embodiments, the mutation (i) is selected from the group consisting of N88D, N88R, D20A, D20N, V91T, Q126D, and H16A/D84S; in some embodiments, the mutation (i) is N88D or D20A.

Regarding mutation (ii):

The present disclosure provides a mutation that makes the IL-2 variant have an altered (e.g., reduced) avidity for IL-2Rα as compared to wild-type IL-2, or not bind to IL-2Rα.

In some embodiments, the IL-2 variant comprises a mutation (ii) that makes the variant have a reduced avidity for IL-2Rα as compared to wild-type IL-2, or not bind to IL-2Rα. The avidity can be measured by conventional methods, such as the SPR measuring method provided in Example 13 of the present disclosure.

In some specific embodiments, the aforementioned mutation (ii) is selected from the group consisting of positions 35, 37, 38, 41, 42, 43, 45, 61, 62, 65, 68, and 72, and any combination thereof; in some embodiments, the mutation (ii) is selected from the group consisting of positions K35, T37, R38, E62, P65, E68, F42, Y45, and L72, and any combination thereof; in some embodiments, the mutation is selected from the group consisting of R38A, R38D, R38E, E62Q, F42A, F42G, F42S, F42T, F42Q, F42E, F42N, F42D, F42R, F42K, Y45A, Y45G, Y45S, Y45T, Y45Q, Y45E, Y45N, Y45D, Y45R, Y45K, P65R, P65E, P65K, P65H, P65Y, P65Q, P65D, P65N, E68A, E68Q, E68K, E68R, L72G, L72A, L72S, L72T, L72Q, L72E, L72N, L72D, L72R, and L72K; in some embodiments, the mutation (ii) is selected from the group consisting of F42A, Y45A, L72G, F42A/L72G, F42A/Y45A, Y45A/L72G, and F42A/Y45A/L72G.

Regarding mutation (iii):

The present disclosure provides a mutation (iii) that makes the IL-2 variant have increased stability, expression, purity, and druggability.

The IL-2 variants in WO2020125743A are incorporated herein by reference in their entirety.

In some embodiments, the IL-2 variant comprises a mutation (iii) that is selected from the group consisting of positions 11, 26, 27, 29, 30, 31, 32, 33, 34, 35, 36, 37, 38, 39, 40, 41, 42, 43, 44, 70, 71, 72, 78, 82, 88, and 132, and any combination thereof; in some embodiments, the mutation (iii) is selected from the group consisting of positions N26, N29, N30, N71, Q11, L132, L70, P82, G27, and F78, and any combination thereof; in some embodiments, the mutation (iii) is selected from the group consisting of N26Q, N29S, N30S, N71Q, Q11C/L132C, L70C/P82C, and G27C/F78C, and any combination thereof; in some embodiments, the mutation (iii) is selected from the group consisting of N26Q, N29S, N30S, N26Q/N29S/N71Q, N26Q/N29S, N26Q/N30S, N26Q/N30S/Q11C/L132C, N26Q/N29S/L70C/P82C, and N26Q/N30S/G27C/F78C.

Regarding mutation (iv):

In some embodiments, the IL-2 variant comprises a mutation (iv) that is selected from the group consisting of position 3 and/or position 125; in some embodiments, the mutation (iv) is selected from the group consisting of position T3 and/or position C125; in some embodiments, the mutation (iv) is selected from the group consisting of mutation(s) T3A, T3G, T3Q, T3E, T3N, T3D, T3R, T3K, or T3P and/or C125A, C125S, C125T, or C125V.

I. Exemplary IL-2 Variants

The present disclosure provides an IL-2 comprising or only comprising the following mutations:

(a) F42A and N88D, N88A, N88S, N88E, N88T, N88R, or N88I;
(b) F42A and D20A, D20H, D20Y, D20N, D20Q, D20E, D20R, or D20S;
(c) F42A and Q126D, Q126L, Q126A, Q126S, Q126T, or Q126E;
(d) F42A and V91A, V91T, V91E, V91I, V91L, V91D, V91N, V91Q, V91S, or V91H;
(e) F42A/L72G and N88D, N88A, N88S, N88E, N88T, N88R, or N88I;
(f) F42A/L72G and D20A, D20H, D20Y, D20N, D20Q, D20E, D20R, or D20S;
(g) F42A/L72G and Q126D, Q126L, Q126A, Q126S, Q126T, or Q126E;
(h) F42A/L72G and V91A, V91T, V91E, V91I, V91L, V91D, V91N, V91Q, V91S, or V91H;
(i) any one of the aforementioned (a)-(h) and N26Q;
(j) any one of the aforementioned (a)-(h) and N29S;
(k) any one of the aforementioned (a)-(h) and N71Q;
(l) any one of the aforementioned (a)-(h) and N26Q/N29S;
(m) any one of the aforementioned (a)-(h) and N26Q/N29S/N71Q;
(n) any one of the aforementioned (a)-(h) and Q11C/L132C;
(o) any one of the aforementioned (a)-(h) and L70C/P82C;
(p) any one of the aforementioned (a)-(h) and G27C/F78C;
(q) any one of the aforementioned (a)-(h) and T3A; or
(r) any one of the aforementioned (a)-(h) and T3A/C125A.

II. Exemplary IL-2 Variants

The present disclosure provides an IL-2 comprising or only comprising the following mutations:

42A/72G/88D,
42A/72G/88R,
42A/72G/20A,
42A/72G/20N,
42A/72G/91T,
42A/72G/126D,
42A/72G/16A/84S,
42A/72G/26Q/29S/71Q,
38E/42A/91T,
42A/72G/88D/26Q/29S/71Q,
42A/72G/88R/26Q/29S/71Q,
42A/72G/20A/26Q/29S/71Q,
42A/72G/20N/26Q/29S/71Q,
42A/72G/91T/26Q/29S/71Q,
42A/72G/126D/26Q/29S/71Q, or
42A/72G/16A/84S/26Q/29S/71Q.

III. Exemplary IL-2 Variants

The present disclosure provides an IL-2 comprising or only comprising the following mutations:

T3A/F42A/L72G/N88D/C 125A,
T3A/F42A/L72G/N88R/C125A,
T3A/F42A/L72G/D20A/C 125A,
T3A/F42A/L72G/D20N/C 125A,
T3A/F42A/L72G/V91T/C125A,
T3A/F42A/L72G/Q126D/C125A,
T3A/F42A/L72G/H16A/D84S/C125A,
T3A/F42A/L72G/N26Q/N29S/N71Q/C125A,
T3A/R38E/F42A/V91T/C125A,
T3A/F42A/L72G/N88D/N26Q/N29S/N71Q/C125A,
T3A/F42A/L72G/N88R/N26Q/N29S/N71Q/C125A,
T3A/F42A/L72G/D20A/N26Q/N29S/N71Q/C125A,
T3A/F42A/L72G/D20N/N26Q/N29S/N71Q/C125A,
T3A/F42A/L72G/V91T/N26Q/N29S/N71Q/C125A,
T3A/F42A/L72G/Q126D/N26Q/N29S/N71Q/C125A, or
T3A/F42A/L72G/H16A/D84S/N26Q/N29S/N71Q/C125A.

In some embodiments, the present disclosure provides an IL-2 variant comprising or having the amino acid sequence set forth in any one of SEQ ID NOs: 23-31 or has at least 80%, at least 85%, or at least 90% sequence identity thereto.

In some embodiments, any of the aforementioned IL-2 variants that comprise the mutation (i) has any one or more of 1)-5) as compared to wild-type IL-2:

1) a reduced avidity for the high-avidity IL-2R receptor (IL-2Rαβγ);
2) a reduced avidity for the moderate-avidity IL-2R receptor (IL-2Rβγ);
3) reduced activation of T cells (e.g., CD4+ and CD8+ T cells);
4) reduced activation of NK cells;
5) reduced IL-2-stimulated release of T cell and/or NK cell inflammatory factors.

In some embodiments, any of the aforementioned IL-2 variants that comprise the mutations (i) and (ii) has any one or more of the aforementioned 1)-5) and the following 6)-8) as compared to wild-type IL-2:

6) a reduced avidity for IL-2Rα or no binding to IL-2Rα;
7) the avidity for IL-2Rαβγ being reduced more than the avidity for IL-2Rβγ;
8) reduced activation of CD25+ cells (e.g., CD25+ CD8+ T cells, and Treg cells).

In some embodiments, any of the aforementioned IL-2 variants that comprise the mutations (i) and (ii) and (iii) and/or (iv) has any one or more of the aforementioned 1)-8) and the following 9) as compared to wild-type IL-2:
9) increased stability, druggability, expression, purity, and/or extended *in vivo* half-life.

Immunoconjugate

The present disclosure provides an immunoconjugate comprising an antigen-binding domain and IL-2 or a variant thereof.

In some embodiments, the antigen-binding domain in the immunoconjugate targets an immune effector cell or a tumor cell; in some specific embodiments, the immune effector cell is capable of responding to IL-2 stimulation or activation; in some specific embodiments, the immune effector cell has IL-2Rβγ thereon; in some specific embodiments, the immune effector cell is, for example, an NK cell, a CD4+ CD25- T cell, or a CD8+ CD25- T cell.

In some embodiments, the antigen-binding domain in the immunoconjugate targets PD-1, CTLA-4, LAG3, TIM3, 4-1BB, OX40, GITR, CD8a, CD8b, CD4, NKp30, NKG2A, TIGIT, TGFβR1, TGFβR2, Fas, NKG2D, NKp46, PD-L1, CD107a, ICOS, TNFR2, FAP, TNC A1, TNC A2, CEA, EDB, MCSP, or CD16a; in some specific embodiments, the antigen-binding domain targets PD-1 (i.e., a PD-1-binding domain).

In some embodiments, the PD-1-binding domain is any of the aforementioned PD-1-binding domains provided by the present disclosure.

Illustratively, in some specific embodiments, the PD-1-binding domain comprises at least one immunoglobulin single variable domain comprising a CDR1, a CDR2, and a CDR3 from the amino acid sequence set forth in any one of SEQ ID NOs: 2 and 8-15; the CDR1, the CDR2, and the CDR3 are defined according to the Kabat, IMGT, Chothia, AbM, or Contact numbering scheme.

Illustratively, in some specific embodiments, in the PD-1-binding domain, according to the Kabat numbering scheme, the amino acid sequences of the CDR1, the CDR2, and the CDR3 of the immunoglobulin single variable domain are set forth in SEQ ID NOs: 3, 16, and 17, respectively.

Illustratively, in some specific embodiments, the amino acid sequence of the CDR1 of the immunoglobulin single variable domain in the PD-1-binding domain is set forth in SEQ ID NO: 3, the amino acid sequence of the CDR2 is set forth in SEQ ID NO: 4 or 18, and the amino acid sequence of the CDR3 is set forth in any one of SEQ ID NOs: 5 and 19-20.

Illustratively, in some specific embodiments, the immunoglobulin single variable domain comprises a CDR1, a CDR2, and a CDR3 that are set forth in SEQ ID NOs: 3, 4, and 5, or SEQ ID NOs: 3, 18, and 19, or SEQ ID NOs: 3, 4, and 20.

Illustratively, in some specific embodiments, the amino acid sequence of the immunoglobulin single variable domain is set forth in any one of SEQ ID NOs: 2 and 8-15 or has at least 80% sequence identity to any one of the sequences.

In some embodiments, the PD-1-binding domain is selected from the group consisting of the PD-1-binding domains in the following PD-1 antibodies: pembrolizumab, nivolumab, sintilimab, tislelizumab, cemiplimab, toripalimab, camrelizumab, penpulimab, zimberelimab, sasanlimab, spartalizumab, retifanlimab, balstilimab, cetrelimab, dostarlimab (TSR-042), CS-1003, SCT-I10A, SSI-361, MEDI-0680(AMP-514), BAT-1306, JTX-4014, JTX-4014, BI-754091, and AK-103. In some other embodiments, the antigen-binding domain in the immunoconjugate is selected from the group consisting of the antigen-binding domains of the following antibodies: PD-L1 antibodies (e.g., durvalumab, atezolizumab, durvalumab, avelumab, adebrelimab, envafolimab, STI-A1110, CS-1001, TQB-2450, KL-A167, IMC-001, and CX-072); CTLA-4 antibodies (e.g., tremelimumab and ipilimumab); and 4-1BB antibodies (e.g., urelumab and utomilumab). The antigen-binding domain (e.g., the PD-1-binding domain) is a portion of an antibody (e.g., a PD-1 antibody) that binds to an antigen (e.g., PD-1), e.g., the Fab' thereof, the F(ab')2 thereof, or the heavy chain variable region thereof and the light chain variable region thereof, or a domain that comprises the HCDR1, the HCDR2, and the HCDR3 of the heavy chain variable region thereof and the LCDR1, the LCDR2, and the LCDR3 of the light chain variable region thereof.

In some embodiments, the PD-1-binding domain comprises pembrolizumab or an antigen-binding fragment thereof (e.g., the Fab); for example, the domain comprises a heavy chain variable region (VH) and a light chain variable region (VL), wherein the heavy chain variable region comprises a HCDR1, a HCDR2, and a HCDR3 of the amino acid sequences set forth in SEQ ID NOs: 54, 55, and 56, and the light chain variable region comprises a LCDR1, a LCDR2, and a LCDR3 of the amino acid sequences set forth in SEQ ID NOs: 57, 58, and 59. In some specific embodiments, the heavy chain variable region is a heavy chain variable region from a Fab heavy chain of the amino acid sequence set forth in SEQ ID NO: 52, and the light chain variable region comprises a light chain variable region from a Fab light chain of the amino acid sequence set forth in SEQ ID NO: 53.

In some embodiments, the IL-2 or the variant thereof in the immunoconjugate is any of the aforementioned IL-2 or the variants thereof provided by the present disclosure.

Illustratively, in some specific embodiments, the IL-2 is wild-type human IL-2.

Illustratively, in some specific embodiments, the IL-2 variant comprises or consists of the aforementioned mutation (i), mutation (ii), mutation (iii), or mutation (iv) provided by the present disclosure or any combination thereof.

Illustratively, in some specific embodiments, the IL-2 variant comprises or only comprises a mutation of any one of the aforementioned (a)-(r) provided by the present disclosure.

Illustratively, in some specific embodiments, the IL-2 variant comprises or consists of the amino acid sequence set forth in any one of SEQ ID NOs: 23-31 or a sequence having at least 80%, at least 85%, or at least 90% identity thereto.

In some embodiments, the immunoconjugate further comprises a human immunoglobulin Fc region, e.g., an Fc region of human IgG1, IgG2, IgG3, or IgG4.

In some embodiments, the antigen-binding domain (e.g., the PD-1-binding domain) in the immunoconjugate is located at the N-terminus of the Fc region, and the IL-2 or the variant thereof is located at the N-terminus or C-terminus of the Fc region. In some specific embodiments, the antigen-binding domain is located at the N-terminus of the Fc region, and the IL-2 or the variant thereof is located at the C-terminus of the Fc region.

In some embodiments, the valence ratio of the antigen-binding domain (e.g., the PD-1-binding domain) to the IL-2 or the variant thereof in the immunoconjugate is between 6:1 and 1:3 (e.g., 4:1 to 1:2), specifically, e.g., 4:1, 2:1, or 1:1.

In some embodiments, in the immunoconjugate, one immunoconjugate comprises 1 or 2 IL-2 or variants thereof and 1, 2, 3, 4, 5, or 6 antigen-binding domains (e.g., PD-1-binding domains).

In some embodiments, the immunoconjugate further comprises an Fc region, and the Fc region comprises a first subunit Fc1 and a second subunit Fc2 that are capable of associating with each other.

In some embodiments, Fc1 and Fc2 comprise such amino acid mutations that Fc1 preferentially pairs with Fc2 or preferentially forms a heterodimer as compared to Fc1. In some embodiments, the mutations are located in the CH3 domains of Fc1 and Fc2. In some embodiments, the amino acid mutations in Fc1 and Fc2 result in greater electrostatic complementarity than a wild type without the mutations. In some embodiments, the amino acid mutations in Fc1 and Fc2 result in greater steric complementarity than a wild type without the mutations.

In some embodiments, in Fc1 and Fc2, for example, within the CH3/CH3 interface, one or more amino acid residues in the CH3 domain of Fc1 are mutated with one or more amino acid residues having a larger side-chain volume, thereby forming protuberances (or knobs) in the surface of the CH3 domain of Fc1; one or more, preferably two or three, of the amino acid residues in the CH3 domain of Fc2 that interact with the CH3 domain of Fc1 are mutated with amino acid residues having a smaller side-chain volume, thereby forming cavities (or holes) in the surface of the CH3 domain of Fc2 that interacts with the CH3 domain of Fc1. In some embodiments, an import residue having a larger side-chain volume is phenylalanine (F), tyrosine (Y), arginine (R), or tryptophan (W). In some embodiments, an import residue having a smaller side-chain volume is serine (S), alanine (A), valine (V), or threonine (T).

In some specific embodiments, the Fc1 comprises at least one or at least two amino acid mutations selected from the group consisting of T366S, L368A, and Y407V (hole mutation modifications), and the Fc2 comprises T366W (a knob mutation modification); or the Fc1 comprises T366W (a knob mutation modification), and the Fc2 comprises at least one or at least two amino acid mutations selected from the group consisting of T366S, L368A, and Y407V (hole mutation modifications).

In some specific embodiments, the IL-2 or the variant thereof is located in the polypeptide chain where Fc1 is present.

In some specific embodiments, Fc1 and Fc2 may comprise natural non-cysteine-to-cysteine mutations, for example, in the CH3 domains; for example, Fc1 comprises S354C, and Fc2 comprises Y349C; or Fc1 comprises Y349C, and Fc2 comprises S354C.

In some specific embodiments, Fc1 and Fc2 comprise the following amino acid mutations or a combination thereof within, for example, the Fc1 CH3/Fc2 CH3 interface:

T366Y/Y407T;
T366W/Y407A;
T366Y/Y407T;
T394W/F405A;
T366Y/F405A/T394W/Y407T;
T366W/F405W/T394S/Y407A;
F405W/T394S;
D399C/K392C;
T366W/T366S/L368A/Y407V;
T366W/D399C/T366S/L368A/K392C/Y407V;
T366W/K392C/T366S/D399C/L368A/Y407V;
S354C/T366W/Y349C/T366S/L368A/Y407V;
Y349C/T366W/S354C/T366S/L368A/Y407V;
E356C/T366W/Y349C/T366S/L368A/Y407V;
Y349C/T366W/E356C/T366S/L368A/Y407V;
E357C/T366W/Y349C/T366S/L368A/Y407V; and
Y349C/T366W/E357C/T366S/L368A/Y407V.

In some specific embodiments, Fc1 and Fc2 further comprise amino acid mutations that cause the formation of an electrostatic interaction interface between Fc1 and Fc2 (e.g., CH3 and CH3). The amino acid mutations that cause the formation of the electrostatic interaction interface are selected from the group consisting of, for example, the following:

K370E/D399K/K439D/D356K/E357K/K409D;
K409D/D399K;
K409E/D399K;
K409E/D399R;
K409D/D399R;
D339K/E356K;
D399K/E356K/K409D/K392D;
D399K/E356K/K409D/K439D;
D399K/E357K/K409D/K370D;
D399K/E356K/E357K/K409D/K392D/K370D;
D399K/E357K/K409D/K392D;
K392D/K409D/D399K; and
K409D/K360D/D399K.

In some embodiments, Fc1 and/or Fc2 comprise(s) domains from different antibody subtypes, e.g., CH3 domains from different antibody subtypes.

In addition, the present disclosure cites the scheme of modifying the CH3 region of the Fc region to enhance heterodimerization in WO96/27011, WO98/050431, EP1870459, WO2007/110205, WO2007/147901, WO2009/089004, WO2010/129304, WO2011/90754, WO2011/143545, WO2012058768, WO2013157954, and WO2013096291.

In some embodiments, Fc1 and/or Fc2 comprise(s) amino acid mutations for altering effector functions in, for example, the CH3 domain(s). Examples of the antibody effector functions include, but are not limited to: C1q binding and complement-dependent cytotoxicity, Fc receptor (e.g., FcγR) binding, antibody-dependent cellular cytotoxicity (ADCC), phagocytosis, down-regulation of cell surface receptors (e.g., B-cell receptor), and B cell activation. The amino acid mutations for altering effector functions are selected from the group consisting of, for example, the following:

S298A/E333A/K334A;
S239D/I332E/A330L;
S239D/I332E/G236A;
G236A/S239D/A330L/I332E;
F243L/R292P/Y300L/V305I/P396L;
K326A/E333A;
K326W/E333S;
K326M/E333S;
C221D/D222C;
S267E/H268F/S324T;
E345R;
S298A/E333A/K334A/N434A;
E294 deletion/T307P/N434Y;
T256N/A378V/S383N/N434Y;
T252L/T253S/T254F;
M252Y/S254T/T256E;
M428L/N434S;
L234A/L235A;
S228P/L235E;
L234A/L235A/P331S;
L234A/L235A/P329G;
D265A/E233P;
H268Q/V309L/A330S/P331S;
V234A/G237A/P238S/H268A/V309L/A300S/P331S;
L234A/L235A/G237A/P238S/H268A/V309L/A300S/P331S;
S228P/F234A/L235A;
D270A/P329A;
L234F/L235E;
L234F/L235E/P331S;
F241A/V264A/D265A;
N297G/D265A; and
L234Y/G236W/S298A.

In some specific embodiments, the Fc1 and/or the Fc2 comprises mutations that reduce an effector function (e.g., ADCC), e.g., L234A/L235A or L234A/L235A/P329G.

In some embodiments, Fc1 and Fc2 comprise amino acid mutations for altering half-life (e.g., extending half-life) in, for example, the CH3 domains thereof. In some embodiments, the half-life is dependent on FcRn binding avidity. The extension of half-life can allow for a decrease in the amount of a drug given to a patient and/or a reduction in the frequency of administration. In some specific embodiments, the Fc region has mutation(s) M252Y, S254T, and/or T256E. In some embodiments, the immunoconjugate has a PEGylation modification or is conjugated/fused to (human serum) albumin or an albumin-binding protein.

In some embodiments, Fc1 and/or Fc2 comprise(s) one or more isoallotype mutations in, for example, the CH3 domain(s). In some embodiments, the isoallotype mutations are D356E and L358M.

In some embodiments, the immunoconjugate comprises a polypeptide chain combination of the following amino acid sequences: SEQ ID NOs: 21 and 22; SEQ ID NOs: 36 and 37; SEQ ID NOs: 38 and 39; SEQ ID NOs: 40 and 41; SEQ ID NOs: 42 and 43; or amino acid sequences having at least 80%, at least 85%, at least 90%, at least 95%, at least 96%, at least 97%, at least 98%, or at least 99% identity to any one of the above polypeptide chain combinations.

In some specific embodiments, the immunoconjugate comprises a polypeptide chain combination of the following amino acid sequences: SEQ ID NOs: 42 and 43, or amino acid sequences having at least 80%, at least 85%, at least 90%, at least 95%, at least 96%, at least 97%, at least 98%, or at least 99% identity to any one of the above polypeptide chain combinations.

Exemplary Immunoconjugates

Regarding immunoconjugates, the present disclosure provides the following embodiments:

Embodiment 1. An immunoconjugate, comprising:

1) a PD-1-binding domain, and an IL-2 variant, wherein the IL-2 variant comprises a mutation selected from the group consisting of N88D and D20A; or
2) a PD-1-binding domain, and an IL-2 variant, wherein the IL-2 variant has a reduced or eliminated avidity for the high-avidity receptors (IL-2R$\alpha$/$\beta$/$\gamma$) as compared to wild-type IL-2 and has a reduced avidity for the moderate-avidity receptors (IL-2R$\beta$/$\gamma$) as compared to wild-type IL-2, and the avidity for the high-avidity receptors is reduced more than the avidity for the moderate-avidity receptors; for example, the IL-2 variant comprises a mutation selected from the group consisting of N88D and D20A.

Embodiment 2. The immunoconjugate according to embodiment 1, wherein the immunoconjugate comprises a mutation selected from the group consisting of F42, Y45, and L72, or any combination thereof;

for example, the immunoconjugate comprises a mutation selected from the group consisting of F42A, Y45A, and L72G, or any combination thereof;
in another example, the immunoconjugate comprises mutations F42A/L72G, F42A/Y45A, Y45A/L72G, or F42A/Y45A/L72G.

Embodiment 3. The immunoconjugate according to embodiment 1 or 2, wherein the immunoconjugate comprises a mutation selected from the group consisting of N26Q, N29S, N30S, N71Q, Q11C and L132C, L70C and P82C, and G27C and F78C, or any combination thereof;
for example, the immunoconjugate comprises a mutation or combination selected from the group consisting of the following:
N26Q, N29S, N30S, N26Q/N29S/N71Q, N26Q/N29S, N26Q/N30S, N26Q/N30S/Q11C/L132C, N26Q/N29S/L70C/P82C, and N26Q/N30S/G27C/F78C.

Embodiment 4. An immunoconjugate, comprising:

an antigen-binding domain, and an IL-2 or a variant thereof, wherein the IL-2 variant comprises a first class of mutations that makes the IL-2 variant have a reduced avidity for the moderate-avidity receptors (IL-2R$\beta$/$\gamma$) as compared to wild-type IL-2;
for example, the first class of mutations is selected from the group consisting of L12, E15, H16, L18, L19, D20, M23, Q22, K43, V69, D84, S87, N88, V91, I92, E95, T123, Q126, S127, I129, and S130;
in another example, the first class of mutations is selected from the group consisting of L12R, L12K, L12E, L12Q, E15Q, E15R, E15A, E15S, H16A, H16E, H16D, H16G, H16S, H16T, H16V, H16P, D20A, D20H, D20Y, D20N, M23A, M23R, M23K, M23G, M23S, M23T, M23V, M23P, S87K, S87A, D84S, D84L, D84N, D84V, D84H, D84Y, D84R, D84K, D84G, D84A, D84T, D84P, N88D, N88A, N88S, N88T, N88R, N88I, V91A, V91T, V91E, I92A, E95S, E95A, E95R, E95Q, E95G, E95T, E95V, E95P, E95H, E95N, T123A, T123E, T123K, T123Q, Q126D, Q126L, Q126A, Q126S, Q126T, Q126E, S127A, S127E, S127K, and S127Q;
in another example, the first class of mutations is selected from the group consisting of N88D, N88R, D20A, D20N, V91T, Q126D, and H16A/D84S;
in yet another example, the first class of mutations is selected from the group consisting of N88D and D20A.

Embodiment 5. The immunoconjugate according to embodiment 4, wherein the IL-2 variant comprises a second class of mutations that makes the IL-2 variant have a reduced or eliminated avidity for the high-avidity receptors (IL-2R$\alpha$/$\beta$/$\gamma$) as compared to wild-type IL-2;

for example, the second class of mutations is selected from the group consisting of R38, E62, P65, E68, F42, Y45, and L72, and any combination thereof;
in another example, the second class of mutations is selected from the group consisting of R38A, R38D, R38E, E62Q, P65R, P65E, P65K, P65H, P65Y, P65Q, P65D, P65N, E68A, E68Q, E68K, E68R, F42A, F42G, F42S, F42T, F42Q, F42E, F42N, F42D, F42R, F42K, Y45A, Y45G, Y45S, Y45T, Y45Q, Y45E, Y45N, Y45D, Y45R, Y45K, L72G, L72A, L72S, L72T, L72Q, L72E, L72N, L72D, L72R, and L72K;

in yet another example, the second class of mutations is selected from the group consisting of F42A, Y45A, L72G, F42A/L72G, F42A/Y45A, Y45A/L72G, and F42A/Y45A/L72G.

Embodiment 6. The immunoconjugate according to embodiment 4 or 5, wherein the IL-2 variant comprises a third class of mutations that makes the IL-2 variant have increased stability as compared to wild-type IL-2;

for example, the third class of mutations is selected from the group consisting of N26, N29, N30, N71, Q11, L132, L70, P82, G27, and F78, and any combination thereof;

in another example, the third class of mutations is selected from the group consisting of N26Q, N29S, N30S, N71Q, Q11C/L132C, L70C/P82C, and G27C/F78C, and any combination thereof;

in yet another example, the third class of mutations is selected from the group consisting of N26Q, N29S, N30S, N26Q/N29S/N71Q, N26Q/N29S, N26Q/N30S, N26Q/N30S/Q11C/L132C, N26Q/N29S/L70C/P82C, and N26Q/N30S/G27C/F78C.

Embodiment 7. An immunoconjugate, comprising a PD-1-binding domain, and an IL-2 or a variant thereof, wherein

the PD-1-binding domain comprises at least one immunoglobulin single variable domain comprising: a CDR1, a CDR2, and a CDR3 from the amino acid sequence set forth in any one of SEQ ID NOs: 2 and 8-15; the CDR1, the CDR2, and the CDR3 are defined according to the Kabat, IMGT, Chothia, AbM, or Contact numbering scheme; for example, according to the Kabat numbering scheme, the amino acid sequences of the CDR1, the CDR2, and the CDR3 of the immunoglobulin single variable domain are set forth in SEQ ID NOs: 3, 16, and 17, respectively; in another example, the amino acid sequence of the CDR1 of the immunoglobulin single variable domain is set forth in SEQ ID NO: 3, the amino acid sequence of the CDR2 is set forth in SEQ ID NO: 4 or 18, and the amino acid sequence of the CDR3 is set forth in any one of SEQ ID NOs: 5 and 19-20; in yet another example, the amino acid sequences of the CDR1, the CDR2, and the CDR3 of the immunoglobulin single variable domain are set forth in SEQ ID NOs: 7, 18, and 19, respectively.

Embodiment 8. The immunoconjugate according to any one of embodiments 1 to 7, wherein the IL-2 variant comprises a mutation combination selected from the group consisting of groups 1) and 2):

group 1) mutations are selected from the group consisting of F42A/L72G, F42A/Y45A, Y45A/L72G, and F42A/Y45A/L72G;

group 2) mutations are selected from the group consisting of N88R, N88G, N88I, N88D, D20H, D20Y, V91T, Q126L, Q126D, and H16A/D84S;

for example, the IL-2 variant comprises the following mutation combination: F42A/L72G/N88D, F42A/L72G/N88R, F42A/L72G/D20A, F42A/L72G/D20N, F42A/L72G/V91T, F42A/L72G/Q126D, or F42A/L72-G/H16A/D84S.

Embodiment 9. The immunoconjugate according to embodiment 8, wherein the IL-2 variant further comprises group 3) mutations, and the group 3) mutations are selected from the group consisting of N26Q, N29S, N30S, N26Q/N29S/N71Q, N26Q/N29S, N26Q/N30S, N26Q/N30S/Q11C/L132C, N26Q/N29S/L70C/P82C, and N26Q/N30S/G27C/F78C;

for example, the IL-2 variant comprises the following mutation combination:

F42A/L72G/N88D/N26Q/N29S/N71Q,
F42A/L72G/N88R/N26Q/N29S/N71Q,
F42A/L72G/D20A/N26Q/N29S/N71Q,
F42A/L72G/D20N/N26Q/N29S/N71Q,
F42A/L72G/V91T/N26Q/N29S/N71Q,
F42A/L72G/Q126D/N26Q/N29S/N71Q, or
F42A/L72G/H16A/D84S/N26Q/N29S/N71Q.

Embodiment 10. The immunoconjugate according to any one of embodiments 1 to 9, comprising a mutation selected from the group consisting of T3 and/or C125 or a combination thereof,
for example, mutation(s) T3A and/or C125A, C125S, C125T, or C125V.

Embodiment 11. The immunoconjugate according to any one of embodiments 1 to 10, wherein the IL-2 variant comprises the amino acid sequence set forth in any one of SEQ ID NOs: 23-31. Embodiment 12. The immunoconjugate according to any one of embodiments 4 to 6 and 8 to 10, wherein

1) the antigen is selected from the group consisting of:

1-1) antigens on tumor cells or on immune cells in the tumor microenvironment;
1-2) PD-1, CTLA-4, LAG3, TIM3, 4-1BB, OX40, GITR, CD8a, CD8b, CD4, NKp30, NKG2A, TIGIT, TGFβR1, TGFβR2, Fas, NKG2D, NKp46, PD-L1, CD107a, ICOS, TNFR2, FAP, TNC A1, TNC A2, CEA, EDB, MCSP, or CD16a; and
1-3) immune checkpoint antigens, such as PD-1, PD-L1, and CTLA-4;

2) the antigen-binding domain comprises a VH domain and a VL domain, or a HCDR1, a HCDR2, and a HCDR3 of a VH and a LCDR1, a LCDR2, and a LCDR3 of a VL, of an antibody selected from the group consisting of the following:

PD-1 antibodies (e.g., pembrolizumab, nivolumab, sintilimab, tislelizumab, cemiplimab, toripalimab, camrelizumab, penpulimab, zimberelimab, sasanlimab, spartalizumab, retifanlimab, balstilimab, cetrelimab, dostarlimab (TSR-042), CS-1003, SCT-I10A, SSI-361, MEDI-0680(AMP-514), BAT-1306, JTX-4014, JTX-4014, BI-754091, and AK-103);
CTLA-4 antibodies (e.g., tremelimumab and ipilimumab);
4-1BB antibodies (e.g., urelumab and utomilumab); and
PD-L1 antibodies (e.g., durvalumab, atezolizumab, durvalumab, avelumab, adebrelimab, envafolimab, STI-A1110, CS-1001, TQB-2450, KL-A167, IMC-001, and CX-072).

Embodiment 13. The immunoconjugate according to any one of embodiments 1 to 3 and 12, wherein the PD-1-binding domain comprises at least one immunoglobulin single variable domain comprising:

a CDR1, a CDR2, and a CDR3 from the amino acid sequence set forth in any one of SEQ ID NOs: 2 and 8-15; the CDR1, the CDR2, and the CDR3 are defined according to the Kabat, IMGT, Chothia, AbM, or Contact numbering scheme;
for example, according to the Kabat numbering scheme, the amino acid sequences of the CDR1, the CDR2, and the CDR3 of the immunoglobulin single variable domain are set forth in SEQ ID NOs: 3, 16, and 17, respectively;
in another example, the amino acid sequence of the CDR1 of the immunoglobulin single variable domain is set forth in SEQ ID NO: 3, the amino acid sequence of the CDR2 is set forth in SEQ ID NO: 4 or 18, and the amino acid sequence of the CDR3 is set forth in any one of SEQ ID NOs: 5 and 19-20;
in yet another example, the amino acid sequences of the CDR1, the CDR2, and the CDR3 of the immunoglobulin single variable domain are set forth in SEQ ID NOs: 3, 18, and 19, respectively, SEQ ID NOs: 3, 4, and 5, respectively, or SEQ ID NOs: 3, 4, and 20, respectively.

Embodiment 14. The immunoconjugate according to embodiment 7 or 13, wherein the immunoglobulin single variable domain is:

1) engineered by humanization, avidity maturation, T cell epitope removal, reduction of antibody deamidation, and/or reduction of antibody isomerization; for example, the heavy chain framework region of the human germline template used in the humanization engineering process is IGHV3-23*01 or IGHV3-23*04;
2) a camelid antibody, a chimeric antibody, a humanized antibody, a fully human antibody, or an antigen-binding fragment thereof.

Embodiment 15. The immunoconjugate according to embodiment 13 or 14, wherein the amino acid sequence of the immunoglobulin single variable domain is

set forth in any one of SEQ ID NOs: 2 and 8-15; or
has at least 90% sequence identity to any one of the aforementioned sequences.

Embodiment 17. The immunoconjugate according to any one of the preceding embodiments, wherein the immunoconjugate further comprises a human immunoglobulin Fc region; for example, the Fc region is an Fc region of human IgG1, IgG2, IgG3, or IgG4.

Embodiment 18. The immunoconjugate according to embodiment 17, wherein the Fc region of human IgG1 comprises one or more amino acid mutations that reduce binding to Fc receptors (FcRs, e.g., FcγRs) and/or reduce an effector function;

for example, the Fc region of human IgG1 comprises one or more amino acid mutations that reduce binding to Fcγ receptors (FcγRs) and/or reduce ADCC effects;

in another example, the Fc region of human IgG1 comprises a mutation of L234, L235, or P329, or any combination thereof;

in yet another example, the Fc region of human IgG1 comprises mutations L234A/L235A or L234A/L235A/P329G.

Embodiment 19. The immunoconjugate according to embodiment 17 or 18, wherein the Fc region comprises mutations that promote the association (or heterodimerization) of a first subunit and a second subunit of the Fc region; for example, an amino acid residue in a CH3 region of the first subunit of the Fc region is replaced with an amino acid residue having a larger side-chain volume, thereby forming a protuberance within the CH3 region of the first subunit that is positionable in a cavity within a CH3 region of the second subunit, and an amino acid residue in the CH3 region of the second subunit of the Fc region is replaced with an amino acid residue having a smaller side-chain volume, thereby forming a cavity within the CH3 region of the second subunit in which the protuberance within the CH3 region of the first subunit is positionable; in another example, the amino acid residue having a larger side-chain volume is selected from the group consisting of R, F, Y, and W, and the amino acid residue having a smaller side-chain volume is selected from the group consisting of A, S, T, and V.

Embodiment 20. The immunoconjugate according to embodiment 19, wherein

the first subunit of the Fc region comprises a T366 mutation, and the second subunit comprises a mutation selected from the group consisting of T366, L368, and Y407, and any combination thereof;

the first subunit of the Fc region comprises an S354 or E356 mutation, and the second subunit comprises a mutation selected from the group consisting of Y349; or

the first subunit of the Fc region comprises an S354 mutation and a T366 mutation, and the second subunit comprises a Y349 mutation, a T366 mutation, an L368 mutation, and a Y407 mutation;

for example,

the first subunit of the Fc region comprises mutation T366W, and the second subunit comprises a mutation selected from the group consisting of T366S, L368A, and Y407V, and any combination thereof;

the first subunit of the Fc region comprises mutation S354C or E356C, and the second subunit comprises a mutation selected from the group consisting of Y349C; or

the first subunit of the Fc region comprises mutations S354C/T366W, and the second subunit comprises mutations Y349C/T366S/L368A/Y407V.

Embodiment 21. The immunoconjugate according to embodiment 19 or 20, wherein the second subunit of the Fc region further comprises an H435 mutation and/or an R436 mutation, preferably mutations H435R/Y436F, mutation H435R, or mutation H435K.

Embodiment 22. The immunoconjugate according to any one of embodiments 17 to 21, wherein the Fc region of human IgG1 comprises a mutation that enhances FcRn-mediated recycling and/or increases half-life;

for example, the Fc region of human IgG1 comprises an M252 mutation and/or an M428 mutation; for example, the Fc region of human IgG1 comprises mutations M252Y/M428V or M252Y/M428L.

Embodiment 23. The immunoconjugate according to any one of the preceding embodiments, wherein the PD-1-binding domain or the antigen-binding domain is located at the N-terminus of the Fc region, and the IL-2 variant is located at the N-terminus or C-terminus of the Fc region;

for example, the PD-1-binding domain or the antigen-binding domain is located at the N-terminus of the Fc region, and the IL-2 variant is located at the C-terminus of the Fc region.

Embodiment 24. The immunoconjugate according to any one of the preceding embodiments, wherein the valence ratio of the PD-1-binding domain or the antigen-binding domain to IL-2 or the variant thereof is between 6:1 and 1:3 (e.g., 4:1 to 1:2), preferably 4:1, 2:1, or 1:1; or one immunoconjugate comprises 1 or 2 IL-2 or variants thereof and 1, 2, 3, 4, 5, or 6 PD-1-binding domains or antigen-binding domains.

Embodiment 25. The immunoconjugate according to any one of embodiments 1 to 3, 7, and 11 to 23, comprising a polypeptide chain combination set forth in any one of the following I)-VI):

a first polypeptide chain: [PD-1-binding domain 1]-[the first subunit of the Fc region]-[linker 1]a-[IL-2 or the variant thereof], and

a second polypeptide chain: [PD-1-binding domain 1]-[the second subunit of the Fc region],

wherein - represents a peptide bond, the linker is a polypeptide capable of fulfilling the function of linking, and a is selected from the group consisting of 0 and 1; [PD-1-binding domain 1] is selected from the group consisting of any of the PD-1-binding domains according to embodiments 11 to 13;

for example, the linker is a $(G_xS)_y$ linker, wherein x is selected from the group consisting of integers from 1 to 5, and y is selected from the group consisting of integers from 1 to 6; in another example, linker 1 is $(G_4S)_3$, linker 2 is $(G_4S)_2$, and linker 3 is $G_4S$;

some other linkers include, but are not limited to, amino acid sequences represented by $(G_mS_n)_h$ or $(GGNGT)_h$ or $(YGNGT)_h$ or $(EPKSS)_h$, wherein m and n are each independently selected from the group consisting of integers from 1 to 8 (e.g., 1, 2, 3, 4, 5, 6, 7, and 8), and h is independently selected from the group consisting of integers from 1 to 20 (e.g., 1, 2, 3, 4, 5, 6, 7, 8, 9, 10, 11, 12, 13, 14, 15, 16, 17, 18, 19, and 20);

the order of the above polypeptide chains is from the N-terminus to the C-terminus.

Embodiment 26. The immunoconjugate according to any one of the preceding embodiments, comprising a polypeptide chain combination of the following amino acid sequences:

SEQ ID NOs: 42 and 43,
SEQ ID NOs: 21 and 22,
SEQ ID NOs: 36 and 37,
SEQ ID NOs: 38 and 39, or
SEQ ID NOs: 40 and 41;

or amino acid sequences having at least 80%, at least 85%, at least 90%, at least 95%, at least 96%, at least 97%, at least 98%, or at least 99% identity to any one of the above polypeptide chain combinations.

Unless otherwise specified, the mutation sites in the Fc regions described above are numbered according to the EU numbering scheme.

The present disclosure provides a fusion protein that comprises or is any of the aforementioned immunoconjugates.

In some embodiments, the pharmaceutical composition according to any one of the foregoing further comprises a solvent. Illustratively, the solvent in the pharmaceutical composition is selected from the group consisting of, but is not limited to, non-toxic physiologically acceptable liquid carriers, such as normal saline, water for injection, and glucose solutions (e.g., 5% glucose injections and glucose-sodium chloride injections).

In some embodiments, the pharmaceutical composition according to any one of the foregoing may be an intravenous injection, a subcutaneous injection, an intraperitoneal injection, or an intramuscular injection.

The present disclosure further provides a liquid formulation comprising the pharmaceutical composition according to any one of the foregoing.

The pharmaceutical composition of the present disclosure already has sufficient stability for being prepared into a drug and can remain stable during long-term storage.

The present disclosure further provides a method for preparing a freeze-dried formulation of a pharmaceutical composition comprising an immunoconjugate, and the method comprises a step of freeze-drying the aforementioned pharmaceutical composition.

The present disclosure further provides a freeze-dried formulation of a pharmaceutical composition comprising an immunoconjugate, and the formulation is obtained by freeze-drying a liquid formulation comprising the pharmaceutical composition.

In some embodiments, the liquid formulation comprises:

(e-1) about 10 mg/mL to about 80 mg/mL immunoconjugate,

about 5 mM to about 10 mM succinic acid-histidine,
about 0.04% (w/v) to about 0.08% (w/v) polysorbate 80,
about 4% (w/v) to about 10% (w/v) trehalose, and
about 10 mM to about 100 mM arginine hydrochloride, which is optionally present,
the pharmaceutical composition having a pH of about 4.0 to about 4.5; or

(e-1-1) about 10 mg/mL to about 80 mg/mL immunoconjugate,

about 5 mM to about 10 mM succinic acid-histidine,
about 0.04% (w/v) to about 0.08% (w/v) polysorbate 80, and

about 4% (w/v) to about 10% (w/v) trehalose,
the pharmaceutical composition having a pH of about 4.0 to about 4.5; or

(e-1-2) about 10 mg/mL to about 80 mg/mL immunoconjugate,

about 5 mM to about 10 mM succinic acid-histidine,
about 0.04% (w/v) to about 0.08% (w/v) polysorbate 80,
about 4% (w/v) to about 10% (w/v) trehalose, and
about 10 mM to about 100 mM arginine hydrochloride,
the pharmaceutical composition having a pH of about 4.0 to about 4.5; or

(e-2) about 10 mg/mL to about 40 mg/mL immunoconjugate,

about 10 mM succinic acid-histidine,
about 0.04% (w/v) to about 0.08% (w/v) polysorbate 80, and
about 4% (w/v) to about 8.8% (w/v) trehalose,
the pharmaceutical composition having a pH of about 4.0 to about 4.5; or

(f-1) about 10 mg/mL to about 80 mg/mL immunoconjugate,

about 5 mM to about 10 mM succinic acid-histidine,
about 0.1% (w/v) to about 0.2% (w/v) polysorbate 80,
about 4% (w/v) to about 8% (w/v) sucrose, and
about 10 mM to about 100 mM arginine hydrochloride, which is optionally present,
the pharmaceutical composition having a pH of about 4.5 to about 5.0; or

(f-1-1) about 10 mg/mL to about 80 mg/mL immunoconjugate,

about 5 mM to about 10 mM succinic acid-histidine,
about 0.1% (w/v) to about 0.2% (w/v) polysorbate 80, and
about 4% (w/v) to about 8% (w/v) sucrose,
the pharmaceutical composition having a pH of about 4.5 to about 5.0; or

(f-1-2) about 10 mg/mL to about 80 mg/mL immunoconjugate,

about 5 mM to about 10 mM succinic acid-histidine,
about 0.1% (w/v) to about 0.2% (w/v) polysorbate 80,
about 4% (w/v) to about 8% (w/v) sucrose, and
about 10 mM to about 100 mM arginine hydrochloride,
the pharmaceutical composition having a pH of about 4.5 to about 5.0; or

(f-2) about 40 mg/mL to about 80 mg/mL immunoconjugate,

about 5 mM succinic acid-histidine,
about 0.1% (w/v) to about 0.2% (w/v) polysorbate 80, and
about 4% (w/v) to about 8% (w/v) sucrose,
the pharmaceutical composition having a pH of about 4.5 to about 5.0.

In some embodiments, the liquid formulation comprises any one of the following:

(1) about 10 mg/mL to about 120 mg/mL immunoconjugate,

about 10 mM succinic acid-histidine,
about 0.04% (w/v) polysorbate 80, and
about 4.4% (w/v) trehalose,
the pharmaceutical composition having a pH of about 4.0 to about 4.5;

(2) about 10 mg/mL to about 80 mg/mL immunoconjugate,

about 10 mM succinic acid-histidine,
about 0.04% (w/v) polysorbate 80, and
about 4.4% (w/v) trehalose,
the pharmaceutical composition having a pH of about 4.0 to about 4.5;

(3) about 10 mg/mL to about 40 mg/mL immunoconjugate,

about 10 mM succinic acid-histidine,
about 0.04% (w/v) polysorbate 80, and
about 4.4% (w/v) trehalose,
the pharmaceutical composition having a pH of about 4.5;

(7) about 40 mg/mL immunoconjugate,

about 10 mM succinic acid-histidine,
about 0.04% (w/v) polysorbate 80, and
about 4.4% (w/v) trehalose,
the pharmaceutical composition having a pH of about 4.5;

(8) about 40 mg/mL immunoconjugate,

about 10 mM succinic acid-histidine,
about 0.08% (w/v) polysorbate 80, and
about 8% (w/v) trehalose,
the pharmaceutical composition having a pH of about 4.5;

(9) about 40 mg/mL immunoconjugate,

about 10 mM succinic acid-histidine,
about 0.04% (w/v) polysorbate 80, and
about 8% (w/v) trehalose,
the pharmaceutical composition having a pH of about 4.0;

(10) about 40 mg/mL immunoconjugate,

about 10 mM succinic acid-histidine,
about 0.08% (w/v) polysorbate 80, and
about 5% (w/v) trehalose,
the pharmaceutical composition having a pH of about 4.0;

(11) about 40 mg/mL immunoconjugate,

about 10 mM succinic acid-histidine,
about 0.08% (w/v) polysorbate 80, and
about 8% (w/v) trehalose,
the pharmaceutical composition having a pH of about 4.0; and

(12) about 40 mg/mL immunoconjugate,

about 10 mM succinic acid-histidine,
about 0.08% (w/v) polysorbate 80,
about 8% (w/v) trehalose, and
about 50 mM arginine hydrochloride,
the pharmaceutical composition having a pH of about 4.0.

In some embodiments, provided is the freeze-dried formulation according to any one of the above, wherein the PD-1-binding domain in the immunoconjugate comprises at least one immunoglobulin single variable domain comprising a CDR1, a CDR2, and a CDR3 from the amino acid sequence set forth in any one of SEQ ID NOs: 2 and 8-15; the CDR1, the CDR2, and the CDR3 are defined according to the Kabat, IMGT, Chothia, AbM, or Contact numbering scheme.

In some embodiments, the amino acid sequences of the CDR1, the CDR2, and the CDR3 of the immunoglobulin single variable domain are set forth in SEQ ID NOs: 3, 16, and 17, respectively.

In some embodiments, the amino acid sequence of the CDR1 of the immunoglobulin single variable domain in the aforementioned PD-1-binding domain is set forth in SEQ ID NO: 3, the amino acid sequence of the CDR2 is set forth in SEQ ID NO: 4 or 18, and the amino acid sequence of the CDR3 is set forth in any one of SEQ ID NOs: 5 and 19-20.

In some embodiments, the amino acid sequences of the CDR1, the CDR2, and the CDR3 of the immunoglobulin single variable domain in the aforementioned PD-1-binding domain are set forth in SEQ ID NOs: 3, 18, and 19, SEQ ID NOs: 3, 4, and 5, or SEQ ID NOs: 3, 4, and 20, respectively. In some embodiments, the cytokine is IL-2 or a variant thereof. In some specific embodiments, the IL-2 variant comprises the amino acid sequence set forth in any one of SEQ ID NOs: 23-31 or an amino acid sequence having at least 90% sequence identity thereto.

In some embodiments, the immunoconjugate comprises a polypeptide chain combination of the following amino acid sequences:

SEQ ID NOs: 42 and 43,
SEQ ID NOs: 21 and 22,
SEQ ID NOs: 36 and 37,
SEQ ID NOs: 38 and 39, or
SEQ ID NOs: 40 and 41.

In some embodiments, the freeze-dried formulation is stored in a dark place at 2-8 °C and is stable for at least 1 month, at least 3 months, at least 6 months, at least 12 months, at least 18 months, at least 24 months, or at least 30 months.

In some embodiments, the freeze-dried formulation is stable at 25 °C for at least 1 month, at least 3 months, at least 6 months, or at least 12 months.

In some embodiments, the freeze-dried formulation is stable at 40 °C for at least 7 days, at least 14 days, or at least 30 days.

The freeze-dried formulation provided by the present disclosure dissolves quickly, and solutions obtained by reconstituting the freeze-dried formulation have good appearances and good stability. The present disclosure further provides a reconstituted solution comprising an immunoconjugate, and the reconstituted solution is prepared by reconstituting the freeze-dried formulation according to any one of the foregoing.

In some embodiments, the reconstituted solution is obtained by reconstituting the freeze-dried formulation according to any one of the foregoing with a solvent. In some embodiments, the solvent includes, but is not limited to, water, normal saline, glucose, etc.; for example, the solvent is water for injection.

In some embodiments, a concentration of the immunoconjugate in the reconstituted solution is greater than a concentration of the immunoconjugate in a stock solution for preparing the freeze-dried formulation. In some embodiments, the ratio of the concentration of the immunoconjugate in the reconstituted solution to the concentration of the immunoconjugate in the stock solution is (1-5):1, e.g., 1:1, 1.5:1, 1.6:1, 1.7:1, 1.8:1, 1.9:1, 2.0:1, 2.1:1, 2.2:1, 2.3:1, 2.4:1, 2.5:1, 2.6:1, 2.7:1, 2.8:1, 2.9:1, 3.0:1, 3.5:1, 4:1, 4.5:1, 5:1, or any range between these point values. In some embodiments, the reconstituted solution comprises:

(e-3) about 40 mg/mL to about 120 mg/mL immunoconjugate,

about 10 mM to about 20 mM succinic acid-histidine,
about 0.04% (w/v) to about 0.08% (w/v) polysorbate 80, and
about 4% (w/v) to about 10% (w/v) trehalose,
the pharmaceutical composition having a pH of about 4.0 to about 4.5; or

(e-4) about 40 mg/mL to about 80 mg/mL immunoconjugate,

about 20 mM succinic acid-histidine,
about 0.04% (w/v) to about 0.08% (w/v) polysorbate 80, and
about 4.4% (w/v) to about 8.8% (w/v) trehalose,
the pharmaceutical composition having a pH of about 4.0 to about 4.5; or

(f-3) about 40 mg/mL to about 120 mg/mL immunoconjugate,

about 10 mM to about 20 mM succinic acid-histidine,

about 0.1% (w/v) to about 0.2% (w/v) polysorbate 80, and
about 4% (w/v) to about 8% (w/v) sucrose,
the pharmaceutical composition having a pH of about 4.5 to about 5.0; or

(f-4) about 80 mg/mL to about 120 mg/mL immunoconjugate,

about 10 mM succinic acid-histidine,
about 0.1% (w/v) to about 0.2% (w/v) polysorbate 80, and
about 4% (w/v) to about 8% (w/v) sucrose,
the pharmaceutical composition having a pH of about 4.5 to about 5.0; or

(f-5) about 80 mg/mL to about 120 mg/mL immunoconjugate,

about 10 mM succinic acid-histidine,
about 0.2% (w/v) polysorbate 80, and
about 8% (w/v) sucrose,
the pharmaceutical composition having a pH of about 4.8; or

(f-6) about 80 mg/mL to about 120 mg/mL immunoconjugate,

about 10 mM succinic acid-histidine,
about 0.2% (w/v) polysorbate 80, and
about 8% (w/v) sucrose,
the pharmaceutical composition having a pH of about 5.0.

In some embodiments, the reconstituted solution comprises any one of the following:

(4) about 10 mg/mL to about 120 mg/mL immunoconjugate,

about 20 mM succinic acid-histidine,
about 0.08% (w/v) polysorbate 80, and
about 8.8% (w/v) trehalose,
the pharmaceutical composition having a pH of about 4.0 to about 4.5;

(5) about 40 mg/mL to about 120 mg/mL immunoconjugate,

about 20 mM succinic acid-histidine,
about 0.08% (w/v) polysorbate 80, and
about 8.8% (w/v) trehalose,
the pharmaceutical composition having a pH of about 4.0 to about 4.5;

(6) about 40 mg/mL to about 80 mg/mL immunoconjugate,

about 20 mM succinic acid-histidine,
about 0.08% (w/v) polysorbate 80, and
about 8.8% (w/v) trehalose,
the pharmaceutical composition having a pH of about 4.5; and

(13) about 80 mg/mL immunoconjugate,

about 20 mM succinic acid-histidine,
about 0.08% (w/v) polysorbate 80, and
about 8.8% (w/v) trehalose,
the pharmaceutical composition having a pH of about 4.5.

In some embodiments, provided is the reconstituted solution according to any one of the above, wherein the PD-1-binding domain in the immunoconjugate comprises at least one immunoglobulin single variable domain comprising a CDR1, a CDR2, and a CDR3 from the amino acid sequence set forth in any one of SEQ ID NOs: 2 and 8-15; the CDR1, the CDR2, and the CDR3 are defined according to the Kabat, IMGT, Chothia, AbM, or Contact numbering scheme.

In some embodiments, the amino acid sequences of the CDR1, the CDR2, and the CDR3 of the immunoglobulin single variable domain are set forth in SEQ ID NOs: 3, 16, and 17, respectively.

In some embodiments, the amino acid sequence of the CDR1 of the immunoglobulin single variable domain in the aforementioned PD-1-binding domain is set forth in SEQ ID NO: 3, the amino acid sequence of the CDR2 is set forth in SEQ ID NO: 4 or 18, and the amino acid sequence of the CDR3 is set forth in any one of SEQ ID NOs: 5 and 19-20.

In some embodiments, the amino acid sequences of the CDR1, the CDR2, and the CDR3 of the immunoglobulin single variable domain in the aforementioned PD-1-binding domain are set forth in SEQ ID NOs: 3, 18, and 19, SEQ ID NOs: 3, 4, and 5, or SEQ ID NOs: 3, 4, and 20, respectively.

In some embodiments, the cytokine is IL-2 or a variant thereof. In some specific embodiments, the IL-2 variant comprises the amino acid sequence set forth in any one of SEQ ID NOs: 23-31 or an amino acid sequence having at least 90% sequence identity thereto.

In some embodiments, the immunoconjugate comprises a polypeptide chain combination of the following amino acid sequences:

SEQ ID NOs: 42 and 43,
SEQ ID NOs: 21 and 22,
SEQ ID NOs: 36 and 37,
SEQ ID NOs: 38 and 39, or
SEQ ID NOs: 40 and 41.

In some embodiments, the present disclosure surprisingly finds that freeze-dried formulations prepared using (e-1), (e-2), (f-1), (f-2), (1)-(3), and (7)-(12) as stock solutions exhibit relatively good stability after 4 W of standing at 40 °C, neither the SEC monomer purity nor the NR-CE main peak purity significantly changes, and the freeze-dried formulations have relatively good appearances. Moreover, the reconstitution time is short, and the reconstituted (e-3), (e-4), (f-3), (f-4), (4)-(6), and (13) all have relatively good reconstitution appearances and insoluble particle and water contents after reconstitution, effectively solving the problem that in the process of developing an immunoconjugate (e.g., the fusion protein of the PD-1-binding domain and the IL-2 variant), the immunoconjugate exhibits poor stability at the high temperature of 40 °C, particularly the problem that the SEC monomer purity decreases significantly when the concentration of the immunoconjugate is greater than 50 mg/mL.

The present disclosure provides a method for preparing a liquid formulation, comprising the following steps:

1) preparing a stock solution comprising a pharmaceutical composition, and freeze-drying the stock solution to obtain a freeze-dried formulation; and
2) reconstituting the freeze-dried formulation to obtain a reconstituted solution.

In some embodiments, the ratio of a concentration of an immunoconjugate in the reconstituted solution to a concentration of the immunoconjugate in the stock solution is (1-5):1, e.g., 1:1, 1.5:1, 1.6:1, 1.7:1, 1.8:1, 1.9:1, 2.0:1, 2.1:1, 2.2:1, 2.3:1, 2.4:1, 2.5:1, 2.6:1, 2.7:1, 2.8:1, 2.9:1, 3.0:1, 3.5:1, 4:1, 4.5:1, 5:1, or any range between these point values.

In some embodiments, the method for preparing a liquid formulation comprises:

1) preparing a stock solution comprising a pharmaceutical composition, and freeze-drying the stock solution to obtain a freeze-dried formulation; and
2) reconstituting the freeze-dried formulation with a solvent to obtain the liquid formulation.

The present disclosure provides a liquid formulation comprising any one of the following:

(e) about 10 mg/mL to about 120 mg/mL immunoconjugate,

about 5 mM to about 20 mM succinic acid-histidine,
about 0.04% (w/v) to about 0.08% (w/v) polysorbate 80,
about 1% (w/v) to about 10% (w/v) trehalose, and
about 10 mM to about 100 mM arginine hydrochloride, which is optionally present,
the pharmaceutical composition having a pH of about 4.0 to about 4.5; or

(e-1) about 10 mg/mL to about 80 mg/mL immunoconjugate,

about 5 mM to about 10 mM succinic acid-histidine,

about 0.04% (w/v) to about 0.08% (w/v) polysorbate 80,
about 4% (w/v) to about 10% (w/v) trehalose, and
about 10 mM to about 100 mM arginine hydrochloride, which is optionally present,
the pharmaceutical composition having a pH of about 4.0 to about 4.5; or

(e-1-1) about 10 mg/mL to about 80 mg/mL immunoconjugate,

about 5 mM to about 10 mM succinic acid-histidine,
about 0.04% (w/v) to about 0.08% (w/v) polysorbate 80, and
about 4% (w/v) to about 10% (w/v) trehalose,
the pharmaceutical composition having a pH of about 4.0 to about 4.5; or

(e-1-2) about 10 mg/mL to about 80 mg/mL immunoconjugate,

about 5 mM to about 10 mM succinic acid-histidine,
about 0.04% (w/v) to about 0.08% (w/v) polysorbate 80,
about 4% (w/v) to about 10% (w/v) trehalose, and
about 10 mM to about 100 mM arginine hydrochloride,
the pharmaceutical composition having a pH of about 4.0 to about 4.5; or

(e-2) about 10 mg/mL to about 40 mg/mL immunoconjugate,

about 10 mM succinic acid-histidine,
about 0.04% (w/v) to about 0.08% (w/v) polysorbate 80, and
about 4% (w/v) to about 8.8% (w/v) trehalose,
the pharmaceutical composition having a pH of about 4.0 to about 4.5; or

(e-3) about 40 mg/mL to about 120 mg/mL immunoconjugate,

about 10 mM to about 20 mM succinic acid-histidine,
about 0.04% (w/v) to about 0.08% (w/v) polysorbate 80, and
about 4% (w/v) to about 10% (w/v) trehalose,
the pharmaceutical composition having a pH of about 4.0 to about 4.5; or

(e-4) about 40 mg/mL to about 80 mg/mL immunoconjugate,

about 20 mM succinic acid-histidine,
about 0.04% (w/v) to about 0.08% (w/v) polysorbate 80, and
about 4.4% (w/v) to about 8.8% (w/v) trehalose,
the pharmaceutical composition having a pH of about 4.0 to about 4.5; or

(f-1) about 10 mg/mL to about 80 mg/mL immunoconjugate,

about 5 mM to about 10 mM succinic acid-histidine,
about 0.1% (w/v) to about 0.2% (w/v) polysorbate 80,
about 4% (w/v) to about 8% (w/v) sucrose, and
about 10 mM to about 100 mM arginine hydrochloride, which is optionally present,
the pharmaceutical composition having a pH of about 4.5 to about 5.0; or

(f-1-1) about 10 mg/mL to about 80 mg/mL immunoconjugate,

about 5 mM to about 10 mM succinic acid-histidine,
about 0.1% (w/v) to about 0.2% (w/v) polysorbate 80, and
about 4% (w/v) to about 8% (w/v) sucrose,
the pharmaceutical composition having a pH of about 4.5 to about 5.0; or

(f-1-2) about 10 mg/mL to about 80 mg/mL immunoconjugate,

about 5 mM to about 10 mM succinic acid-histidine,
about 0.1% (w/v) to about 0.2% (w/v) polysorbate 80,
about 4% (w/v) to about 8% (w/v) sucrose, and
about 10 mM to about 100 mM arginine hydrochloride,
the pharmaceutical composition having a pH of about 4.5 to about 5.0; or

(f-2) about 40 mg/mL to about 80 mg/mL immunoconjugate,

about 5 mM succinic acid-histidine,
about 0.1% (w/v) to about 0.2% (w/v) polysorbate 80, and
about 4% (w/v) to about 8% (w/v) sucrose,
the pharmaceutical composition having a pH of about 4.5 to about 5.0; or

(f-3) about 40 mg/mL to about 120 mg/mL immunoconjugate,

about 10 mM to about 20 mM succinic acid-histidine,
about 0.1% (w/v) to about 0.2% (w/v) polysorbate 80, and
about 4% (w/v) to about 8% (w/v) sucrose,
the pharmaceutical composition having a pH of about 4.5 to about 5.0; or

(f-4) about 80 mg/mL to about 120 mg/mL immunoconjugate,

about 10 mM succinic acid-histidine,
about 0.1% (w/v) to about 0.2% (w/v) polysorbate 80, and
about 4% (w/v) to about 8% (w/v) sucrose,
the pharmaceutical composition having a pH of about 4.5 to about 5.0; or

(f-5) about 80 mg/mL to about 120 mg/mL immunoconjugate,

about 10 mM succinic acid-histidine,
about 0.2% (w/v) polysorbate 80, and
about 8% (w/v) sucrose,
the pharmaceutical composition having a pH of about 4.8; or

(f-6) about 80 mg/mL to about 120 mg/mL immunoconjugate,

about 10 mM succinic acid-histidine,
about 0.2% (w/v) polysorbate 80, and
about 8% (w/v) sucrose,
the pharmaceutical composition having a pH of about 5.0.

In some embodiments, the liquid formulation comprises any one of the following:

(1) about 10 mg/mL to about 120 mg/mL immunoconjugate,

about 10 mM succinic acid-histidine,
about 0.04% (w/v) polysorbate 80, and
about 4.4% (w/v) trehalose,
the pharmaceutical composition having a pH of about 4.0 to about 4.5;

(2) about 10 mg/mL to about 80 mg/mL immunoconjugate,

about 10 mM succinic acid-histidine,
about 0.04% (w/v) polysorbate 80, and
about 4.4% (w/v) trehalose,
the pharmaceutical composition having a pH of about 4.0 to about 4.5;

(3) about 10 mg/mL to about 40 mg/mL immunoconjugate,

about 10 mM succinic acid-histidine,
about 0.04% (w/v) polysorbate 80, and
about 4.4% (w/v) trehalose,
the pharmaceutical composition having a pH of about 4.5;

(4) about 10 mg/mL to about 120 mg/mL immunoconjugate,

about 20 mM succinic acid-histidine,
about 0.08% (w/v) polysorbate 80, and
about 8.8% (w/v) trehalose,
the pharmaceutical composition having a pH of about 4.0 to about 4.5;

(5) about 40 mg/mL to about 120 mg/mL immunoconjugate,

about 20 mM succinic acid-histidine,
about 0.08% (w/v) polysorbate 80, and
about 8.8% (w/v) trehalose,
the pharmaceutical composition having a pH of about 4.0 to about 4.5;

(6) about 40 mg/mL to about 80 mg/mL immunoconjugate,

about 20 mM succinic acid-histidine,
about 0.08% (w/v) polysorbate 80, and
about 8.8% (w/v) trehalose,
the pharmaceutical composition having a pH of about 4.5;

(7) about 40 mg/mL immunoconjugate,

about 10 mM succinic acid-histidine,
about 0.04% (w/v) polysorbate 80, and
about 4.4% (w/v) trehalose,
the pharmaceutical composition having a pH of about 4.5;

(8) about 40 mg/mL immunoconjugate,

about 10 mM succinic acid-histidine,
about 0.08% (w/v) polysorbate 80, and
about 8% (w/v) trehalose,
the pharmaceutical composition having a pH of about 4.5;

(9) about 40 mg/mL immunoconjugate,

about 10 mM succinic acid-histidine,
about 0.04% (w/v) polysorbate 80, and
about 8% (w/v) trehalose,
the pharmaceutical composition having a pH of about 4.0;

(10) about 40 mg/mL immunoconjugate,

about 10 mM succinic acid-histidine,
about 0.08% (w/v) polysorbate 80, and
about 5% (w/v) trehalose,
the pharmaceutical composition having a pH of about 4.0;

(11) about 40 mg/mL immunoconjugate,

about 10 mM succinic acid-histidine,
about 0.08% (w/v) polysorbate 80, and

about 8% (w/v) trehalose,
the pharmaceutical composition having a pH of about 4.0;

(12) about 40 mg/mL immunoconjugate,

about 10 mM succinic acid-histidine,
about 0.08% (w/v) polysorbate 80,
about 8% (w/v) trehalose, and
about 50 mM arginine hydrochloride,
the pharmaceutical composition having a pH of about 4.0; and

(13) about 80 mg/mL immunoconjugate,

about 20 mM succinic acid-histidine,
about 0.08% (w/v) polysorbate 80, and
about 8.8% (w/v) trehalose,
the pharmaceutical composition having a pH of about 4.5.

In the present disclosure, a reconstituted solution with an increased protein concentration can be prepared by means of dilution-freezing-concentration-dissolution, and the reconstituted solution has a relatively good appearance and good stability.

The present disclosure further provides a product comprising a container containing the pharmaceutical composition, the liquid formulation, the freeze-dried formulation, or the reconstituted solution according to any one of the above. In some embodiments, the container may be, but is not limited to, a tubular injection vial made of neutral borosilicate glass.

The present disclosure further provides use of the pharmaceutical composition according to any one of the above, the liquid formulation according to any one of the above, the freeze-dried formulation according to any one of the above, the reconstituted solution according to any one of the above, or the product according to any one of the above in the manufacture of a medicament for treating a disease.

The present disclosure further provides a method for treating or preventing a disease, comprising administering to a subject in need thereof an effective amount of the pharmaceutical composition according to any one of the above, the liquid formulation according to any one of the above, the freeze-dried formulation according to any one of the above, the reconstituted solution according to any one of the above, or the product according to any one of the above.

The present disclosure further provides the pharmaceutical composition according to any one of the above, the liquid formulation according to any one of the above, the freeze-dried formulation according to any one of the above, the reconstituted solution according to any one of the above, or the product according to any one of the above for use in the treatment of a disease.

Treatment Method and Pharmaceutical Use

In some embodiments, the aforementioned disease is a proliferative disorder or any other disease or disorder characterized by uncontrolled cell growth (e.g., cancer; in the present disclosure, cancer and tumor are used interchangeably), e.g., a disease associated with PD-L1 overexpression, or a related disease responsive to treatment with a PD-1 antibody or an antigen-binding fragment thereof, e.g., a cancer.

In some embodiments, the aforementioned cancer is a solid tumor or hematological tumor.

In some embodiments, the aforementioned cancer is advanced or metastatic.

In some embodiments, the aforementioned cancer is selected from the group consisting of the following and combinations thereof: lung cancer, prostate cancer, breast cancer, head and neck cancer, esophageal cancer, stomach/-gastric cancer, colon cancer, colorectal cancer, bladder cancer, cervical cancer, uterine cancer, ovarian cancer, hepatocellular carcinoma, melanoma, renal cell carcinoma, squamous cell carcinoma, hematological cancer, and any other diseases or disorders characterized by uncontrolled cell growth.

For example, the cancer is selected from the group consisting of prostate cancer, hepatocellular carcinoma (HCC), colorectal cancer, ovarian cancer, endometrial cancer, breast cancer, triple-negative breast cancer, pancreatic cancer, stomach/gastric cancer, cervical cancer, head and neck cancer, thyroid cancer, testicular cancer, urothelial carcinoma, lung cancer (small cell lung cancer or non-small cell lung cancer), melanoma, non-melanoma skin cancer (squamous and basal cell carcinoma), neuroglioma, renal cell carcinoma (RCC), lymphoma (NHL or HL), acute myelogenous leukemia (AML), T-cell acute lymphoblastic leukemia (T-ALL), diffuse large B-cell lymphoma, testis germ cell tumor, mesothelioma, esophageal cancer, Merkel cells cancer, MSI-high cancer, KRAS-mutant tumor, adult T-cell leukemia/lymphoma, and myelodysplastic syndrome (MDS).

For example, the cancer is selected from the group consisting of basal cell carcinoma, biliary tract cancer, bladder cancer, bone cancer, brain and central nervous system cancer, breast cancer, peritoneal cancer, cervical cancer, choriocarcinoma, colorectal cancer, connective tissue cancer, digestive system cancer, endometrial cancer, esophageal cancer, eye cancer, head and neck cancer, stomach/gastric cancer, gastrointestinal cancer, glioblastoma, hepatocellular carcinoma, liver tumor, intraepithelial tumor, kidney cancer or renal cell carcinoma, laryngeal cancer, liver cancer, lung cancer, small cell lung cancer, non-small cell lung cancer, lung adenocarcinoma, lung squamous carcinoma, melanoma, myeloma, neuroblastoma, oral cancer, ovarian cancer, pancreatic cancer, prostate cancer, retinoblastoma, rhabdomyosarcoma, rectal cancer, respiratory system cancer, salivary gland cancer, sarcoma, skin cancer, squamous cell carcinoma, stomach/gastric cancer, testicular cancer, thyroid cancer, uterine or endometrial cancer, urinary system cancer, vulvar cancer, lymphoma, lymphoma, Hodgkin lymphoma, non-Hodgkin lymphoma, B-cell lymphoma, low-grade/follicular non-Hodgkin lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cleaved cell NHL, bulky disease NHL, mantle cell lymphoma, AIDS-related lymphoma, Waldenström macroglobulinemia, chronic lymphocytic leukemia (CLL), acute lymphoblastic leukemia (ALL), hairy cell leukemia, and chronic myeloblastic leukemia.

In some embodiments, the aforementioned subject has a PD-L1-associated condition. In some specific embodiments, the subject's condition includes cancers that abnormally express or do not abnormally express PD-L1, and further includes non-metastatic or non-infiltrative and infiltrative or metastatic cancers.

In some embodiments, provided are a method for activating the immune system of a subject, a method for activating cytotoxic T cells (CTLs) of a subject *in vivo* or *in vitro,* a method for increasing the proliferation of CD4$^+$ and/or CD8$^+$ T cells *in vivo* or *in vitro,* a method for activating NK cells of a subject *in vivo* or *in vitro,* a method for activating γδT cells of a subject *in vivo* or *in vitro,* a method for activating Th1 cells of a subject *in vivo* or *in vitro,* a method for activating, reducing, or eliminating the cell number and/or activity of at least one type of regulatory T cells (Tregs) in a subject, a method for increasing IFN-γ production and/or proinflammatory cytokine secretion in a subject, and a method for blocking or inhibiting the interaction between PD-1 and PD-L1/PD-L2 in a subject, each comprising administering to the subject an effective amount of the aforementioned PD-1 antibody or antigen-binding fragment thereof, IL-2 variant, immunoconjugate, polynucleotide, or composition (including the pharmaceutical composition) of the present disclosure.

## BRIEF DESCRIPTION OF THE DRAWINGS

FIG. 1 shows the extent to which A17m09_hIgG4 activates T cells as measured through the release of cytokines in a DC cell:T cell mixed lymphocyte reaction, with pembrolizumab as a positive control and hIgG4 as a negative control.

FIG. 2 shows the construction of a fusion protein of an anti-PD-1 antibody and an IL-2 mutant, wherein molecules of the anti-PD-1 nanobody A17m0902 are coupled to the N-termini of the Knob and Hole chains, respectively, by the IgG1 heavy chain hinge region. The IL-2 mutant is linked to the C-terminus of the Knob chain of the Fc by a $(G_4S)_3$ linker, and IL-2v (V91T) in the F molecule is coupled to the N-terminus of the Knob chain by a $G_4S$ linker.

FIG. 3 shows the proliferation activity of PD-1-IL-2v fusion proteins of the same format containing different IL-2 mutants on the M07e and M07e-hPD-1 cell strains, wherein FIG. 3A shows the proliferation activity of PD-1-IL-2v fusion proteins on M07e cells, and FIG. 3B shows the proliferation activity of PD-1-IL-2v fusion proteins on M07e-hPD-1 cells.

FIG. 4 shows the proliferation promoting activity of PD-1-IL-2v or IgG-IL-2v on M07e-hPD-1 cells.

FIG. 5 shows the proliferation activity of PD-1-IL-2v fusion proteins containing different IL-2 mutants on unstimulated T cells, wherein FIG. 5A shows the proliferation of CD4$^+$ T cells, and FIG. 5B shows the proliferation of CD8$^+$ T cells.

FIG. 6 shows the proliferation activity of PD-1-IL-2v fusion proteins containing different IL-2 mutants on T cells stimulated with an anti-CD3 antibody, wherein FIG. 6A shows the proliferation of CD4$^+$ T cells, and FIG. 6B shows the proliferation of CD8$^+$ T cells.

## DETAILED DESCRIPTION

### Terminology

In order to facilitate the understanding of the present disclosure, certain technical and scientific terms are specifically defined below. Unless otherwise specifically defined herein, all other technical and scientific terms used herein have the meanings generally understood by those of ordinary skill in the art to which the present disclosure pertains.

"Buffer" refers to a buffer that resists changes in pH by the action of its acid-base conjugate components. Examples of buffers that maintain the pH within an appropriate range include acetate, succinate, gluconate, histidine, oxalate, lactate, phosphate, citrate, tartrate, fumarate, glycylglycine, and other organic acid buffers.

"Histidine buffer" is a buffer comprising histidine ions. Examples of histidine buffers include acetic acid-histidine, succinic acid-histidine, histidine-histidine hydrochloride, hydrochloric acid-histidine, sulfuric acid-histidine, etc. Illustratively, the histidine-hydrochloride buffer is prepared from histidine and hydrochloric acid, or from histidine and histidine hydrochloride.

"Pharmaceutical composition" refers to a mixture containing one or more of the compounds described herein or physiologically/pharmaceutically acceptable salts or prodrugs thereof, and other chemical components, wherein the other components are, for example, physiologically/pharmaceutically acceptable carriers and excipients. The pharmaceutical composition is intended to promote administration to an organism and facilitate the absorption of the active ingredient so that it can exert its biological activity. As used herein, "pharmaceutical composition" and "formulation" are not mutually exclusive.

Unless otherwise specified, the solvent in the pharmaceutical composition described in the present disclosure in solution form is water.

The term "about" as used herein means that a numerical value is within an acceptable error range for the specific value determined by one of ordinary skill in the art, and the numerical value depends in part on how the value is measured or determined (i.e., the limits of the measurement system). For example, "about" may mean a standard deviation within 1 or more than 1 in each practice in the art. Alternatively, "about" or "substantially comprising" may mean a range of up to 20%. Furthermore, particularly for biological systems or processes, the term may mean up to an order of magnitude or up to 5-fold of a numerical value. Unless otherwise specified, when a specific value is provided in the present application and claims, the meaning of "about" or "substantially comprising" should be assumed to be within an acceptable error range for that specific value.

The pharmaceutical composition described in the present disclosure can achieve the effect of being stable: the antibody in the pharmaceutical composition substantially retains its physical and/or chemical stability and/or biological activity after storage; preferably, the pharmaceutical composition substantially retains its physical and chemical stability as well as its biological activity after storage. The storage period is generally selected based on a predetermined shelf life of the pharmaceutical composition. There are a variety of analytical techniques currently available for measuring protein stability, and the stability after storage for a selected period of time at a selected temperature can be measured.

A stable pharmaceutical antibody formulation is one in which no significant change is observed under the following conditions: stored at refrigeration temperature (2-8 °C) for at least 3 months, preferably 6 months, more preferably 1 year, and even more preferably at most 2 years. In addition, stable liquid formulations include liquid formulations that exhibit desirable features after storage at 25 °C for periods including 1 month, 3 months, and 6 months, or storage at 40 °C for periods including 1 month. Typical acceptable criteria for stability are as follows: typically, no more than about 10%, preferably no more than about 5%, of antibody monomer is degraded as measured by SEC-HPLC. The pharmaceutical antibody formulation is colorless or yellow, or clear to slightly opalescent, by visual analysis. The concentration, pH, and osmolality of the formulation have a change of no more than ±10%. Typically, clippings of no more than about 10%, preferably no more than about 5%, are observed. Typically, aggregation of no more than about 10%, preferably no more than about 5%, is formed.

An antibody "retains its physical stability" in a pharmaceutical formulation if it shows no significant increase in aggregation, precipitation and/or denaturation upon visual inspection of color and/or clarity, or as determined by UV light scattering, size exclusion chromatography (SEC), and dynamic light scattering (DLS). Changes in protein conformation can be assessed by fluorescence spectroscopy (which determines the protein tertiary structure) and by FTIR spectroscopy (which determines the protein secondary structure).

An antibody "retains its chemical stability" in a pharmaceutical formulation if it shows no significant chemical change. Chemical stability can be evaluated by detecting and quantifying chemically changed proteins. Degradation processes that often change the chemical structure of proteins include hydrolysis or clipping (assessed by methods such as size exclusion chromatography and SDS-PAGE), oxidation (assessed by methods such as peptide mapping in combination with mass spectroscopy or MALDI/TOF/MS), deamidation (assessed by methods such as ion-exchange chromatography, capillary isoelectric focusing, peptide mapping, and isoaspartic acid determination), and isomerization (assessed by isoaspartic acid content determination, peptide mapping, etc.).

An antibody "retains its biological activity" in a pharmaceutical formulation if the biological activity of the antibody at a given time is within a predetermined range of the biological activity exhibited during the preparation of the pharmaceutical formulation. The biological activity of an antibody can be determined, for example, by an antigen-binding assay.

The three-letter and single-letter codes for amino acids used in the present disclosure are as described in J. biol. chem., 243, p3558 (1968).

"Programmed death-1", "programmed cell death-1", "protein PD-1", "PD-1", "PDCD1", and "hPD-1" are used interchangeably and include variants, isoforms, and species homologs of human PD-1, and analogs having at least one common epitope with PD-1. The complete PD-1 sequence can be found under GenBank accession No. U64863.

"Programmed death ligand-1 (PD-L1)" is one of two cell surface glycoprotein ligands of PD-1 (the other being PD-L2) that down-regulates T cell activation and cytokine secretion upon binding to PD-1. "PD-L1" as used herein includes human PD-L1 (hPD-L1), variants, isoforms, and interspecies homologs of hPD-L1, and analogs sharing at least one common epitope with hPD-L1. The complete hPD-L1 sequence can be found under GenBank accession No. Q9NZQ7.

"PD-1 binding protein" refers to any protein capable of specifically binding to PD-1 or any molecule comprising the protein. The PD-1-binding protein may include an antibody against PD-1 defined in the present disclosure, an antigen-binding fragment thereof, or a conjugate thereof. The PD-1-binding protein also encompasses immunoglobulin superfamily antibodies (IgSF) or CDR-grafted molecules. The "PD-1-binding protein" of the present disclosure may comprise at least one immunoglobulin single variable domain (such as a VHH) that binds to PD-1. In some embodiments, the "PD-1-binding protein" may comprise 2, 3, 4, or more immunoglobulin single variable domains (such as VHHs) that bind to PD-1. The PD-1-binding protein of the present disclosure may also comprise, in addition to the immunoglobulin single variable domain of PD-1, a linker and/or a moiety with an effector function, such as a half-life extending moiety (e.g., an immunoglobulin single variable domain that binds to serum albumin), and/or a fusion partner (such as serum albumin) and/or a conjugated polymer (such as PEG) and/or an Fc region. In some embodiments, the "PD-1-binding protein" of the present disclosure also encompasses bispecific/multi-specific antibodies comprising immunoglobulins that bind to different antigens (e.g., a first antibody that binds to a first antigen (e.g., PD-1) and a second antibody that binds to a second antigen (e.g., 4-1BB), optionally a third antibody that binds to a third antigen, and further optionally a fourth antibody that binds to a fourth antigen).

The "PD-1-binding protein" or "PD-1 antibody or antigen-binding fragment thereof" of the present disclosure may comprise one or more effector molecules in, for example, a conjugated manner. The "effector molecule" includes, for example, antineoplastic agents, drugs, toxins, biologically active proteins (such as enzymes), other antibody or antibody fragments, synthetic or naturally occurring polymers, nucleic acids, and fragments thereof (such as DNA, RNA, and fragments thereof), radionuclides, particularly radioiodide, radioisotopes, chelated metals, nanoparticles and reporter groups such as fluorescent compounds or compounds which may be detected by NMR or ESR spectroscopy. In some embodiments, the effector molecule is a cytokine (e.g., IL-2, IL-15, or a variant thereof). In some other embodiments, the effector molecule is a polymer, which may generally be a synthetic or naturally occurring polymer. Specific examples of synthetic polymers include optionally substituted straight or branched chain poly(ethylene glycol), poly(propylene glycol), poly(vinyl alcohol), or derivatives thereof. Specific naturally occurring polymers include lactose, amylose, dextran, glycogen, or derivatives thereof. In some other embodiments, the polymer is albumin or a fragment thereof, e.g., human serum albumin or a fragment thereof. Conjugation of the polymer to the PD-1-binding protein or PD-1 antibody can be achieved by conventional methods.

"Cytokine" is a general term for proteins that are released by a cell population to act on other cells as intercellular mediators. Examples of such cytokines include lymphokines, monokines, chemokines, and traditional polypeptide hormones. Exemplary cytokines include: human IL-2, IL-15, IFN-y, IL-6, TNFα, IL-17, and IL-5 and variants thereof.

"Interleukin-2" or "IL-2" refers to any natural IL-2 of any vertebrate source, including mammals such as primates (e.g., humans) and rodents (e.g., mice and rats). The term encompasses unprocessed IL-2 as well as any form of processed IL-2 derived from cells. The term also encompasses naturally occurring IL-2 variants, such as splice variants or allelic variants. An exemplary amino acid sequence of wild-type human IL-2 is set forth in SEQ ID NO: 87. Unprocessed human IL-2 additionally comprises an N-terminal signal peptide of 20 amino acids (set forth in SEQ ID NO: 272 in WO2012107417), which is absent in the mature IL-2 molecule.

"Mutated IL-2" or "IL-2 variant" refers to a polypeptide that differs from the wild-type IL-2 amino acid sequence by a mutation (e.g., substitution, deletion, or addition) in at least one amino acid position.

In the present disclosure, when referring to an IL-2 variant, single amino acid substitutions are described in the following manner: [original amino acid residue/position/amino acid residue for substitution]. For example, the substitution of alanine for phenylalanine at position 42 may be represented by F42A. Single amino acid substitutions may be linked by "/" to indicate a combination of mutations at multiple given positions. For example, a combination of mutations at positions F42A, L72G, and N88R can be expressed as: F42A/L72G/N88R.

In the present disclosure, when referring to amino acid positions in an IL-2 protein or IL-2 sequence segment, they are determined by reference to the amino acid sequence SEQ ID NO: 87 of the wild-type human IL-2 protein (also referred to as wt IL-2). Corresponding amino acid positions on other IL-2 proteins or polypeptides (including full-length sequences or truncated fragments) can be identified by amino acid sequence alignment with SEQ ID NO: 87. Thus, in the present disclosure, unless otherwise indicated, amino acid positions of an IL-2 protein or polypeptide are amino acid positions numbered according to SEQ ID NO: 87. For example, when referring to "F42", it refers to the phenylalanine residue F at position 42 of SEQ ID NO: 40, or an amino acid residue at the corresponding position on other IL-2 polypeptide sequences determined by alignment. Sequence alignments for amino acid position determination can be performed using the Basic Local Alignment Search Tool available at https://blast.ncbi.nlm.nih.gov/Blast.cgi with default parameters.

"CD25" or "α subunit of IL-2 receptor" refers to any natural CD25 of any vertebrate source, including mammals such as primates (e.g., humans) and rodents (e.g., mice and rats). The term encompasses unprocessed "full-length" CD25 as well as any form of processed CD25 derived from cells; and naturally occurring CD25 variants, such as splice variants or allelic variants. In certain embodiments, CD25 is human CD25.

"High-avidity IL-2 receptor" refers to the heterotrimer form of the IL-2 receptor that consists of a receptor γ subunit (also known as a common cytokine-receptor γ subunit, yc, or CD132), a receptor β subunit (also known as CD122 or p70), and a receptor α subunit (also known as CD25 or p55). In contrast, "moderate-avidity IL-2 receptor" refers to an IL-2 receptor that comprises only γ and β subunits but no α subunit (see, e.g., Olejniczak and Kasprzak, MedSci Monit 14, RA179-189(2008)).

"Antibody" is used in the broadest sense and encompasses a variety of antibody structures, including but not limited to monoclonal antibodies, polyclonal antibodies, monospecific antibodies, multispecific antibodies (e.g., bispecific antibodies), full-length antibodies, and antibody fragments (or antigen-binding fragments, or antigen-binding moieties) so long as they exhibit the desired antigen-binding activity. An antibody may refer to an immunoglobulin that is a four-peptide-chain structure formed by linking two identical heavy chains and two identical light chains by interchain disulfide bonds. The heavy chain constant regions of immunoglobulins differ in amino acid composition and arrangement; therefore, they also differ in antigenicity. Accordingly, immunoglobulins can be divided into five classes, or isotypes of immunoglobulins, namely IgM, IgD, IgG, IgA, and IgE, and their corresponding heavy chains are μ chains, δ chains, γ chains, α chains, and ε chains, respectively. Igs of the same class can be divided into different subclasses based on the amino acid composition of their hinge regions and the number and positions of heavy chain disulfide bonds; for example, IgGs can be divided into IgG1, IgG2, IgG3, and IgG4. Light chains are divided into κ or λ chains based on their constant regions. Each of the five Ig classes may have a κ chain or λ chain. In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (CDRs) and 4 framework regions (FRs) with relatively conservative sequences. The 3 hypervariable regions determine the specificity of the antibody and thus are also known as complementarity-determining regions (CDRs). Each of the light chain variable regions (VLs) and the heavy chain variable regions (VHs) consists of 3 CDRs and 4 FRs arranged from the amino-terminus to the carboxyl-terminus as follows: FR1, CDR1, FR2, CDR2, FR3, CDR3, and FR4. The 3 CDRs of the light chain refer to LCDR1, LCDR2, and LCDR3, and the 3 CDRs of the heavy chain refer to HCDR1, HCDR2, and HCDR3.

"Antigen-binding fragment" encompasses a single-chain antibody (i.e., full-length heavy and light chains); Fab, a modified Fab, Fab', a modified Fab', F(ab')2, Fv, Fab-Fv, Fab-dsFv, a single-domain antibody (e.g., VH or VL or VHH), scFv, a bivalent or trivalent or tetravalent antibody, Bis-scFv, a diabody, a tribody, a triabody, a tetrabody and an epitope-binding fragment of any of the above (see, e.g., Holliger and Hudson, 2005, Nature Biotech. 23(9): 1126-1136; Adair and Lawson, 2005, Drug Design Reviews-Online 2(3), 209-217). In some embodiments, the antigen-binding fragment is a VHH. Methods for producing and preparing such antigen-binding fragments are well known in the art (see, e.g., Verma et al., 1998, Journal of Immunological Methods, 216, 165-181). Fab-Fv was first disclosed in WO2009/040562, and its disulfide-stabilized form Fab-dsFv was first disclosed in WO2010/035012. The antigen-binding fragment of the present disclosure also includes Fab and Fab' fragments described in WO2005/003169, WO2005/003170, and WO2005/003171. Multivalent antibodies may comprise multiple specificities (e.g., bispecificities) or may be monospecific (see, e.g., WO92/22583 and WO05/113605), and an example of the latter is Tri-Fab (or TFM) described in WO 92/22583.

"Fc domain" or "Fc region" is used to define a C-terminal region of an immunoglobulin heavy chain that comprises at least a portion of the constant region. The term includes native sequence Fc regions and variant Fc regions. Although the boundaries of the Fc region of an IgG heavy chain might vary slightly, the human IgG heavy chain Fc region is usually defined to extend from Cys226 or Pro230 to the carboxyl-terminus of the heavy chain. However, antibodies produced by host cells may undergo post-translational cleavage of one or more, particularly one or two, amino acids from the C-terminus of the heavy chain. Therefore, an antibody produced by a host cell by expressing a specific nucleic acid molecule encoding a full-length heavy chain may include the full-length heavy chain, or it may include a cleaved variant of the full-length heavy chain (also referred to herein as a "cleaved variant heavy chain"). This may be the case where the final two C-terminal amino acids of the heavy chain are glycine (G446) and lysine (K447, numbered according to the Kabat EU index). Therefore, the C-terminal lysine (K447), or the C-terminal glycine (G446) and lysine (K447), of the Fc region may or may not be present. If not indicated otherwise, amino acid sequences of heavy chains including Fc regions (or a subunit of an Fc region defined in the present disclosure) are denoted in the present disclosure without a C-terminal glycine-lysine dipeptide. In one embodiment, a heavy chain of a subunit of an Fc region included in an immunoconjugate comprises an additional C-terminal glycine-lysine dipeptide (G446 and K447, numbered according to the EU index of Kabat). In one embodiment, a heavy chain of a subunit of an Fc region included in an immunoconjugate comprises an additional C-terminal glycine residue (G446, numbered according to the EU index of Kabat). The composition of the present disclosure (such as the pharmaceutical composition) comprises a population of immunoconjugates of the present disclosure. The population of immunoconjugates may comprise molecules having a full-length heavy chain and molecules having a cleaved variant heavy chain. The population of immunoconjugates may consist of a mixture of molecules having a full-length heavy chain and molecules having a cleaved variant heavy chain, wherein at least 50%, at least 60%, at least 70%, at least 80%, or at least 90% of the immunoconjugates have a cleaved variant heavy chain. The cleaved variant heavy chain may be the case where the C-terminal lysine (K447), or the C-terminal glycine (G446) and lysine (K447), of the Fc region is/are not present. Unless otherwise specified in the present disclosure, the numbering of amino acid residues in the Fc region or constant region conforms to the EU numbering scheme, also known as the EU index, as described in Kabat et al., Sequences of Proteins of Immunological Interest, 5th Edition, Public Health Service, National Institutes of Health, Bethesda, MD, 1991.

A "subunit" of an Fc region refers to one of the two polypeptides forming the dimeric Fc region, i.e., a polypeptide comprising C-terminal constant regions of an immunoglobulin heavy chain, capable of stable self-association. For example, a subunit of an IgG Fc region comprises an IgG CH2 constant domain and an IgG CH3 constant domain. In the present disclosure, the use of "first subunit" or Fc1 and "second subunit" or Fc2 is not intended to confer a specific order or orientation but is for ease of distinguishing.

"Modification (or mutation) promoting the association of the first subunit and the second subunit of the Fc domain" is a manipulation of the peptide backbone or a post-translational modification of an Fc domain subunit that reduces or prevents the association of a polypeptide comprising the Fc domain subunit with an identical polypeptide to form a homodimer. As used herein, a modification promoting association specifically includes separate modifications made to each of the two Fc domain subunits desired to associate (i.e., the first subunit and the second subunit of the Fc domain), wherein the modifications are complementary to each other so as to promote the association of the two Fc domain subunits. For example, a modification promoting association may alter the structure or charge of one or both of the Fc domain subunits so as to promote their association sterically or electrostatically, respectively. Thus, (hetero)dimerization occurs between a polypeptide comprising the first Fc domain subunit and a polypeptide comprising the second Fc domain subunit, which might be different in the sense that other components fused to each of the subunits (e.g., antigen-binding moieties) are different. In some embodiments, the modification promoting association comprises an amino acid mutation in the Fc domain, specifically an amino acid substitution.

"Complementarity" refers to, for example, a combination of interactions that affect heavy/light chain pairing at the interface of CH1 and CL (or CH3 and CH3) of the antigen-binding protein described herein. "Steric complementarity" or "conformational complementarity" refers to, for example, the compatibility of three-dimensional structures at the interaction surface of CH1 and CL (or CH3 and CH3). "Electrostatic complementarity" refers to, for example, the compatibility of negatively and/or positively charged atoms placed at the interaction surface of CH1 and CL (or CH3 and CH3). Methods for measuring electrostatic complementarity at a protein/protein interface are known in the art and are described, for example, in McCoy et al., (1997) J Mol Biol 268,570-584; Lee et al., (2001) Protein Sci. 10,362-377; and Chau et al., (1994) J Comp Mol Des 8,51325. Methods for measuring steric complementarity at a protein/protein interface are known in the art and are described, for example, in Lawrence et al., (1993) J Mol Biol 234,946-950; Walls et al., (1992) J Mol Biol 228,277-297; and Schueler-Furman et al., (2005) Proteins 60,187-194.

"Effector function" when used in reference to antibodies refers to those biological activities attributable to the Fc region of an antibody, which vary with the antibody isotype. Examples of antibody effector functions include: C1q binding and complement-dependent cytotoxicity (CDC), Fc receptor (FcR) binding, antibody-dependent cell-mediated cytotoxicity (ADCC), antibody-dependent cellular phagocytosis (ADCP), cytokine secretion, immune complex-mediated antigen uptake by antigen-presenting cells, down-regulation of cell surface receptors (e.g., B cell receptor), and B cell activation. ADCC is an immune mechanism leading to the lysis of antibody-coated target cells by immune effector cells. The target cells are cells to which antibodies or derivatives thereof comprising an Fc region specifically bind, generally via the N-terminal protein part of the Fc region. "Reduced ADCC" is defined as a reduction in the number of target cells that are lysed in a given time, at a given concentration of antibody in the medium surrounding the target cells, by the mechanism of ADCC defined above, and/or an increase in the concentration of antibody in the medium surrounding the target cells, required to achieve the lysis of a given number of target cells in a given time, by the mechanism of ADCC. The reduction in ADCC is relative to the ADCC mediated by the same antibody that is produced by the same type of host cells using the same standard production, purification, formulation, and storage methods known to those skilled in the art, but that has not been engineered. For example, the reduction in ADCC mediated by an antibody comprising in its Fc domain an amino acid substitution that reduces ADCC is relative to the ADCC mediated by the same antibody without this amino acid substitution in the Fc domain. Suitable assays for measuring ADCC are well known in the art (see, e.g., WO2006/082515 or WO2012/130831).

"Activating Fc receptor" is an Fc receptor that, after engagement by an Fc domain of an antibody, elicits signaling events that stimulate cells bearing the receptor to perform effector functions. Human activating Fc receptors include FcγRIIIa (CD16a), FcγRI (CD64), FcγRIIa (CD32), and FcαRI(CD89).

"Immunoconjugate" refers to a polypeptide molecule comprising at least one IL-2 molecule and at least one antibody. As described herein, an IL-2 molecule may be linked to an antibody through a variety of interactions and in a variety of configurations. In a particular embodiment, the IL-2 molecule is fused to the antibody via a peptide linker. A particular immunoconjugate according to the present disclosure essentially consists of one IL-2 molecule and an antibody linked by one or more linker sequences.

"Reduced avidity" or "reduced binding", for example, reduced binding to IL-2R$\alpha$, refers to a reduction in avidity for the corresponding interaction, as measured by, for example, SPR. The term also includes reduction of the avidity to 0 or below the detection limit of the analytic method, i.e., complete elimination of the interaction. Conversely, "reduced avidity" or "increased binding" refers to an increase in binding avidity for the corresponding interaction.

For the determination or definition of CDRs, the deterministic depiction of CDRs and identification of residues comprising binding sites of an antibody can be accomplished by resolving the structure of the antibody and/or resolving the structure of the antibody-ligand complex. This can be accomplished by any one of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition.

The Kabat numbering scheme is a standard for numbering residues in antibodies and is generally used to identify CDRs (see, e.g., Johnson & Wu, 2000, Nucleic Acids Res., 28: 214-8). The Chothia numbering scheme is similar to the Kabat numbering scheme, except that it takes into account the positions of certain structural loop regions (see, e.g., Chothia et al., 1986, J. Mol. Biol., 196: 901-17; Chothia et al., 1989, Nature, 342: 877-83). The AbM numbering scheme adopts a computer program integration suite for modeling antibody structures manufactured by Oxford Molecular Group (see, e.g., Martin et al., 1989, Proc Natl Acad Sci (USA), 86: 9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3: 194-198). The contact definition is based on the analysis of the available complex crystal structures (see, e.g., MacCallum et al., 1996, J. Mol. Biol., 5: 732-45). In the conformational definition, the positions of the CDRs can be identified as residues that contribute enthalpy to the antigen binding (see, e.g., Makabe et al., 2008, Journal of Biological Chemistry, 283: 1156-1166). In addition, other CDR boundary definitions may not strictly follow one of the methods described above, but still overlap with at least a portion of the Kabat CDRs, although they may be shortened or lengthened based on predictions or experimental results that a particular residue or a particular group of residues does not significantly affect the antigen binding. As used in the present disclosure, a CDR may refer to a CDR defined by any method known in the art, including combinations of methods. The correspondence between the various numbering schemes is well known to those skilled in the art, and examples are shown in Table 1 below.

Table 1. The relationships between CDR numbering schemes

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

The CDR amino acid residues of the VL and VH regions of the antibody of the present disclosure accord with the known Kabat numbering scheme in terms of number and positions.

The antibodies of the present disclosure may be polyclonal, monoclonal, xenogenic, allogeneic, syngeneic, or modified forms thereof, with the monoclonal antibodies being especially applicable in various examples. Generally, the antibodies of the present disclosure are recombinant antibodies. As used herein, "recombinant" generally refers to such products as a cell, a nucleic acid, a protein, or a vector, and indicates that the cell, the nucleic acid, the protein, or the vector has been modified by introduction of a heterologous nucleic acid or protein or alteration of a natural nucleic acid or protein, or that the cell is derived from a cell modified in this way. For example, recombinant cells express genes that are not found within the native (non-recombinant) cellular form or express native genes that are abnormally expressed, lowly expressed, or not expressed at all.

"Domain" of a polypeptide or protein refers to a folded protein structure that is capable of maintaining its tertiary structure independently of the rest of the protein. In general, a domain is responsible for a single functional property of a protein and, in many cases, may be added, deleted, or transferred to other proteins without loss of functions of the rest of the protein and/or the domain of the protein.

"Immunoglobulin domain" refers to a globular region of an antibody chain (e.g., a chain of a conventional antibody with a four-peptide-chain structure or a heavy-chain antibody) or a polypeptide essentially consisting of such globular regions. The immunoglobulin domain is characterized in that it retains the immunoglobulin fold characteristic of an antibody molecule, and it consists of a 2-layer sandwich of about 7 antiparallel β-strands arranged in two β-sheets, optionally stabilized by a conserved disulfide bond.

"Immunoglobulin variable domain" refers to an immunoglobulin domain essentially consisting of four "framework regions" referred to in the art and hereinafter as "framework region 1" or "FR1", "framework region 2" or "FR2", "framework region 3" or "FR3", and "framework region 4" or "FR4", where the framework regions are spaced apart by three "complementarity-determining regions" or "CDRs" referred to in the art and hereinafter as "complementarity-determining region 1" or "CDR1", "complementarity-determining region 2" or "CDR2", and "complementarity-determining region 3" or "CDR3". Thus, the general structure or sequence of an immunoglobulin variable domain can be expressed as follows: FR1-CDR1-FR2-CDR2-FR3-CDR3-FR4. Immunoglobulin variable domains possess specificity for an antigen by virtue of having an antigen-binding site.

"Immunoglobulin single variable domain" is generally used to refer to an immunoglobulin variable domain (which may be a heavy or light chain domain, including a VH, VHH, or VL domain) that can form a functional antigen-binding site without interacting with other variable domains (e.g., without VH/VL interactions as are required between the VH and VL domains of conventional four-chain monoclonal antibodies). Examples of "immunoglobulin single variable domains" include nanobodies (including a VHH, humanized VHH and/or camelized VH, e.g., a camelized human VH), IgNAR, domains, (single-domain) antibodies as VH domains or derived from VH domains (such as dAbsTM) and (single-domain) antibodies as VL domains or derived from VL domains (such as dAbsTM). Immunoglobulin single variable domains based on and/or derived from heavy chain variable domains (such as VH or VHH domains) are generally preferred. A specific example of an immunoglobulin single variable domain is a "VHH domain" (or "VHH" for short) as defined below.

"VHH domain", also known as a heavy chain single-domain antibody, a VHH, a VHH antibody fragment, a VHH antibody, or a nanobody, is a variable domain of an antigen-binding immunoglobulin known as a "heavy chain antibody" (i.e., "an antibody devoid of light chains") (Hamers-Casterman C, Atarhouch T, Muyldermans S, Robinson G, Hamers C, Songa EB, Bendahman N, Hamers R., "Naturally occurring antibodies devoid of light chains"; Nature 363, 446-448 (1993)). "VHH domain" is used to distinguish the variable domain from the heavy chain variable domain (which is referred to in the present disclosure as a "VH domain") and the light chain variable domain (which is referred to in the present disclosure as a "VL domain") present in conventional antibodies with a four-peptide-chain structure. VHH domains specifically bind to an epitope without the need for an additional antigen-binding domain (as opposed to the VH or VL domain in conventional antibodies with a four-peptide-chain structure, in which case the epitope is recognized by the VL domain together with the VH domain). A VHH domain is a small, stable, and efficient antigen recognition unit formed by a single immunoglobulin domain. "Heavy chain single-domain antibody", "VHH domain", "VHH", "VHH antibody fragment", "VHH antibody", and "domain" ("Nanobody" is a trademark of Ablynx N.V., Ghent, Belgium) are used interchangeably. "VHH domains" include, but are not limited to, natural antibodies produced by camelids, antibodies produced by camelids and then humanized, or antibodies obtained by screening with phage display techniques. In some embodiments, the VHH domain comprises three CDRs and four framework regions designated FR1, CDR1, FR2, CDR2, FR3, CDR3, and FR4. In some embodiments, the VHH domain may be truncated at the N-terminus or C-terminus such that it comprises only part of FR1 and/or FR4, or lacks one or two of those framework regions, as long as the VHH substantially retains antigen binding and specificity. The total number of amino acid residues in a VHH domain will usually be in the range of 110 to 120, often between 112 and 115. However, it should be noted that smaller and longer sequences may also be suitable for the purposes described in the present disclosure.

As is well known in the art for VH domains and for VHH domains, the total number of amino acid residues in each of the CDRs may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, in general, the numbering according to Kabat may or may not correspond to the actual numbering of the amino acid residues in the actual sequence. Other numbering systems or numbering schemes include Chothia, IMGT, and AbM.

"Humanized antibody", also known as CDR-grafted antibody, refers to an antibody produced by grafting non-human CDR sequences into the framework of variable regions of a human antibody. Chimeric antibodies, due to their significant non-human protein content, can induce a strong immune response. This issue can be overcome by using humanized antibodies. To avoid the decrease in activity caused by the decrease in immunogenicity, the variable regions of a fully human antibody can be subjected to minimum reverse mutation to maintain activity. Examples of "humanization" include "humanization" of VHH domains derived from camelidae by replacing one or more amino acid residues in the amino acid sequence of the original VHH sequence with one or more amino acid residues present at the corresponding positions in a VH domain of a human conventional antibody with a four-peptide-chain structure (also referred to in the present disclosure as "sequence optimization"; in addition to humanization, "sequence optimization" may also encompass other modifications to the sequence by one or more mutations providing improved properties of the VHH, such as removal of potential post-translational modification sites). The humanized VHH domain may contain one or more fully human framework region sequences, and in some specific embodiments, may contain the human framework region sequence of IGHV3. Methods for humanization include, e.g., protein surface amino acid humanization (resurfacing) and universal framework grafting method for antibody humanization (CDR grafting to a universal framework), i.e., "grafting" CDRs onto other "frameworks" (including but not limited to human scaffolds or non-immunoglobulin scaffolds). Scaffolds and techniques suitable for such CDR grafting are known in the art. For example, germline DNA sequences of genes of the human heavy and light chain variable regions can be found in the "VBase" human germline sequence database, as well as in Kabat, E. A. et al., 1991 Sequences of Proteins of Immunological Interest, 5th Edition. The humanized antibody of the present disclosure also includes humanized antibodies that are further subjected to CDR avidity maturation by phage display. In addition, in order to avoid the decrease in activity caused by the decrease in immunogenicity, the framework sequence in the human antibody variable region can be subjected to minimum reverse mutation or back mutation to maintain activity.

An "avidity-matured" antibody is an antibody that has one or more changes in one or more hypervariable regions (HVRs) that result in an improvement in the avidity of the antibody for the antigen, as compared to the parent antibody, which does not have such changes. For example, an "avidity-matured" PD-1 antibody has one or more changes in one or more CDRs that result in an increase in the avidity for the antigen, as compared to its parent antibody. Avidity-matured antibodies can be prepared, for example, by methods known in the art as described below: Marks et al., 1992, Biotechnology 10: 779-783 or Barbas et al., 1994, Proc. Nat. Acad. Sci, USA 91: 3809-3813; Shier et al., 1995, Gene 169: 147-155; Yelton et al., 1995, Immunol. 155: 1994-2004; Jackson et al., 1995, J. Immunol. 154(7): 3310-9; Hawkins et al., 1992, J. Mol. Biol. 226(3): 889896; KS Johnson and RE Hawkins, "Affinity maturation of antibodies using phage display", Oxford University Press 1996.

Generally, the PD-1 antibody or the immunoconjugate of the present disclosure will bind to an antigen to be bound (i.e., PD-1) with a dissociation constant (KD) of preferably $10^{-7}$ to $10^{-10}$ mol/L (M), more preferably $10^{-8}$ to $10^{-10}$ mol/L, even more preferably $10^{-9}$ to $10^{-10}$ or less, and/or with an association constant (KA) of at least $10^{-7}$ M, preferably at least $10^{-8}$ M, more preferably at least $10^{-9}$ M, and more preferably at least $10^{-10}$ M, as measured by a Biacore or KinExA or Fortibio assay. Any KD value greater than $10^{-4}$ M is generally considered to indicate non-specific binding. Specific binding of an antigen-binding protein to an antigen or epitope can be measured in any suitable manner known, including, for example, surface plasmon resonance (SPR) assay, Scatchard assay, and/or competitive binding assay (e.g., radioimmunoassay (RIA), enzyme immunoassay (EIA), and sandwich competitive assay) described in the present disclosure.

"Antigen" refers to a molecule used for immunization of an immunocompetent vertebrate to produce an antibody that recognizes the antigen or to screen an expression library (e.g., particularly phage, yeast, or ribosome display library). In the present disclosure, the antigen is defined in a broader sense, and includes a target molecule that is specifically recognized by the antibody, and a portion or a mimic of a molecule used in an immunization process for producing the antibody or in library screening for selecting the antibody. For example, for the antibody of the present disclosure that binds to human PD-1, monomers and multimers (e.g., dimers, trimers, etc.) of human PD-1, and truncated variants and other variants of human PD-1 are all referred to as PD-1 antigens.

"Epitope" refers to a site on an antigen to which an immunoglobulin or an antibody binds. An epitope may be formed from contiguous amino acids, or non-contiguous amino acids juxtaposed by tertiary folding of the protein. An epitope formed from contiguous amino acids is generally retained after exposure to a denaturing solvent, while an epitope formed by tertiary folding is generally lost after treatment with a denaturing solvent. An epitope generally comprises, for example, at least 3-15 amino acids in a distinctive spatial conformation. Methods for determining what epitope is bound by a given antibody are well known in the art and include immunoblotting, immunoprecipitation analysis, etc. Methods for determining the spatial conformation of an epitope include techniques in the art and techniques described in the present disclosure, such as X-ray crystallography and two-dimensional nuclear magnetic resonance.

"Specific binding" or "selective binding" refers to the binding of an antibody to an epitope on a predetermined antigen. For example, an antibody binds to a predetermined antigen or epitope thereof with an equilibrium dissociation constant ($K_D$) of about less than $10^{-7}$ M or even less and with an avidity that is at least twice as high as its avidity for binding to a non-specific antigen other than the predetermined antigen or the epitope thereof (or non-specific antigens other than closely related antigens, e.g., BSA, etc.), when measured by surface plasmon resonance (SPR) techniques in an instrument using human PD-1 or an epitope thereof as an analyte and the antibody as a ligand. "Antigen-recognizing antibody" is used interchangeably in the present disclosure with "specifically binding antibody".

"Binding avidity" or "avidity" is used in the present disclosure as a measure of the strength of a non-covalent interaction between two molecules (e.g., an antibody or a portion thereof and an antigen). The binding avidity between two molecules can be quantified by determining the dissociation constant (KD). KD can be determined by measuring the kinetics of complex formation and dissociation using, for example, the surface plasmon resonance (SPR) method (Biacore). The rate constants corresponding to the association and dissociation of a monovalent complex are referred to as the association rate constant ka (or kon) and the dissociation rate constant kd (or koff), respectively. $K_D$ is related to ka and kd by the equation $K_D = kd/ka$. The value of the dissociation constant can be determined directly by well-known methods and can be calculated by methods such as those described by Caceci et al. (1984, Byte 9: 340-362) even for complex mixtures. For example, $K_D$ can be determined by using a dual filtration nitrocellulose filter binding assay such as that disclosed by Wong & Lohman (1993, Proc. Natl. Acad. Sci. USA 90: 5428-5432). Other standard assays for evaluating the binding ability of an antibody to a target antigen are known in the art and include, for example, ELISA, western blot, RIA, and flow cytometry, as well as other assays exemplified elsewhere in the present disclosure. The binding kinetics and binding avidity of the antibody can also be evaluated by standard assays known in the art, such as surface plasmon resonance (SPR), e.g., by using the Biacore™ system or KinExA. The binding avidities associated with different molecular interactions, e.g., the binding avidities of different antibodies for a given antigen, can be compared by comparing the $K_D$ values of antibodies/antigen complexes. Similarly, the specificity of an interaction can be evaluated by determining and comparing the $K_D$ value for the interaction of interest (e.g., a specific interaction between an antibody and an antigen) with the $K_D$ value for an interaction not of interest (e.g., a control antibody known not to bind to CD40).

"Conservative replacement" refers to replacement by another amino acid residue having similar properties to the original amino acid residue. For example, lysine, arginine, and histidine have similar properties in that they have basic side chains, and aspartic acid and glutamic acid have similar properties in that they have acidic side chains. In addition, glycine, asparagine, glutamine, serine, threonine, tyrosine, cysteine, and tryptophan have similar properties in that they have uncharged polar side chains, and alanine, valine, leucine, threonine, isoleucine, proline, phenylalanine, and methionine have similar properties in that they have nonpolar side chains. In addition, tyrosine, phenylalanine, tryptophan, and histidine have similar properties in that they have aromatic side chains. Thus, it will be apparent to those skilled in the art that even when an amino acid residue in a group exhibiting similar properties as described above is replaced, it will not exhibit a particular change in properties.

"Homology", "identity", or "sequence identity" refers to sequence similarity between two polynucleotide sequences or between two polypeptides. When positions in two compared sequences are occupied by identical nucleotides or amino acid monomers, e.g., if the position of each of two DNA molecules is occupied by an identical nucleotide, the molecules are homologous at that position. The percent homology between two sequences is a function of the number of matching or homologous positions shared by the two sequences divided by the number of compared positions × 100%. For example, if 6 out of 10 positions are matched or homologous when two sequences are optimally aligned, the two sequences are 60% homologous. In general, when two sequences are aligned, a comparison is performed to obtain the maximum percent homology.

"Administer" and "treat", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs, or biological fluids. "Administer" and "treat" can refer to, for example, therapeutic, pharmacokinetic, diagnostic, research, and experimental methods. The treatment of cells comprises contacting a reagent with cells and contacting the reagent with a fluid, wherein the fluid is in contact with the cells. "Administer" and "treat" also refer to treating, e.g., cells, by reagents, diagnosis, binding compositions or by another cell *in vitro* and *ex vivo.* "Treating", when applied to human, veterinary, or research subjects, refers to therapeutic treatment, preventive or prophylactic measures, and research and diagnostic applications.

"Treatment" refers to administering a therapeutic agent, such as a composition comprising any one of the conjugation compounds of the present disclosure, either internally or externally to a patient with one or more disease symptoms on which the therapeutic agent is known to have a therapeutic effect. Typically, the therapeutic agent is administered in an amount effective to alleviate one or more disease symptoms in the patient or population being treated to induce regression of such symptoms or to inhibit the development of such symptoms to any clinically measurable degree. The amount of therapeutic agent effective to alleviate any specific disease symptom (also known as a "therapeutically effective amount") may vary depending on a variety of factors, such as the disease state, age, and weight of the patient, and the ability of the drug to produce a desired therapeutic effect in the patient. Whether a disease symptom has been palliated can be evaluated by any clinical testing methods commonly used by doctors or other health care professionals to evaluate the severity or progression of the symptom. Although the embodiments of the present disclosure (for example, treatment methods or products) may not be effective in alleviating all the target disease symptoms in every patient, as determined according to any statistical testing methods known in the art, such as Student t-test, chi-square test, Mann and Whitney's U test, Kruskal-Wallis test (H test), Jonckheere-Terpstra test and Wilcoxon test, they should palliate the target disease symptoms in a statistically significant number of patients.

"Effective amount" comprises an amount sufficient to ameliorate or prevent a symptom or disorder of a medical disease. An effective amount also means an amount sufficient to allow or facilitate diagnosis. The effective amount for a particular patient or veterinary subject may vary depending on factors such as the disorder to be treated, the general health of the patient, the method and route and dose of administration, and the severity of side effects. An effective amount may be the maximum dose or administration regimen to avoid significant side effects or toxic effects.

"Tm value" refers to the thermal denaturation temperature of a protein, i.e., the temperature at which half of the protein is unfolded and the spatial structure of the protein is destroyed. Therefore, the higher the Tm value, the higher the thermal stability of the protein.

**Examples**

The present disclosure is further described below with reference to examples, but these examples are not intended to limit the scope of the present disclosure. In the examples of the present disclosure, experimental methods without specific conditions specified were generally conducted under conventional conditions, such as those outlined in Antibodies: A Laboratory Manual and Molecular Cloning: A Laboratory Manual by Cold Spring Harbor Laboratory, or under conditions recommended by the manufacturers of the starting materials or commercial products. Reagents without specific sources indicated were commercially available conventional reagents.

**Example 1. Screening, Preparation, and Engineering of Anti-PD-1 Nanobodies**

1. Antibody screening

In the present disclosure, His-tagged human PD-1 was used as an immunizing antigen (AcroBiosystems, PD1-H5221) to immunize alpacas, a nanobody (VHH) yeast display library was constructed, and antibody screening was performed. Biotinylated human PD-1 and cynomolgus monkey PD-1 were used as screening antigens (AcroBiosystems, PD1-H82E4; PD1-C82E6). After identification of single clones, sequencing, and sequence analysis, 18 unique VHH sequences that cross-bind to human and monkey PD-1 antigens were obtained.

The sequence of human PD-1 protein (NP_005009.2) is shown below. The sequence of the extracellular region is underlined, and it starts with amino acid Leu25 and ends with amino acid Gln167.

> Amino acid sequence of human PD-1

MQIPQAPWPVVWAVLQLGWRPGWF<u>LDSPDRPWNPPTFSPALLVVTEGDNATFTCSFSNTSESFVLN
WYRMSPSNQTDKLAAFPEDRSQPGQDCRFRVTQLPNGRDFHMSVVRARRNDSGTYLCGAISLAPK
AQIKESLRAELRVTERRAEVPTAHPSPSPRPAGQFQ</u>TLVVGVVGGLLGSLVLLVWVLAVICSRAARG
TIGARRTGQPLKEDPSAVPVFSVDYGELDFQWREKTPEPPVPCVPEQTEYATIVFPSGMGTSSPARR
GSADGPRSAQPLRPEDGHCSWPL (SEQ ID NO: 1)

The A17 sequence, one of the obtained sequences, is shown below. Its CDRs are numbered according to the Kabat numbering scheme.

> Variable region of A17

EVQVVESGGGLVQPGGSLRLSCVASGLTFS<u>DYSMS</u>WYRQAPGKERELVA<u>IISGSGVIAHYVDSVKG</u>
RFTISRDNAKSTVYLQMVSLKPEDRGVYYCRA<u>VSDWDDY</u>WGQGTQVTVSS (SEQ ID NO: 2)

> A17 CDR1
DYSMS (SEQ ID NO: 3)
> A17 CDR2
IISGSGVIAHYVDSVKG (SEQ ID NO: 4)
> A17 CDR3
VSDWDDY (SEQ ID NO: 5)

A17 was linked to a human IgG4 Fc (containing a hinge region and S228P, according to the EU numbering scheme) fragment to construct a VHH-Fc antibody. Plasmids were constructed and transiently transfected into 293 cells. The antibody was expressed and purified using a protein A column. The column was washed with PBS, and the antibody was eluted with a 0.1 M glycine buffer (pH 2.5). The antibody was transferred to a PBS buffer (pH 7.4) by dialysis. Analysis showed that the protein of interest was obtained.

> hIgG4

ESKYGPPCPPCPAPEFLGGPSVFLFPPKPKDTLMISRTPEVTCVVVDVSQEDPEVQFNWYVDGVEVH
NAKTKPREEQFNSTYRVVSVLTVLHQDWLNGKEYKCKVSNKGLPSSIEKTISKAKGQPREPQVYTL
PPSQEEMTKNQVSLTCLVKGFYPSDIAVEWESNGQPENNYKTTPPVLDSDGSFFLYSRLTVDKSRW
QEGNVFSCSVMHEALHNHYTQKSLSLSLGK (SEQ ID NO: 6)

> A17_hIgG4

EVQVVESGGGGLVQPGGSLRLSCVASGLTFS<u>DYSMS</u>WYRQAPGKERELVA<u>IISGSGVIAHYVDSVKG</u>
RFTISRDNAKSTVYLQMVSLKPEDRGVYYCRA<u>VSDWDDY</u>WGQGTQVTVSS*ESKYGPPCPPCPAPE*
*FLGGPSVFLFPPKPKDTLMISRTPEVTCVVVDVSQEDPEVQFNWYVDGVEVHNAKTKPREEQFNSTYRVV*
*SVLTVLHQDWLNGKEYKCKVSNKGLPSSIEKTISKAKGQPREPQVYTLPPSQEEMTKNQVSLTCLVKGFYP*
*SDIAVEWESNGQPENNYKTTPPVLDSDGSFFLYSRLTVDKSRWQEGNVFSCSVMHEALHNHYTQKSLSLSL*
*GK*                                                                (SEQ ID NO: 7)

2. Humanization and back mutation

Antibody humanization was performed by CDR-grafting. Through the website of IMGT or NCBI, the parent PD-1 antibodies were aligned with fully human germline genes in the IMGT or NCBI/igblast database, and the human germline gene IGHV 3-23 (IGHV3-23*01; IGHV3-23*04), which is highly homologous with the PD-1 nanobodies, was selected as a humanization template. CDRs were grafted, and 4 key residues (Y37, E44, R45, and L47) in the FR2 of the nanobodies were kept. The key core residues close to the CDRs and the Vernier zone residues that interact with the CDRs were back-mutated. Humanized antibodies A17hl, A17h2, and A17h3 were obtained.

> Variable region of A17h1

EVQLQESGGGGLVQPGGSLRLSCTASGLTFS<u>DYSMS</u>WYRQAPGKGRELVA<u>IISGSGVIAHYVDSVKG</u>
RFTISRDNSKNTVYLQMRSLTPEDRAVYYCRA<u>VSDWDDY</u>WGQGTQVTVSS          (SEQ ID NO: 8)

> Variable region of A17h2

EVQLVESGGGGLVQPGGSLRLSCAASGLTFS<u>DYSMS</u>WYRQAPGKGLEWVA<u>IISGSGVIAHYVDSVKG</u>
RFTISRDNSKNTVYLQMNSLRAEDTAVYYCRA<u>VSDWDDY</u>WGQGTQVTVSS          (SEQ ID NO: 9)

> Variable region of A17h3

EVQLVESGGGGLVQPGGSLRLSCAASGLTFS<u>DYSMS</u>WYRQAPGKGLEWVA<u>IISGSGVIAHYVDSVKG</u>
RFTISRDNSKNTVYLQMNSLRAEDTAVYYCRA<u>VSDWDEY</u>WGQGTQVTVSS          (SEQ ID NO: 10)

3. Engineering for TCE (T cell epitope) removal, reduction of antibody deamidation, and reduction of antibody isomerization
The following sequences were obtained:
> Variable region of A17m09

EVQLQESGGGGLVQPGGSLRLSCTASGLTFS<u>DYSMS</u>WYRQAPGKGRELVA<u>IISGSGVITHYVDSVKG</u>
RTISRDNAKNTVYLQMRSLTPEDRAVYYCRA<u>VSDWEDY</u>WGQGTQVTVSS          (SEQ ID NO: 11)

A17m09 was selected, and the antibody sequence was further subjected to germlining engineering to improve the degree of humanization. The following sequences were obtained:

> Variable region of A17m0901

EVQLVESGGGGLVQPGGSLRLSCAASGFTFS<u>DYSMS</u>WYRQAPGKGRELVA<u>IISGSGVITHYVDSVKG</u>
RFTISRDNAKNTLYLQMRSLRAEDTAVYYCRA<u>VSDWEDY</u>WGQGTQVTVSS          (SEQ ID NO: 12)

> Variable region of A17m0902

EVQLVESGGGGLVQPGGSLRLSCAASGLTFS<u>DYSMS</u>WYRQAPGKGRELVA<u>IISGSGVITHYVDSVKG</u>

RFTISRDNAKNTLYLQMRSLRAEDTAVYYCRA<u>VSDWEDY</u>WGQGTQVTVSS          (SEQ ID NO: 13)

> Variable region of A17m0903

EVQLVESGGGLVQPGGSLRLSCAASGLTFS<u>DYSMS</u>WYRQAPGKGRELVA<u>IISGSGVITHYVDSVKG</u>
RFTISRDNAKNTVYLQMRSLRAEDTAVYYCRA<u>VSDWEDY</u>WGQGTQVTVSS     (SEQ ID NO: 14)

> Variable region of A17m0905

EVQLVESGGGLVQPGGSLRLSCAASGLTFS<u>DYSMS</u>WYRQAPGKGRELVA<u>IISGSGVITHYVDSVKG</u>
RFTISRDNAKNTVYLQMRSLRAEDRAVYYCRA<u>VSDWEDY</u>WGQGTQVTVSS     (SEQ ID NO: 15)

That is, A17 of the present disclosure has the following general sequence:

CDR1: DYSMS (SEQ ID NO: 3)
CDR2: IISGSGVIX$_1$HYVDSVKG, wherein X$_1$ is selected from the group consisting of T and A (SEQ ID NO: 16)
CDR3: VSDWX$_4$X$_5$Y, wherein X$_4$ is selected from the group consisting of D and E, and X$_5$ is selected from the group consisting of D and E (SEQ ID NO: 17)

Specifically, the CDR2 may be:

IISGSGVIAHYVDSVKG (SEQ ID NO: 4)
IISGSGVITHYVDSVKG (SEQ ID NO: 18)
the CDR3 may be:

VSDWDDY (SEQ ID NO: 5)
VSDWEDY (SEQ ID NO: 19)
VSDWDEY (SEQ ID NO: 20)

## Example 2. Functional Assays of Anti-PD-1 Nanobodies

1. ELISA

Human PD-1 protein (his tag) was dissolved in PBS to form a 1 $\mu$g/mL solution, and the solution was added to a 96-well plate at 100 $\mu$L/well. The plate was coated with the antigen overnight at 4 °C. The plate was washed with a PBST (PBS + 0.05% tween20) solution three times. PBST/1% BSA solution was added, and the plate was incubated at 37 °C for 1 h for blocking. After the plate was washed three times with PBST solution, solutions of the PD-1 candidate VHH-Fc antibodies with different concentrations (diluted with PBST/1% BSA solution) were added, and the plate was incubated at 37 °C for 1.5 h. The plate was washed with PBST solution three times. 100 $\mu$L of HRP-conjugated anti-human IgG4 Fc secondary antibody (Thermo) solution was added, and the plate was incubated at 37 °C for 1 h. After the plate was washed three times with PBST solution, TMB solution was added at 100 $\mu$L/well. After 5 min of reaction at room temperature, 50 $\mu$L of stop solution was added. Then, 450 nM values were measured using a microplate reader, and EC$_{50}$ was calculated. Table 4 shows that A17_hIgG4 exhibited a relatively strong ability to bind to PD-1 antigen.

Table 4. The EC$_{50}$ value of an anti-PD-1 nanobody determined by ELISA

| Antibody No. | EC$_{50}$ (nM) |
|---|---|
| A17_hIgG4 | 0.06099 |

2. FACS

To measure the ability of the anti-PD-1 nanobodies to bind to PD-1 expressed on the cell membrane, solutions of the candidate anti-PD-1 VHH-Fc antibodies with different concentrations were added to 10$^5$/well Jurkat cells over-expressing human PD-1 (Cat: J1250, Promega). The plate was incubated at room temperature for 20 min and then washed with PBS twice. 100 $\mu$L of anti-human IgG Fc fluorescent secondary antibody (Biolegend) was added. The plate was incubated at room temperature for 20 min and then washed with PBS twice, and the cells were resuspended in 250 $\mu$L of PBS. Fluorescence signals were detected by FACS, and EC$_{50}$ was calculated. Pembrolizumab (purchased from Biointron) was used as a control. Table 5 shows that the EC$_{50}$ value of A17 _hIgG4 is 0.3568 nM, which is significantly superior to those of the other antibodies obtained from the same screening process (results not shown).

Table 5. The binding $EC_{50}$ value of an anti-PD-1 nanobody to cell surface PD-1 determined by FACS

| Antibody No. | $EC_{50}$ (nM) |
|---|---|
| A17_hIgG4 | 0.3568 |
| Pembrolizumab | 0.2169 |

3. Assessment of blocking of PD-1 by anti-PD-1 nanobodies using PD-1/PD-L1 reporter gene system

CHO-K1 cells overexpressing PD-L1 were diluted to $4 \times 10^5$/mL and added to a 96-well plate at 100 $\mu$L/well, and the plate was incubated overnight. After the culture medium was pipetted off, 40 $\mu$L of $1.25 \times 10^6$/mL PD-1 Jurkat cells was added rapidly, along with anti-PD-1 nanobody solutions with different concentrations (diluted with 1640 + 2% FBS solution) (40 $\mu$L). The plate was incubated at 37 °C for 6 h and cooled to room temperature. Bright-glo reagent (Cat: E2620, promega) was added at 40 $\mu$L/well. The plate was shaken at 350 rpm for 5 min in a dark place and left to stand at room temperature for 5 min in a dark place. Then, fluorescence values were measured using a microplate reader, and $IC_{50}$ was calculated. Pembrolizumab and a hIgG4 isotype antibody were used as controls. As shown in Table 6, the biologically functional activity $IC_{50}$ value of A17_hIgG4 is 1.199 nM, which is significantly superior to those of the other antibodies obtained from the same screening process in the present disclosure (for example, 7.62 for A3_hIgG4, and 3.208 for A13_hIgG4; the sequences of A3_hIgG4 and A13_hIgG4 are not shown).

Table 6. The activity $IC_{50}$ value of an anti-PD-1 nanobody determined using a PD-1/PD-L1 NFAT reporter gene system

| Antibody No. | $IC_{50}$ (nM) |
|---|---|
| A17_hIgG4 | 1.199 |
| Pembrolizumab | 0.5126 |
| hIgG4 | - |

By using the above method, the anti-PD-1 nanobodies were functionally verified. Referring to Table 7, the $IC_{50}$ activity of A17h1_hIgG4 is similar to that of the positive antibody pembrolizumab. Among the candidate molecules with engineered sequences, the antibodies A17m0901_hIgG4 and A17m0902_hIgG4 have biological activity $IC_{50}$ values of 0.5307 nM and 0.4352 nM, respectively, which are superior to that of pembrolizumab.

Table 7. The activity $IC_{50}$ of engineered anti-PD-1 nanobodies determined using a PD-1/PD-L1 NFAT reporter gene system

| Antibody No. | $IC_{50}$ (nM) |
|---|---|
| A17h1_hIgG4 | 1.039 |
| A17m09_hIgG4 | 1.083 |
| A17m0901_hIgG4 | 0.5307 |
| A17m0902_hIgG4 | 0.4352 |
| A17m0903_hIgG4 | 1.284 |
| A17m0905_hIgG4 | 0.5588 |
| Pembrolizumab | 0.9756 |

4. Identification of binding avidities of engineered anti-PD-1 nanobodies for antigen PD-1

The avidities of the anti-PD-1 nanobodies for PD-1 antigen were determined using surface plasmon resonance (SPR). Biacore 8k (GE Healthcare) was used. In the experiment, a CM5 sensor chip was used, and an HBS-EP+ buffer solution (10 mM HEPES, 150 mM NaCl, 3 mM EDTA, 0.05% surfactant P20) was used as the mobile phase. A 30 $\mu$g/mL solution of an anti-human IgG (Fc) antibody was prepared with a 10 mM sodium acetate buffer (pH 5.0), and the Immobilization program was selected to automatically perform anti-human IgG (Fc) antibody channel amino coupling immobilization. The test antibodies were each diluted in the HBS-EP+ buffer solution as ligands and captured using the anti-human IgG (Fc) antibody on the chip channels. A human antigen protein (Sino Biological, 10377-H08H; 90311-C08H) was used as the analyte. The analyte was serially diluted 2-fold with the HBS-EP+ buffer solution, and the dilution was left to flow through the experimental and reference channels at a flow rate of 30 $\mu$L/min. The association time was 1 min, and the dissociation time was 15 min. 10 mM glycine (pH 1.5) (regeneration buffer, GE Healthcare, BR-1003-54) was run at a flow rate of 10 $\mu$L/min for 30 s. The association rate Ka, the dissociation rate Kd, and the dissociation constant (i.e., avidity $K_D$) were calculated. The results are shown in Table 8.

Table 8. Data on the SPR binding avidities of anti-PD-1 nanobodies for human PD-1 antigen

| Antibody No. | ka (1/Ms) | Kd (1/s) | $K_D$ (M) |
|---|---|---|---|
| A17h1_hIgG4 | 4.30E+05 | 8.68E-04 | 2.02E-09 |
| A17m09_hIgG4 | 4.58E+05 | 1.63E-03 | 3.56E-09 |
| A17m0901_hIgG4 | 2.97E+05 | 1.92E-03 | 6.44E-09 |
| A17m0902_hIgG4 | 2.91E+05 | 1.77E-03 | 6.09E-09 |
| Pembrolizumab | 1.25E+06 | 3.89E-03 | 3.11E-09 |

The results show that the humanized antibody A17h1_hIgG4 and the subsequently engineered and optimized antibodies A17m09_hIgG4, A17m0901_hIgG4, and A17m0902_hIgG4 have $K_D$ values similar to that of pembrolizumab. A17h1_hIgG4, A17m09_hIgG4, A17m0901_hIgG4, and A17m0902_hIgG4 all exhibited slower dissociation than pembrolizumab.

5. Assessment of *in vitro* efficacy of engineered anti-PD-1 nanobodies using DC:T mixed lymphocyte reaction (MLR)

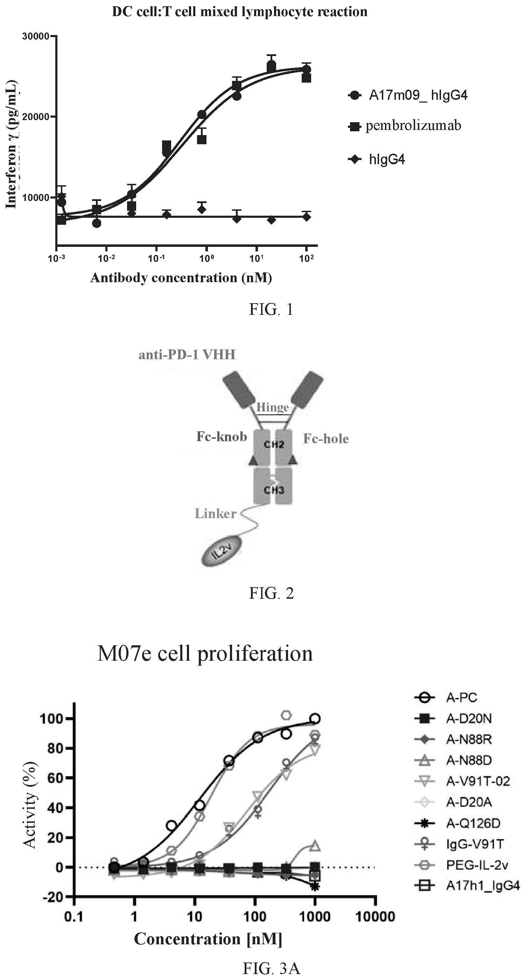

Co-culture of mature dendritic cells (DCs) with allogeneic T cells can activate T cells and promote the secretion of cytokines by T cells. This process is inhibited by PD-1/PD-L1 signaling. The use of a PD-1 antibody can remove the inhibition and promote T cell activation. Thus, a DC:T mixed lymphocyte reaction experiment can be used to assess the *in vitro* efficacy of the PD-1 antibody.
Monocytes were sorted from fresh peripheral blood (PBMCs) of a healthy person using a sorting kit (cat: 19359, stemcell). After seven days of culture using a DC induction kit (cat: 10985, stemcell), the monocytes were induced to differentiate into mature DCs. Then, allogeneic T cells were sorted from fresh PBMCs of another healthy person using a sorting kit (cat: 17951, stemcell). 5000 mature DCs were co-cultured with 50,000 allogeneic T cells, and a candidate antibody was added at a corresponding concentration. After six days of co-culture, the cell supernatant was collected. The IFN-$\gamma$ concentration was determined using a Cisbio-HTRF IFN-$\gamma$ assay kit (Cat: 62HIFNGPEH, Perkinelmer), and $EC_{50}$ was calculated.
As shown in FIG. 1, A17m09_hIgG4 exhibited substantially the same *in vitro* efficacy as pembrolizumab in the MLR experiment. The $EC_{50}$ of A17m09_hIgG4 is 0.2931 nM, and the $EC_{50}$ of pembrolizumab is 0.3271 nM.

**Example 3. Construction and Activity Assay of Fusion Protein of Anti-PD-1 Antibody and IL-2 Mutant (PD-1-IL-2v)**

1. Molecular construction

A fusion protein of an anti-PD-1 antibody and an IL-2 mutant was constructed according to the schematic of a molecular format shown in FIG. 2. For the Fc regions of the molecule in FIG. 2, the Knob-in-Hole (KIH) technique was used; that is, mutations S354C and T366W (constituting a Knob chain) and mutations Y349C, T366S, L368A, and Y407V (Eu numbering scheme) (constituting a Hole chain) were introduced into the two Fc regions, respectively, to promote heterodimer formation. For ease of purification, H435R and Y436F (Eu numbering scheme) were additionally introduced into the Hole chain. In addition, mutations L234A, L235A, and P329G were introduced into both the Knob and Hole chains to eliminate ADCC effects. For heavy chains not paired with light chains, mutation C220S was introduced into the hinge region to remove free, unpaired cysteines. The IL-2 used was IL-2v (V91T), which contains mutations T3A/R38E/F42A/V91T/C125A.
In A-V91T, a molecule of the structure shown in FIG. 2, molecules of the anti-PD-1 nanobody A17m0902 were coupled to the N-termini of the Knob and Hole chains, respectively, by the IgG1 heavy chain hinge region, and IL-2v (V91T) was linked to the C-terminus of the Knob chain of the Fc by a $(G_4S)_3$ linker.
The sequence of the molecule is shown below. The Fc regions are underlined, and the linker is italicized.

> Knob chain of A-V91T

EVQLQESGGGGLVQPGGSLRLSCTASGLTFSDYSMSWYRQAPGKGRELVAIISGSGVIAHYV
DSVKGRFTISRDNSKNTVYLQMRSLTPEDRAVYYCRAVSDWDDYWGQGTQVTVSS<u>EPKS
SDKTHTCPPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALGAPIEKTISKA
KGQPREPQVYTLPPCRDELTKNQVSLWCLVKGFYPSDIAVEWESNGQPENNYKTTPPVLD
SDGSFFLYSKLTVDKSRWQQGNVFSCSVMHEALHNHYTQKSLSLSPG</u>*GGGGSGGGGSGGG
GS*APASSSTKKTQLQLEHLLLDLQMILNGINNYKNPKLTEMLTAKFYMPKKATELKHLQCL
EEELKPLEEVLNLAQSKNFHLRPRDLISNINTIVLELKGSETTFMCEYADETATIVEFLNRWI
TFAQSIISTLT                                                                    (SEQ ID NO: 21)

> Hole chain of A-V91T

EVQLQESGGGGLVQPGGSLRLSCTASGLTFSDYSMSWYRQAPGKGRELVAIISGSGVIAHYV
DSVKGRFTISRDNSKNTVYLQMRSLTPEDRAVYYCRAVSDWDDYWGQGTQVTVSS<u>EPKS
SDKTHTCPPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALGAPIEKTISKA
KGQPREPQVCTLPPSRDELTKNQVSLSCAVKGFYPSDIAVEWESNGQPENNYKTTPPVLDS
DGSFFLVSKLTVDKSRWQQGNVFSCSVMHEALHNRFTQKSLSLSP</u>          (SEQ ID NO: 22)

> IL-2 (containing mutations T3A/R38E/F42A/V91T/C125A)

APASSSTKKTQLQLEHLLLDLQMILNGINNYKNPKLTEMLTAKFYMPKKATELKHLQCLEE
ELKPLEEVLNLAQSKNFHLRPRDLISNINTIVLELKGSETTFMCEYADETATIVEFLNRWITF
AQSIISTLT

(SEQ ID NO: 23)

The above fusion protein was expressed in HEK293 cells and purified through Protein A. It was confirmed by SDS-PAGE that the molecular weight of the purified product agreed with the expectation, indicating that the target protein had been prepared and obtained.

2. Assay for M07e cell proliferation promoting activity of PD-1-IL-2v

M07e is a human cell line that expresses only IL-2Rβ and IL-2Rγ and does not express IL-2Rα. To measure the activity and PD-1 expression dependency of the PD-1-IL2v (V91T) fusion proteins of different formats described above, human PD-1 (SEQ ID NO: 1) was overexpressed in the M07e cell strain to obtain an M07e-hPD-1 cell strain, and the effects of the fusion proteins described above on cell proliferation were assessed in the M07e and M07e-hPD-1 cell strains.

Complete culture solution: RPMI 1640 + 2 mM L-glutamine + 1 mM sodium pyruvate + 10% fetal bovine serum + 15 ng/mL GM-CSF; basic culture medium: RPMI 1640 + 2 mM L-glutamine + 1 mM sodium pyruvate + 10% fetal bovine serum.

M07e cell proliferation experiment: M07e or M07e-hPD1 cells were cultured in a complete culture solution at 37 °C with 5% $CO_2$. The cells were passaged, collected by centrifugation after 1-2 days, washed 3 times with PBS, and resuspended in a basic culture solution to prepare a cell suspension containing $6.0 \times 10^5$ cells per mL. The suspension was added to a 96-well plate at 50 μL/well. 50 μL of a test drug solution ($2\times$ the corresponding concentration) prepared with a basic culture solution was added. After 72 h of cell culture, the CELLTITER-Glo reagent (lysis buffer, Promega) was added at 100 μL/well. After thorough mixing, absorbance was measured at the wavelength of 570 nm with 630 nm as the reference wavelength using a microplate reader.

Data on the proliferation activity of the PD-1-IL-2v (V91T) fusion proteins on M07e and M07e-hPD-1 cells are shown in Table 12. A PEGylated IL-2 mutant (PEG-IL-2v) was used as a positive control. The IL-2 mutant contained mutations T3A, F42A, L72G, N26Q, N29S, N71Q, and C125A. The mutant does not bind to IL-2Rα but can bind to the moderate-avidity IL-2 receptor IL-2Rβγ.

The results show that PEG-IL-2v exhibited comparable proliferation activity on M07e and M07e-hPD-1 cells; the activity of the PD-1-IL-2 (V91T) fusion proteins of different formats on M07e-hPD-1 was significantly stronger than their activity on M07e cell proliferation, indicating that the anti-PD-1 antibody can increase the selectivity of the IL-2 mutant for PD-1-positive cells. In addition, the PD-1-IL-2 (V91T) fusion proteins of different formats exhibited comparable activity on M07e proliferation; however, in terms of activity on M07e-hPD-1 proliferation, C-V91T was slightly stronger than A-V91T, and A-V91T was stronger than B-V91T and E-V91T.

Table 9. The proliferation activity of PD-1-IL-2v (V91T) on M07e and M07e-hPD-1 cells

| Name | M07e | | M07e-hPD1 | |
|---|---|---|---|---|
| | Maximum fluorescence value (Max Lum) | $EC_{50}$ (nM) | Maximum fluorescence value (Max Lum) | $EC_{50}$ (nM) |
| PEG-IL-2v | 4.45E+06 | 20.88 | 7.94E+06 | 13.34 |
| A-V91T | 4.24E+06 | 84.58 | 7.02E+06 | 0.032 |

**Example 4. Construction of PD-1-IL-2v Carrying Different IL-2 Mutants**

PD-1-IL-2v (V91T), despite being less active than PEG-IL-2v in M07e that does not express PD-1, was still able to stimulate cell proliferation, indicating that IL-2v (V91T) can still relatively strongly bind to the moderate-avidity IL-2 receptor IL-2R$\beta\gamma$, and that PD-1-IL-2 (V91T) may still systemically activate NK and CD8$^+$ T cells and cause toxicity. To further reduce the toxicity of PD-1-IL-2v and make the activity of IL-2 more dependent on PD-1 expression, PD-1-IL-2v fusion proteins containing different IL-2 mutants were constructed based on the molecular format of A-V91T. All the IL-2 mutants contained mutations F42A and L72G to eliminate the binding of IL-2 to IL-2R$\alpha$. In addition, considering stability, N26Q, N29S, and N71Q were introduced into the IL-2 mutants to remove potential deamidation modifications in IL-2, thereby increasing the stability of IL-2. Moreover, they contained their respective different unique mutations, which are shown in Table 10. The mutations reduce the avidity of IL-2 for the moderate-avidity receptor IL-2R$\beta\gamma$ to varying extents.

Table 10. IL-2 mutations in PD-1-IL-2v

| Name | Common mutations in IL-2 | Unique mutations in IL-2 | Corresponding sequence number |
|---|---|---|---|
| A-PC | T3A/F42A/L72G/N26Q/N29S/N71Q/C125A | / | SEQ ID NO:24 |
| A-V91T-02 | | V91T | SEQ ID NO:25 |
| A-D20A | | D20A | SEQ ID NO:26 |
| A-D20N | | D20N | SEQ ID NO:27 |
| A-N88D | | N88D | SEQ ID NO:28 |
| A-N88R | | N88R | SEQ ID NO:29 |
| A-Q126D | | Q126D | SEQ ID NO:30 |
| A-H16A/D84S | | H16A/D84S | SEQ ID NO:31 |

> IL-2 sequence in A-PC

APASSSTKKTQLQLEHLLLDLQMILQGISNYKNPKLTRMLTAKFYMPKKATELKHLQCLEE
ELKPLEEVLQGAQSKNFHLRPRDLISNINVIVLELKGSETTFMCEYADETATIVEFLNRWITF
AQSIISTLT

(SEQ ID NO: 24)

> IL-2 sequence in A-V91T-02

APASSSTKKTQLQLEHLLLDLQMILQGISNYKNPKLTRMLTAKFYMPKKATELKHLQCLEE
ELKPLEEVLQGAQSKNFHLRPRDLISNINTIVLELKGSETTFMCEYADETATIVEFLNRWITF
AQSIISTLT

(SEQ ID NO: 25)

> IL-2 sequence in A-D20A

APASSSTKKTQLQLEHLLLALQMILQGISNYKNPKLTRMLTAKFYMPKKATELKHLQCLEE
ELKPLEEVLQGAQSKNFHLRPRDLISNINVIVLELKGSETTFMCEYADETATIVEFLNRWITF
AQSIISTLT

(SEQ ID NO: 26)

> IL-2 sequence in A-D20N

APASSSTKKTQLQLEHLLLNLQMILQGISNYKNPKLTRMLTAKFYMPKKATELKHLQCLEE
ELKPLEEVLQGAQSKNFHLRPRDLISNINVIVLELKGSETTFMCEYADETATIVEFLNRWITF
AQSIISTLT

(SEQ ID NO: 27)

> IL-2 sequence in A-N88D

APASSSTKKTQLQLEHLLLDLQMILQGISNYKNPKLTRMLTAKFYMPKKATELKHLQCLEE
ELKPLEEVLQGAQSKNFHLRPRDLISDINVIVLELKGSETTFMCEYADETATIVEFLNRWITF
AQSIISTLT

(SEQ ID NO: 28)

> IL-2 sequence in A-N88R

APASSSTKKTQLQLEHLLLDLQMILQGISNYKNPKLTRMLTAKFYMPKKATELKHLQCLEE
ELKPLEEVLQGAQSKNFHLRPRDLISRINVIVLELKGSETTFMCEYADETATIVEFLNRWITF
AQSIISTLT

(SEQ ID NO: 29)

> IL-2 sequence in A-Q126D

APASSSTKKTQLQLEHLLLDLQMILQGISNYKNPKLTRMLTAKFYMPKKATELKHLQCLEE
ELKPLEEVLQGAQSKNFHLRPRDLISNINVIVLELKGSETTFMCEYADETATIVEFLNRWITF
ADSIISTLT

(SEQ ID NO: 30)

> IL-2 sequence in A-H16A/D84S

APASSSTKKTQLQLEALLLDLQMILQGISNYKNPKLTRMLTAKFYMPKKATELKHLQCLEE
ELKPLEEVLQGAQSKNFHLRPRSLISNINVIVLELKGSETTFMCEYADETATIVEFLNRWITF
AQSIISTLT

(SEQ ID NO: 31)

Mutations L234A and L235A were introduced into the Fc regions to eliminate ADCC effects. Mutations S354C and T366W (constituting the Knob chain) and mutations Y349C, T366S, L368A, and Y407V (Eu numbering scheme) (constituting the Hole chain) were introduced to promote heterodimer formation. In addition, mutation C220S was introduced into the hinge region. For ease of purification, the antibodies may contain mutations H435R and Y436F (Eu numbering scheme) in the Hole chain. Furthermore, the amino acids at positions 356 and 358 of the Hole chains of the Fc regions of these antibodies may be D356 and L358, or E356 and M358 (Eu numbering scheme), respectively. They appeared in different alleles and did not affect antibody function.

> Knob chain of Fc (D356, L358)

EPKSSDKTHTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTIS
KAKGQPREPQVYTLPPCRDELTKNQVSLWCLVKGFYPSDIAVEWESNGQPENNYKTTPPV
LDSDGSFFLYSKLTVDKSRWQQGNVFSCSVMHEALHNHYTQKSLSLSPG

(SEQ ID NO: 32)

> Hole chain of Fc (D356, L358)

EPKSSDKTHTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTIS
KAKGQPREPQVCTLPPSRDELTKNQVSLSCAVKGFYPSDIAVEWESNGQPENNYKTTPPVL
DSDGSFFLVSKLTVDKSRWQQGNVFSCSVMHEALHNHYTQKSLSLSP     (SEQ ID NO: 33)

> Knob chain of Fc (E356, M358)

EPKSSDKTHTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTIS
KAKGQPREPQVYTLPPCREEMTKNQVSLWCLVKGFYPSDIAVEWESNGQPENNYKTTPPV
LDSDGSFFLYSKLTVDKSRWQQGNVFSCSVMHEALHNHYTQKSLSLSPG

(SEQ ID NO: 34)

> Hole chain of Fc (E356, M358)

EPKSSDKTHTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTIS
KAKGQPREPQVCTLPPSREEMTKNQVSLSCAVKGFYPSDIAVEWESNGQPENNYKTTPPVL
DSDGSFFLVSKLTVDKSRWQQGNVFSCSVMHEALHNHYTQKSLSLSPG

(SEQ ID NO: 35)

Illustratively, the full-length sequences of A-D20A and A-N88D are shown. The Fc regions are underlined, and the linker is italicized.

> Knob chain of A-D20A (D356, L358)

EVQLVESGGGLVQPGGSLRLSCAASGLTFSDYSMSWYRQAPGKGRELVAIISGSGVITHYV
DSVKGRFTISRDNAKNTLYLQMRSLRAEDTAVYYCRAVSDWEDYWGQGTQVTVSSEPKS
SDKTHTCPPCPAPEAAGGPSVFLFPPKPKDTLMISRTPEVTCVVVDVSHEDPEVKFNWYVD

GVEVHNAKTKPREEQYNSTYRVVSVLTVLHQDWLNGKEYKCKVSNKALPAPIEKTISKAK
GQPREPQVYTLPPCRDELTKNQVSLWCLVKGFYPSDIAVEWESNGQPENNYKTTPPVLDS
DGSFFLYSKLTVDKSRWQQGNVFSCSVMHEALHNHYTQKSLSLSPG*GGGGSGGGGSGGG
GS*APASSSTKKTQLQLEHLLLALQMILQGISNYKNPKLTRMLTAKFYMPKKATELKHLQCL
EEELKPLEEVLQGAQSKNFHLRPRDLISNINVIVLELKGSETTFMCEYADETATIVEFLNRWI
TFAQSIISTLT

(SEQ ID NO: 36)

> Hole chain of A-D20A (D356, L358)

EVQLVESGGGLVQPGGSLRLSCAASGLTFSDYSMSWYRQAPGKGRELVAIISGSGVITHYV
DSVKGRFTISRDNAKNTLYLQMRSLRAEDTAVYYCRAVSDWEDYWGQGTQVTVSSEPKS
SDKTHTCPPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKAK
GQPREPQVCTLPPSRDELTKNQVSLSCAVKGFYPSDIAVEWESNGQPENNYKTTPPVLDSD
GSFFLVSKLTVDKSRWQQGNVFSCSVMHEALHNRFTQKSLSLSP

(SEQ ID NO: 37)

> Knob chain of A-D20A (E356, M358)

EVQLVESGGGLVQPGGSLRLSCAASGLTFSDYSMSWYRQAPGKGRELVAIISGSGVITHYV
DSVKGRFTISRDNAKNTLYLQMRSLRAEDTAVYYCRAVSDWEDYWGQGTQVTVSSEPKS
SDKTHTCPPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKAK
GQPREPQVYTLPPCREEMTKNQVSLWCLVKGFYPSDIAVEWESNGQPENNYKTTPPVLDS
DGSFFLYSKLTVDKSRWQQGNVFSCSVMHEALHNHYTQKSLSLSPGGGGGSGGGGSGGG
GSAPASSSTKKTQLQLEHLLLALQMILQGISNYKNPKLTRMLTAKFYMPKKATELKHLQCL
EELKPLEEVLQGAQSKNFHLRPRDLISNINVIVLELKGSETTFMCEYADETATIVEFLNRWI
TFAQSIISTLT

(SEQ ID NO: 38)

> Hole chain of A-D20A (E356, M358)

EVQLVESGGGLVQPGGSLRLSCAASGLTFSDYSMSWYRQAPGKGRELVAIISGSGVITHYV
DSVKGRFTISRDNAKNTLYLQMRSLRAEDTAVYYCRAVSDWEDYWGQGTQVTVSSEPKS
SDKTHTCPPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKAK
GQPREPQVCTLPPSREEMTKNQVSLSCAVKGFYPSDIAVEWESNGQPENNYKTTPPVLDSD
GSFFLVSKLTVDKSRWQQGNVFSCSVMHEALHNHYTQKSLSLSPG

(SEQ ID NO: 39)

> Knob chain of A-N88D (D356, L358)

EVQLVESGGGLVQPGGSLRLSCAASGLTFSDYSMSWYRQAPGKGRELVAIISGSGVITHYV
DSVKGRFTISRDNAKNTLYLQMRSLRAEDTAVYYCRAVSDWEDYWGQGTQVTVSSEPKS
SDKTHTCPPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKAK
GQPREPQVYTLPPCRDELTKNQVSLWCLVKGFYPSDIAVEWESNGQPENNYKTTPPVLDS
DGSFFLYSKLTVDKSRWQQGNVFSCSVMHEALHNHYTQKSLSLSPGGGGGSGGGGSGGG
GSAPASSSTKKTQLQLEHLLLDLQMILQGISNYKNPKLTRMLTAKFYMPKKATELKHLQCL
EELKPLEEVLQGAQSKNFHLRPRDLISDINVIVLELKGSETTFMCEYADETATIVEFLNRWI
TFAQSIISTLT

(SEQ ID NO: 40)

> Hole chain of A-N88D (D356, L358)

EVQLVESGGGLVQPGGSLRLSCAASGLTFSDYSMSWYRQAPGKGRELVAIISGSGVITHYV
DSVKGRFTISRDNAKNTLYLQMRSLRAEDTAVYYCRAVSDWEDYWGQGTQVTVSSEPKS

SDKTHTCPPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKAK
GQPREPQVCTLPPSRDELTKNQVSLSCAVKGFYPSDIAVEWESNGQPENNYKTTPPVLDSD
GSFFLVSKLTVDKSRWQQGNVFSCSVMHEALHNHYTQKSLSLSP

(SEQ ID NO: 41)

> Knob chain of A-N88D (E356, M358)

EVQLVESGGGLVQPGGSLRLSCAASGLTFSDYSMSWYRQAPGKGRELVAIISGSGVITHYV
DSVKGRFTISRDNAKNTLYLQMRSLRAEDTAVYYCRAVSDWEDYWGQGTQVTVSSEPKS
SDKTHTCPPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKAK
GQPREPQVYTLPPCREEMTKNQVSLWCLVKGFYPSDIAVEWESNGQPENNYKTTPPVLDS
DGSFFLYSKLTVDKSRWQQGNVFSCSVMHEALHNHYTQKSLSLSPG*GGGGSGGGGSGGG
GS*APASSSTKKTQLQLEHLLLDLQMILQGISNYKNPKLTRMLTAKFYMPKKATELKHLQCL
EEELKPLEEVLQGAQSKNFHLRPRDLISDINVIVLELKGSETTFMCEYADETATIVEFLNRWI
TFAQSIISTLT

(SEQ ID NO: 42)

> Hole chain of A-N88D (E356, M358)

EVQLVESGGGLVQPGGSLRLSCAASGLTFSDYSMSWYRQAPGKGRELVAIISGSGVITHYV
DSVKGRFTISRDNAKNTLYLQMRSLRAEDTAVYYCRAVSDWEDYWGQGTQVTVSSEPKS
SDKTHTCPPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKAK
GQPREPQVCTLPPSREEMTKNQVSLSCAVKGFYPSDIAVEWESNGQPENNYKTTPPVLDSD
GSFFLVSKLTVDKSRWQQGNVFSCSVMHEALHNHYTQKSLSLSPG

(SEQ ID NO: 43)

In addition, to demonstrate the dependency of PD-1-IL-2v on PD-1, the anti-PD-1 antibody moieties in some PD-1-IL-2v fusion proteins were replaced with antibodies against irrelevant antigens, and the mutations in the IL-2 moieties were kept. This resulted in IgG-PC, IgG-V91T (containing V91T in IL-2), IgG-D20A (containing D20A in IL-2), and IgG-N88D (containing N88D in IL-2). The difference between IgG-PC and IgG-V91T, IgG-D20A, or IgG-N88D lies only in the IL-2 moiety. Their sequences are shown below:

> Knob chain of IgG-PC

QVQLVQSGAEVKKPGASVKVSCKASGYTFTNSWIGWFRQAPGQGLEWIGDIYPGGGYTN
YNEIFKGKATMTADTSTNTAYMELSSLRSEDTAVYYCSRGIPGYAMDYWGQGTLVTVSSA
STKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPEAAGGPSVF
LFPPKPKDTLMISRTPEVTCVVVDVSHEDPEVKFNWYVDGVEVHNAKTKPREEQYNSTYR
VVSVLTVLHQDWLNGKEYKCKVSNKALGAPIEKTISKAKGQPREPQVYTLPPCRDELTKN
QVSLWCLVKGFYPSDIAVEWESNGQPENNYKTTPPVLDSDGSFFLYSKLTVDKSRWQQGN
VFSCSVMHEALHNHYTQKSLSLSPG*GGGGSGGGGSGGGGS*APASSSTKKTQLQLEHLLLDL
QMILQGISNYKNPKLTRMLTAKFYMPKKATELKHLQCLEEELKPLEEVLQGAQSKNFHLR
PRDLISNINVIVLELKGSETTFMCEYADETATIVEFLNRWITFAQSIISTLT

(SEQ ID NO: 44)

> Knob chain of IgG-V91T

QVQLVQSGAEVKKPGASVKVSCKASGYTFTNSWIGWFRQAPGQGLEWIGDIYPGGGYTN
YNEIFKGKATMTADTSTNTAYMELSSLRSEDTAVYYCSRGIPGYAMDYWGQGTLVTVSSA
STKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPEAAGGPSVF

LFPPKPKDTLMISRTPEVTCVVVDVSHEDPEVKFNWYVDGVEVHNAKTKPREEQYNSTYR
VVSVLTVLHQDWLNGKEYKCKVSNKALGAPIEKTISKAKGQPREPQVYTLPPCRDELTKN
QVSLWCLVKGFYPSDIAVEWESNGQPENNYKTTPPVLDSDGSFFLYSKLTVDKSRWQQGN
VFSCSVMHEALHNHYTQKSLSLSPG*GGGGSGGGGSGGGGS*APASSSTKKTQLQLEHLLLDL
QMILNGINNYKNPKLTEMLTAKFYMPKKATELKHLQCLEEELKPLEEVLNLAQSKNFHLR
PRDLISNINTIVLELKGSETTFMCEYADETATIVEFLNRWITFAQSIISTLT

(SEQ ID NO: 45)

> Knob chain of IgG-N88D

QVQLVQSGAEVKKPGASVKVSCKASGYTFTNSWIGWFRQAPGQGLEWIGDIYPGGGYTN
YNEIFKGKATMTADTSTNTAYMELSSLRSEDTAVYYCSRGIPGYAMDYWGQGTLVTVSSA
STKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPEAAGGPSVF
LFPPKPKDTLMISRTPEVTCVVVDVSHEDPEVKFNWYVDGVEVHNAKTKPREEQYNSTYR
VVSVLTVLHQDWLNGKEYKCKVSNKALGAPIEKTISKAKGQPREPQVYTLPPCRDELTKN
QVSLWCLVKGFYPSDIAVEWESNGQPENNYKTTPPVLDSDGSFFLYSKLTVDKSRWQQGN
VFSCSVMHEALHNHYTQKSLSLSPG*GGGGSGGGGSGGGGS*APASSSTKKTQLQLEHLLLDL
QMILQGISNYKNPKLTRMLTAKFYMPKKATELKHLQCLEEELKPLEEVLQGAQSKNFHLR
PRDLISDINVIVLELKGSETTFMCEYADETATIVEFLNRWITFAQSIISTLT

(SEQ ID NO: 46)

> Knob chain of IgG-D20A

QVQLVQSGAEVKKPGASVKVSCKASGYTFTNSWIGWFRQAPGQGLEWIGDIYPGGGYTN
YNEIFKGKATMTADTSTNTAYMELSSLRSEDTAVYYCSRGIPGYAMDYWGQGTLVTVSSA
STKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPEAAGGPSVF
LFPPKPKDTLMISRTPEVTCVVVDVSHEDPEVKFNWYVDGVEVHNAKTKPREEQYNSTYR
VVSVLTVLHQDWLNGKEYKCKVSNKALGAPIEKTISKAKGQPREPQVYTLPPCRDELTKN
QVSLWCLVKGFYPSDIAVEWESNGQPENNYKTTPPVLDSDGSFFLYSKLTVDKSRWQQGN
VFSCSVMHEALHNHYTQKSLSLSPG*GGGGSGGGGSGGGGS*APASSSTKKTQLQLEHLLLAL
QMILQGISNYKNPKLTRMLTAKFYMPKKATELKHLQCLEEELKPLEEVLQGAQSKNFHLR
PRDLISNINVIVLELKGSETTFMCEYADETATIVEFLNRWITFAQSIISTLT

(SEQ ID NO: 47)

> Hole chain of IgG-PC, IgG-V91T, IgG-D20A, or IgG-N88D

QVQLVQSGAEVKKPGASVKVSCKASGYTFTNSWIGWFRQAPGQGLEWIGDIYPGGGYTN
YNEIFKGKATMTADTSTNTAYMELSSLRSEDTAVYYCSRGIPGYAMDYWGQGTLVTVSSA
STKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPEAAGGPSVF
LFPPKPKDTLMISRTPEVTCVVVDVSHEDPEVKFNWYVDGVEVHNAKTKPREEQYNSTYR
VVSVLTVLHQDWLNGKEYKCKVSNKALGAPIEKTISKAKGQPREPQVCTLPPSRDELTKN
QVSLSCAVKGFYPSDIAVEWESNGQPENNYKTTPPVLDSDGSFFLVSKLTVDKSRWQQGN
VFSCSVMHEALHNRFTQKSLSLSP

(SEQ ID NO: 48)

> Light chain of IgG-PC, IgG-V91T, IgG-D20A, or IgG-N88D

DIQMTQSPSSLSASVGDRVTMSCKSSQSLLNSGDQKNYLTWYQQKPGKAPKLLIYWASTG
ESGVPSRFSGSGSGTDFTFTISSLQPEDIATYYCQNDYSYPWTFGQGTKVEIKRTVAAPSVFI
FPPSDEQLKSGTASVVCLLNNFYPREAKVQWKVDNALQSGNSQESVTEQDSKDSTYSLSST
LTLSKADYEKHKVYACEVTHQGLSSPVTKSFNRGEC

(SEQ ID NO: 49)

The above fusion proteins were expressed in HEK293 cells and purified through Protein A. It was confirmed by SDS-PAGE that the molecular weight of the purified products agreed with the expectation, indicating that the target proteins had been obtained.

In addition, the wild-type IL-2 sequence of the present disclosure is:
> wt IL-2

APTSSSTKKTQLQLEHLLLDLQMILNGINNYKNPKLTRMLTFKFYMPKKATELKHLQCLEE
ELKPLEEVLNLAQSKNFHLRPRDLISNINVIVLELKGSETTFMCEYADETATIVEFLNRWITF
CQSIISTLT

(SEQ ID NO: 50)

## Example 5. Assay for M07e Cell Proliferation Promoting Activity of PD-1-IL-2v

To measure the activity and PD-1 expression dependency of the PD-1-IL-2v fusion proteins containing different IL-2 mutants described above, the effects of the fusion proteins of Example 4 on cell proliferation were assessed in the M07e and M07e-hPD-1 cell strains. See Example 3 for the experimental method. The relative cell proliferation activity of PD-1-IL-2v was calculated as follows:

$$Activity^{Conc}_{PD-1-IL-2v} = 100\% * \frac{Lum^{Conc}_{PD-1-IL-2v} - Lum_{Medium}}{Lum^{1000\,nM}_{A-PC} - Lum_{Medium}},$$

wherein $Activity^{Conc}_{PD-1-IL-2v}$ represents the activity of PD-1-IL-2v at a concentration of conc, $Lum^{Conc}_{PD-1-IL-2v}$ represents the luminescence intensity of the cell lysate when stimulated by PD-1-IL-2v at a concentration of conc, $Lum^{1000\,nM}_{A-PC}$ represents the luminescence intensity of the cell lysate after the cells were stimulated by the positive control molecule A-PC at a concentration of 1000 nM in the M07e or M07e-hPD-1 cell strain, and $Lum_{Medium}$ represents the background luminescence intensity when no PD-1-IL2v or PEG-IL-2v stimulation was used in the M07e or M07e-hPD-1 cell strain and only the culture medium was added.

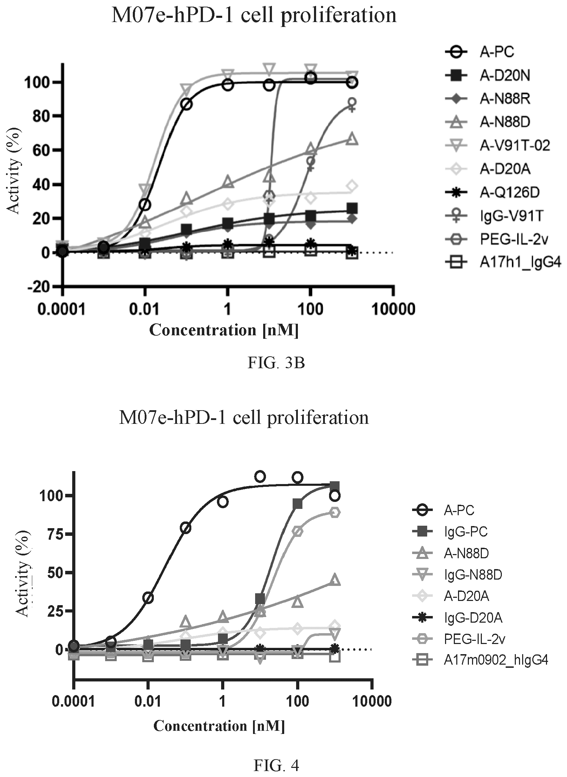

The experimental results are shown in Table 11 and FIGs. 3A and 3B. The results show that: 1) In M07e cells, A-PC and PEG-IL-2v exhibited comparable activity, indicating that in the absence of PD-1 overexpression, the activity of the IL-2 binding to the antibody is consistent with that of the PEG-conjugated IL-2; A-V91T and IgG-V91T exhibited comparable activity, and their activity was weaker than that of A-PC, indicating that V91T did reduce the activity of IL-2, but it still can stimulate M07e proliferation; other mutants, including D20A, D20N, N88R, N88D, and Q126D, barely activated M07e cells, and only A-N88D exhibited weak activity at a concentration of 1000 nM.2) In M07e-hPD-1 cells, due to the overexpression of PD-1 on the cells, the activity of the PD-1-IL-2v fusion proteins at relatively low concentrations far exceeded that of IgG-V91T and PEG-IL-2v, indicating that the anti-PD-1 antibody greatly increased the dependency of the cell proliferation promoting activity of PD-1-IL-2v on PD-1 expression; the other PD-1-IL-2v fusion proteins exhibited slightly lower maximum activity on M07e-hPD-1 cell proliferation than A-PC and A-V91T. 3) A comparison of the activity of the PD-1-IL-2v fusion proteins in the M07e and M07e-hPD-1 cell proliferation experiments shows that the PD-1-IL2v fusion proteins containing mutation D20A, D20N, N88R, N88D, or Q126D barely activate M07e cells but relatively strongly stimulated M07e-hPD-1 cell proliferation, indicating that the PD-1-IL-2v fusion proteins reduced the activation of PD-1-negative cells while retaining the selective activation of PD-1-positive cells; although A-PC and A-V91T have selective activation of PD-1-positive cells, they can also activate PD-1-negative cells.

Table 11. The proliferation promoting activity of PD-1-IL-2v fusion proteins containing different IL-2 mutations on M07e and M07e-hPD-1 cells

| Name | M07e cell proliferation activity (%) | | | | | | | |
|---|---|---|---|---|---|---|---|---|
| | 1000 nM | 333 nM | 111 nM | 37 nM | 12 nM | 4.1 nM | 1.4 nM | 0.46 nM |
| PEG-IL-2v | 89.0 | 102.2 | 88.3 | 68.2 | 34.2 | 11.2 | 2.6 | 0.7 |
| IgG-V91T | 85.3 | 68.5 | 36.9 | 23.9 | 6.1 | 1.7 | -0.1 | 2.0 |
| A-PC | 100.0 | 89.8 | 87.2 | 71.7 | 41.6 | 28.1 | 3.6 | 0.1 |
| A-V91T-02 | 78.7 | 62.0 | 47.3 | 26.6 | 1.7 | -3.3 | -4.4 | -4.6 |
| A-D20A | 0.6 | -1.9 | -2.4 | -3.7 | -3.0 | -1.6 | -2.6 | -3.0 |
| A-D20N | 0.3 | -0.9 | 0.1 | -0.6 | -0.7 | 0.2 | -1.3 | -0.8 |
| A-N88R | -5.1 | -5.2 | -4.5 | -3.0 | -2.2 | -1.9 | -1.3 | -0.9 |
| A-N88D | 14.6 | 0.2 | -1.8 | -2.8 | -1.9 | -1.5 | -1.1 | -1.7 |
| A-Q126D | -12.8 | -5.8 | -2.9 | -2.7 | -0.5 | -1.7 | -0.6 | -0.8 |
| Al7hl_IgG4 | -5.8 | -3.5 | -3.0 | -1.2 | -1.3 | -0.2 | 0.0 | -1.4 |
| Name | M07e-hPD-1 cell proliferation activity (%) | | | | | | | |
| | 1000 nM | 100 nM | 10 nM | 1 nM | 0.1 nM | 0.01 nM | 0.001 nM | 0.0001 nM |
| PEG-IL-2v | 100.1 | 104.0 | 33.3 | 3.3 | 0.4 | 0.5 | 1.1 | 1.9 |
| IgG-V91T | 86.3 | 51.7 | 6.1 | 1.1 | -0.4 | 0.2 | 0.5 | 1.6 |
| A-PC | 100 | 102 | 98 | 99 | 87 | 28 | 3 | 1 |
| A-V91T-02 | 102.9 | 107.0 | 107.4 | 103.9 | 95.5 | 36.6 | 5.1 | 3.4 |
| A-D20A | 39.2 | 32.0 | 30.2 | 28.5 | 24.2 | 11.4 | 2.9 | 2.3 |
| A-D20N | 26.0 | 21.6 | 19.9 | 17.2 | 13.6 | 5.4 | 2.0 | 1.8 |
| A-N88R | 20.0 | 16.8 | 16.9 | 14.9 | 11.5 | 4.8 | 1.8 | 1.3 |
| A-N88D | 66.9 | 61.1 | 45.0 | 41.8 | 32.1 | 18.1 | 3.3 | 2.8 |
| A-Q126D | 1.1 | 5.4 | 6.3 | 5.1 | 3.3 | 1.6 | 1.4 | -0.1 |
| A17h1_IgG4 | -0.1 | 1.6 | 0.7 | 0.3 | 0.0 | 0.3 | 0.2 | 1.8 |

To better demonstrate the dependency of PD-1-IL-2v (N88D) and PD-1-IL-2v (D20A) on PD-1, the effects of A-N88D and IgG-N88D, as well as A-D20A and IgG-D20, on cell proliferation were compared in the M07e-hPD-1 cell strain. The experimental results are shown in Table 12 and FIG. 4. The results show that at the relatively high concentration (1000 nM), IgG-N88D and IgG-D20A still cannot activate M07e-hPD-1 cell proliferation; however, A-N88D and A-D20A can activate M07e-hPD-1 cell proliferation at relatively low concentrations.

Table 12. The proliferation promoting activity of PD-1-IL-2v or IgG-IL-2v on M07e-hPD-1 cells

| Name | M07e-hPD-1 cell proliferation percentage (%) | | | | | | | |
|---|---|---|---|---|---|---|---|---|
| | 1000 nM | 100 nM | 10 nM | 1 nM | 0.1 nM | 0.01 nM | 0.001 nM | 0.0001 nM |
| A-PC | 100.0 | 112.0 | 112.6 | 95.9 | 79.0 | 33.7 | 4.9 | 2.4 |
| IgG-PC | 106.0 | 94.7 | 32.9 | 6.9 | 2.9 | 3.1 | 1.8 | 1.2 |
| A-N88D | 45.5 | 31.1 | 25.2 | 21.3 | 18.3 | 4.8 | 0.0 | 0.8 |
| IgG-N88D | 9.7 | -1.3 | -5.6 | -1.3 | -0.5 | -1.5 | -0.6 | -1.2 |
| A-D20A | 15.4 | 13.5 | 10.8 | 10.8 | 8.4 | 2.6 | -0.3 | 0.6 |
| IgG-D20A | 0.6 | -0.3 | 0.3 | -0.1 | -0.2 | 0.1 | 0.0 | 1.1 |
| PEG-IL-2v | 89.1 | 76.7 | 24.4 | -0.6 | -2.2 | -2.6 | -2.1 | -2.2 |
| A17m0902_hIgG4 | -4.4 | -2.0 | -2.4 | -2.9 | -3.3 | -4.2 | -3.5 | -3.1 |

**Example 6. Assay for T Cell Proliferation Promoting Activity of PD-1-IL-2v**

Unstimulated T cells do not express or lowly express PD-1. T cells stimulated with CD3 antibodies highly express PD-1. To determine whether the PD-1-IL-2v fusion proteins containing different IL-2 mutants described above can activate T cells among human peripheral blood mononuclear cells (PBMCs) that lowly express PD-1 and highly express PD-1, T cells were isolated from PBMCs and stimulated with a CD3 antibody. The effects of the fusion proteins described above on the proliferation of unstimulated and stimulated T cells were assessed.

Proliferation experiment of T cells stimulated with a CD3 antibody: After frozen human PBMCs were thawed, T cells were obtained by isolation using a Pan T Cell Isolation Kit (Miltenyi, 130-096-535). The CD3 antibody was diluted with PBS to 0.1 $\mu$g/mL and added to a 10 cm culture dish. The dish was coated at 37 °C for 2 h and washed with PBS for later use. The T cells were plated at $2 \times 10^6$ cells/mL onto the 10 cm culture dish pre-coated with the CD3 antibody and stimulated at 37 °C with 5% $CO_2$ for 24 h. The stimulated T cells were collected, washed, and counted, and the cell density was then adjusted to $5 \times 10^5$ cells/mL. The cells were plated at 200 $\mu$L/well onto a 96-well plate. The test samples were serially diluted with the culture medium to prepare 5× working solutions, and 50 $\mu$L of each of the test sample solutions with different concentrations was added to the stimulated T cells and mixed well. The plate was incubated for 4 days. After drug treatment, the cells were washed twice with FACS buffer (PBS + 2% FBS), and dead cells were labeled with Fixable Viability Dye (eBioscience, 65-0865-14). After flow cytometry antibody staining with CD3 (BD, 565491), CD4 (BD, 558116), CD8 (BD, 565310), and Ki-67 (eBioscience, 12-5698-82), cell proliferation was assessed by FACS.

Unstimulated T cell proliferation experiment: The isolated T cells were counted and then plated at $5 \times 10^5$ cells/mL onto a 96-well plate at 200 $\mu$L/well. The test samples were serially diluted with the culture medium to prepare 5× working solutions, and 50 $\mu$L of each of the test sample solutions with different concentrations was added to stimulated T cells and mixed well. The plate was incubated for 4 days. After drug treatment, the cells were washed twice with FACS buffer, and dead cells were labeled with FVD780. After flow cytometry antibody staining, cell proliferation was assessed by FACS.

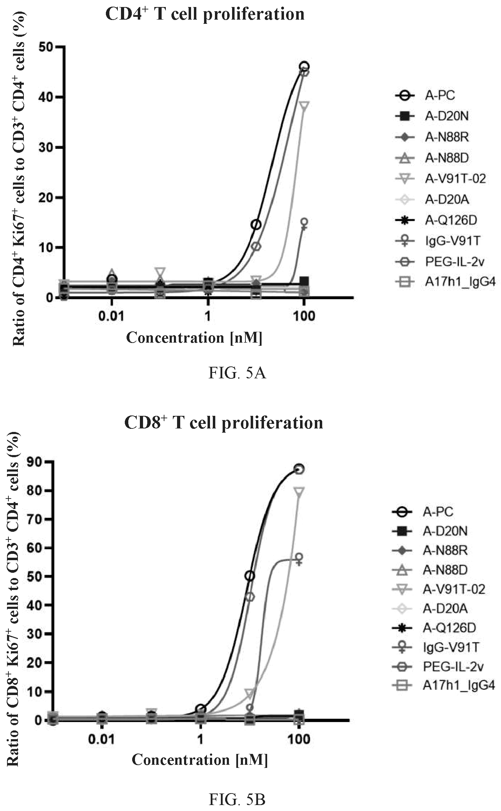

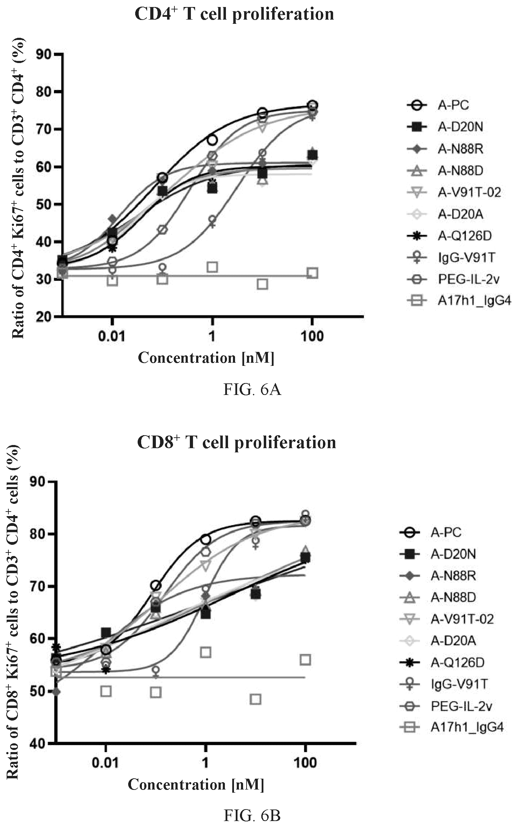

The experimental results are shown in Tables 13 and 14 and FIGs. 5A, 5B, 6A, and 6B. The results show that: 1) In the unstimulated T cells, A-PC exhibited T proliferation activating activity at concentrations above 1 nM (CD8$^+$ T cell proliferation EC$_{50}$ = 21.46 nM, CD4$^+$ T cell proliferation EC$_{50}$ = 8.61 nM); A-V91T exhibited weaker activity than A-PC, indicating that V91T did reduce IL-2 activity, but it still can promote the proliferation of the unstimulated T cells; other mutants, including D20A, D20N, N88R, N88D, and Q126D, did not exhibit T cell activation at a concentration of 100 nM. 2) In the T cells stimulated with the CD3 antibody, the PD-1-IL-2v fusion proteins exhibited significant T cell proliferation promoting activity at a mere relatively low concentration (0.1 nM). The PD-1-IL-2v fusion proteins carrying other mutations exhibited slightly lower maximum activity on the proliferation of the pan T cells stimulated with the CD3 antibody than A-PC and A-V91T. 3) A comparison of the activity of the fusion proteins in the proliferation experiments of the unstimulated T cells and the T cells stimulated with the CD3 antibody shows that the PD-1-IL-2v fusion proteins containing mutation D20A, D20N, N88R, N88D, or Q126D did not activate the proliferation of the unstimulated T cells but relatively strongly stimulated the proliferation of the T cells stimulated with the CD3 antibody; although A-PC and A-V91T can more strongly activate the proliferation of the T cells stimulated with the CD3 antibody, they can also relatively strongly activate the T cells not stimulated with the CD3 antibody.

Table 13. The proliferation activity of PD-1-IL-2v fusion proteins carrying different IL-2 mutations on unstimulated T cells

| Name | CD4$^+$ Ki67$^+$ cells (%) | | | | | |
|---|---|---|---|---|---|---|
| | 100 nM | 10 nM | 1 nM | 0.1 nM | 0.01 nM | 0.001 nM |
| PEG-IL-2v | 45 | 10.2 | 1.77 | 1.38 | 1.51 | 0.29 |
| IgG-V91T | 14.6 | 1.58 | 1.61 | 1.88 | 2.74 | 1.99 |
| A-PC | 46.1 | 14.6 | 2.83 | 1.31 | 3.6 | 1.42 |
| A-V91T-02 | 38.1 | 3.31 | 2.84 | 4.99 | 2.28 | 2.57 |
| A-D20A | 1.37 | 2.7 | 2.66 | 0.94 | 1.47 | 1.91 |
| A-D20N | 3.24 | 1.53 | 3.01 | 2.03 | 2.2 | 1.85 |
| A-N88R | 1.01 | 2.83 | 2.52 | 2.22 | 3.2 | 1.35 |
| A-N88D | 1.84 | 1.71 | 3.13 | 3.48 | 4.72 | 1.42 |
| A-Q126D | 2.97 | 1.78 | 1.23 | 2.74 | 2.64 | 1.76 |
| A17h1_IgG4 | 1.23 | 0.67 | 2.33 | 0.85 | 1.97 | 1.84 |
| Name | CD8$^+$ Ki67$^+$ cells (%) | | | | | |
| | 100 nM | 10 nM | 1 nM | 0.1 nM | 0.01 nM | 0.001 nM |
| PEG-IL-2v | 87.3 | 43 | 1.54 | 0.29 | 0.59 | 0.22 |
| IgG-V91T | 55.9 | 3.46 | 0.63 | 0.99 | 0.83 | 0.59 |
| A-PC | 87.6 | 50.3 | 3.91 | 1.4 | 1.2 | 0 |
| A-V91T-02 | 79.3 | 9.11 | 1.89 | 2.25 | 0.65 | 1.29 |
| A-D20A | 1.34 | 1.23 | 0.75 | 0.33 | 0.24 | 0.71 |
| A-D20N | 1.9 | 0.58 | 0.6 | 0.87 | 0.48 | 0.42 |
| A-N88R | 0.6 | 1.71 | 1.34 | 0.93 | 1.27 | 0.38 |
| A-N88D | 2.21 | 1.12 | 1.76 | 1.5 | 1.44 | 0.83 |
| A-Q126D | 1.47 | 0.88 | 0.34 | 0.77 | 1 | 0.34 |
| A17h1_IgG4 | 0.32 | 0.12 | 0.55 | 0.57 | 0.54 | 0.49 |

Table 14. The proliferation activity of PD-1-IL-2v fusion proteins carrying different IL-2 mutations on T cells stimulated with a CD3 antibody

| Name | CD4$^+$ Ki67$^+$ cells (%) | | | | | |
|---|---|---|---|---|---|---|
| | 100 nM | 10 nM | 1 nM | 0.1 nM | 0.01 nM | 0.001 nM |
| PEG-IL-2v | 74.7 | 73 | 63 | 43.3 | 34.8 | 32.9 |
| IgG-V91T | 73.8 | 63 | 45.3 | 32.5 | 31.7 | 32.4 |
| A-PC | 76.4 | 74.4 | 67.2 | 57.2 | 40.4 | 34.3 |
| A-V91T-02 | 75.6 | 70.5 | 60.2 | 56.9 | 41.3 | 33.6 |
| A-D20A | 61.9 | 56.1 | 54.9 | 52.7 | 40.5 | 31.5 |
| A-D20N | 63.2 | 58.2 | 54.3 | 53.6 | 42.5 | 35.2 |
| A-N88R | 63.4 | 61 | 59 | 56.2 | 46.2 | 32.4 |
| A-N88D | 63.9 | 56.8 | 54.9 | 54 | 40.7 | 33.9 |
| A-Q126D | 63.1 | 58.9 | 55.8 | 53.5 | 38.4 | 34.8 |
| A17h1_IgG4 | 31.7 | 28.8 | 33.3 | 30.1 | 29.7 | 31.7 |

(continued)

| Name | CD8+ Ki67+ cells (%) | | | | | |
|---|---|---|---|---|---|---|
| | 100 nM | 10 nM | 1 nM | 0.1 nM | 0.01 nM | 0.001 nM |
| PEG-IL-2v | 82.5 | 82 | 76.6 | 67.1 | 55.5 | 55.5 |
| IgG-V91T | 83.3 | 78.2 | 69 | 53.5 | 54.4 | 54.3 |
| A-PC | 82.6 | 82.5 | 79 | 70.2 | 57.9 | 55.9 |
| A-V91T-02 | 82.4 | 79.9 | 73.9 | 67.9 | 58.1 | 55 |
| A-D20A | 75.9 | 68.2 | 67.7 | 65 | 57.1 | 54.3 |
| A-D20N | 75.6 | 68.5 | 64.7 | 66 | 61.2 | 56.3 |
| A-N88R | 75.6 | 69.4 | 68.2 | 66.6 | 60.9 | 49.9 |
| A-N88D | 76.8 | 69.3 | 66.3 | 64.8 | 57.4 | 55.5 |
| A-Q126D | 75.2 | 69.4 | 65.8 | 65.9 | 54.2 | 58.4 |
| A17h1_IgG4 | 56 | 48.5 | 57.4 | 49.8 | 50 | 53.8 |

**Example 7. Identification of Binding Avidities of PD-1-IL2v for Antigens PD-1 and IL-2R$\alpha$ or IL-2R$\beta$/$\gamma$**

The binding avidities of the PD-1-IL-2v fusion proteins containing different IL-2 mutants described above for PD-1 and human IL-2R$\alpha$ or IL-2R$\beta$/$\gamma$ were determined using surface plasmon resonance (SPR). The experiment was conducted using a Biacore 8K instrument (GE Healthcare). A CM5 sensor chip was used, and an HBS-EP+ buffer solution (10 mM HEPES, 150 mM NaCl, 3 mM EDTA, 0.05% surfactant P20) was used as the mobile phase. A 25 $\mu$g/mL solution of an anti-human IgG (Fc) antibody was prepared with a 10 mM sodium acetate buffer (pH 5.0), and the Immobilization program was selected to automatically perform anti-human IgG (Fc) antibody channel amino coupling immobilization. The test PD-1-IL2v fusion proteins were each diluted in the HBS-EP+ buffer solution as ligands and captured using the anti-human IgG (Fc) antibody on the chip channels. Human PD-1 antigen protein (Sino Biological, 10377-H08H), human IL-2R$\alpha$ receptor protein (Sino Biological, 10165-H08H), or human IL-2R$\beta$/$\gamma$ receptor heterodimer protein (Acrosystem, ILG-H5283) were used as analytes and diluted in the HBS-EP+ buffer solution. The analytes were serially diluted 2-fold from 200 nM to obtain a total of 7 concentration points. The dilutions were left to flow through the experimental and reference channels at a flow rate of 30 $\mu$L/min. The association time was 140 s, and the dissociation time was 600 s. 10 mM glycine (pH 1.5) (regeneration buffer, GE Healthcare, 29238268-AA) was run at a flow rate of 10 $\mu$L/min for 30 s. The association rate Ka, the dissociation rate Kd, and the dissociation constant (i.e., avidity $K_D$ value) were calculated. The results are shown below.

Table 15. The SPR binding avidities of PD-1-IL2v for human PD-1 antigen

| Antibody No. | ka (1/Ms) | Kd (1/s) | $K_D$ (M) |
|---|---|---|---|
| A-PC | 9.37E+04 | 1.82E-03 | 1.94E-08 |
| A-N88D | 9.98E+04 | 1.75E-03 | 1.76E-08 |
| A-D20A | 9.06E+04 | 1.68E-03 | 1.85E-08 |

Table 16. The SPR binding avidities of fusion proteins of an anti-PD-1 antibody and IL-2 mutants for human IL-2R$\alpha$

| Antibody No. | ka (1/Ms) | Kd (1/s) | $K_D$ (M) |
|---|---|---|---|
| A-PC | NB | NB | NB |
| A-N88D | NB | NB | NB |
| A-D20A | NB | NB | NB |
| (NB: no binding) | | | |

Table 17. The SPR binding avidities of fusion proteins of an anti-PD-1 antibody and IL-2 mutants for human IL-2Rβ/γ

| Antibody No. | ka (1/Ms) | Kd (1/s) | $K_D$ (M) |
|---|---|---|---|
| A-PC | 8.81E+04 | 2.23E-05 | 2.53E-10 |
| A-N88D | 6.37E+04 | 2.15E-04 | 3.38E-09 |
| A-D20A | 4.27E+04 | 6.81E-04 | 1.60E-08 |

The results of binding to human PD-1 antigen show that A-PC, A-N88D, and A-D20A exhibited similar abilities to bind to human PD-1, and their abilities were similar to the ability of A17m0902_hIgG4 to bind to human PD-1 (Table 8). The results of binding to human IL-2Rα (Table 16) show that A-PC, A-N88D, and A-D20A did not bind to human IL-2Rα. The results of binding to human IL-2Rβ/γ (Table 17) show that A-PC exhibited the strongest avidity for human IL-2Rβ/γ, and the avidities of A-N88D and A-D20A for human IL-2Rβ/γ were about 13 times lower and about 63 times lower, respectively.
The development of formulations of PD-1-IL2v (A-N88D, whose Knob and Hole chains are set forth in SEQ ID NO: 42 and SEQ ID NO: 43, respectively) was described below.

### Example 8. Screening for pH Values for PD-1-IL2v Formulations

According to the formula information shown in Table 18, liquid formulations of A-N88D (i.e., the "protein" in the following formulas) were prepared. After aseptic filling, the samples were left to stand at 40 °C for 1 W (week), 2 W, and 4 W, subjected to 5 freeze-thaw cycles (-35 °C/room temperature), and shaken for 3 days (300 rpm, 25 °C). Then, the appearance, SEC, NR-CE, and iCIEF were investigated. The screening results are shown in Tables 18 and 19.

Table 18. Formulas for pH screening

| Formula No. | Buffer | pH | Protein concentration (mg/mL) | Auxiliary materials | Particle size (nm) |
|---|---|---|---|---|---|
| F1 | 20 mM Acetic acid-histidine | 4.5 | 10 | 8% sucrose + 0.04% PS80 | 8.22 |
| F2 | | 5.0 | 10 | | 8.94 |
| F3 | | 5.5 | 10 | | 11.25 |
| F4 | | 5.0 | 10 | 6% sucrose + 30 mM arginine + 0.04% PS80 | 14.27 |

Table 19. pH screening - appearance and purity results

| No. | Condition | Appearance | SEC(%) Monomer | NR-CE(%) Main peak | iCIEF(%) Acidic peak | iCIEF(%) Main peak | iCIEF(%) Basic peak |
|---|---|---|---|---|---|---|---|
| F1 | T0 | Colorless, clear, no particles | 98.15 | 96.64 | 38.15 | 60.97 | 0.88 |
| | Shaking for 3D | Colorless, clear, no particles | 97.87 | 96.53 | 38.62 | 59.77 | 1.59 |
| | 5 freeze-thaw cycles | Colorless, clear, no particles | 98.16 | 96.19 | 38.33 | 60.83 | 0.83 |
| | 40 °C-1 W | Colorless, clear, no particles | 99.39 | 96.07 | 41.59 | 54.18 | 4.23 |
| | 40 °C-2 W | Colorless, clear, no particles | 98.98 | 93.83 | 47.99 | 47.71 | 4.30 |
| | 40 °C-4 W | Colorless, clear, a very small number of particles | 97.47 | 93.83 | 55.78 | 38.00 | 6.22 |

(continued)

| No. | Condition | Appearance | SEC(%) | NR-CE(%) | iCIEF(%) | | |
| --- | --- | --- | --- | --- | --- | --- | --- |
| | | | Monomer | Main peak | Acidic peak | Main peak | Basic peak |
| F2 | T0 | Colorless, clear, no particles | 97.89 | 96.79 | 38.38 | 60.36 | 1.26 |
| | Shaking for 3D | Colorless, clear, no particles | 97.36 | 96.78 | 40.11 | 58.17 | 1.72 |
| | 5 freeze-thaw cycles | Colorless, clear, no particles | 97.94 | 96.34 | 38.59 | 60.55 | 0.87 |
| | 40 °C-1 W | Colorless, clear, no particles | 97.30 | 96.12 | 41.79 | 55.22 | 2.99 |
| | 40 °C-2 W | Colorless, clear, no particles | 97.06 | 94.26 | 48.54 | 48.54 | 2.92 |
| | 40 °C-4 W | Colorless, clear, a very small number of particles | 96.90 | 93.85 | 56.53 | 38.97 | 4.50 |
| F3 | T0 | Colorless, clear, no particles | 97.72 | 96.76 | 38.61 | 59.49 | 1.89 |
| | Shaking for 3D | Colorless, clear, a small number of particles | 95.90 | 96.81 | 40.26 | 57.04 | 2.69 |
| | 5 freeze-thaw cycles | Colorless, clear, a large number of particles | 97.86 | 96.18 | 39.02 | 60.22 | 0.77 |
| | 40 °C-1 W | Colorless, clear, no particles | 95.77 | 96.35 | 42.88 | 54.64 | 2.48 |
| | 40 °C-2 W | Colorless, clear, no particles | 95.63 | 94.33 | 49.78 | 47.53 | 2.69 |
| | 40 °C-4 W | Colorless, clear, a small number of particles | 94.57 | 93.98 | 56.78 | 39.47 | 3.75 |
| F4 | T0 | Colorless, clear, no particles | 97.78 | 96.50 | 38.24 | 60.95 | 0.81 |
| | Shaking for 3D | Colorless, clear, a small number of particles | 96.84 | 96.56 | 38.69 | 57.88 | 3.43 |
| | 5 freeze-thaw cycles | Colorless, clear, a large number of particles | 97.94 | 96.23 | 39.17 | 59.80 | 1.03 |
| | 40 °C-1 W | Colorless, clear, no particles | 96.54 | 95.84 | 43.65 | 53.73 | 2.61 |
| | 40 °C-2 W | Colorless, clear, no particles | 96.44 | 94.54 | 49.12 | 47.94 | 2.94 |
| | 40 °C-4 W | Colorless, clear, a small number of particles | 96.52 | 93.17 | 57.73 | 38.50 | 3.77 |

Both T0 (Table 18) pH and protein concentration results were within the target ranges. The particle size results show that formulas F1 and F2 exhibited relatively small particle sizes, suggesting that the immunoconjugate has relatively good stability under the formula conditions.

The appearance results (Table 19) show that after 3 days of shaking and 5 repeated freeze-thaw cycles, particles were found in formulas F3 (pH 5.5) and F4 (pH 5.0, containing 30 mM arginine), and no visible particles were found in the other formulas. Similarly, after 4 W of standing at 40 °C, a small number of particles were found in formulas F3 (pH 5.5) and F4 (pH 5.0, containing 30 mM arginine), and a very small number of particles were found in formulas F1 (pH 4.5) and F2 (pH 5.0). This indicates that in the 20 mM acetic acid-histidine buffer system, the stability of the samples at pH 5.5 was relatively poor, and that the addition of 30 mM arginine at pH 5.0 reduced the stability of the samples.

The SEC (Table 19) results show that after 3 days of shaking, the SEC monomer purity of each formula slightly decreased, and that the decrease in monomer purity became more significant as the pH increased. After 5 repeated freeze-thaw cycles, there was no significant decrease in the SEC monomer purity of each formula. The NR-CE (Table 19) results show that after 3 days of shaking and 5 repeated freeze-thaw cycles, there was no significant decrease in the NR-CE main peak purity of each formula. The iCIEF (Table 19) results show that after 3 days of shaking and 5 repeated freeze-thaw cycles, there was no significant decrease in the iCIEF main peak purity of each formula.

In summary, PD-1-IL2v has relatively good stability at pH 4.5 to pH 5.0.

**Example 9. Screening for Buffer Systems and Protein Concentrations for PD-1-IL2v Formulations**

The stability of formulas with different protein concentrations was investigated at pH 5.0. In addition, the effects of 3 different buffer systems (20 mM acetic acid-histidine, pH 5.0; 35 mM acetic acid, pH 5.5; and 20 mM histidine-histidine hydrochloride, pH 5.0) on protein stability were compared. Considering that after formulas F1-F4 were shaken for 3 days, the SEC monomer purity of each formula slightly decreased, the PS80 concentration was increased in this round of screening. Samples were prepared according to the formulas shown in Table 20, and the prepared samples were sterilized by passing through a 0.22 μm PES filter membrane, left to stand at a high temperature of 40 °C for 1 W, 2 W, 3 W, and 4 W, subjected to 5 freeze-thaw cycles (-35 °C/room temperature), and shaken for 3 days (300 rpm, 25 °C). Then, the appearance, SEC, and NR-CE were investigated. The results are shown in Table 21.

Table 20. Formulas for buffer system and protein concentration screening

| Formula No. | Buffer | pH | Protein concentration (mg/mL) | Auxiliary materials |
|---|---|---|---|---|
| F5 | 20 mM acetic acid-histidine | 5.0 | 20 | 8% sucrose + 0.08% PS80 |
| F6 | 20 mM acetic acid-histidine | 5.0 | 50 | 8% sucrose + 0.08% PS80 |
| F7 | 20 mM acetic acid-histidine | 5.0 | 110 | 8% sucrose + 0.08% PS80 |
| F8 | 20 mM acetic acid-histidine | 4.8 | 50 | 8% sucrose + 0.08% PS80 |
| F9 | 35 mM acetic acid | 5.5 | 50 | 5% sucrose + 30 mM arginine + 0.08% PS80 |
| F10 | 20 mM histidine-histidine hydrochloride | 5.0 | 50 | 8% sucrose + 0.08% PS80 |

Table 21. Appearance and purity results of buffer system and protein concentration screening

| Formula No. | Condition | Appearance | SEC(%) Monomer | NR-CE(%) Main peak |
|---|---|---|---|---|
| F5 | T0 | Colorless, clear, no particles | 90.88 | 93.08 |
| | Shaking for 3D | Colorless, clear, no particles | 90.91 | 92.74 |
| | 5 freeze-thaw cycles | Colorless, clear, no particles | 91.46 | 92.19 |
| | 40 °C-1W | Colorless, clear, no particles | 91.10 | 91.66 |
| | 40 °C-2W | Colorless, clear, no particles | 90.42 | 92.53 |
| | 40 °C-3W | Colorless, slightly opalescent, no particles | 90.87 | 91.21 |
| | 40 °C-4W | Colorless, opalescent, a very small number of particles | 88.13 | 89.96 |
| F6 | T0 | Colorless, slightly opalescent, no particles | 91.10 | 92.89 |
| | Shaking for 3D | Colorless, slightly opalescent, no particles | 90.48 | 92.49 |
| | 5 freeze-thaw cycles | Colorless, slightly opalescent, no particles | 91.02 | 92.40 |
| | 40 °C-1W | Colorless, slightly opalescent, no particles | 90.04 | 92.62 |
| | 40 °C-2W | Colorless, slightly opalescent, no particles | 88.75 | 92.63 |
| | 40 °C-3W | Colorless, increased opalescence, no particles | 87.82 | 90.64 |
| | 40 °C-4W | Colorless, opalescent, no particles | 81.92 | 91.81 |

(continued)

| Formula No. | Condition | Appearance | SEC(%) Monomer | NR-CE(%) Main peak |
|---|---|---|---|---|
| F7 | T0 | Pale yellow, slightly opalescent, no particles | 90.57 | 93.00 |
| | Shaking for 3D | Pale yellow, slightly opalescent, no particles | 89.64 | 92.46 |
| | 5 freeze-thaw cycles | Pale yellow, slightly opalescent, no particles | 90.60 | 92.25 |
| | 40 °C-1W | Pale yellow, slightly opalescent, no particles | 87.56 | 93.07 |
| | 40 °C-2W | Pale yellow, slightly opalescent, no particles | 83.68 | 92.94 |
| | 40 °C-3W | Pale yellow, increased opalescence, no particles | 80.92 | 89.64 |
| | 40 °C-4W | Pale yellow, opalescent, no particles | 73.59 | 90.97 |
| F8 | T0 | Colorless, slightly opalescent, no particles | 89.13 | 92.80 |
| | Shaking for 3D | Colorless, slightly opalescent, no particles | 91.41 | 92.50 |
| | 5 freeze-thaw cycles | Colorless, slightly opalescent, no particles | 91.40 | 92.34 |
| | 40 °C-1W | Colorless, slightly opalescent, no particles | 91.43 | 92.56 |
| | 40 °C-2W | Colorless, slightly opalescent, no particles | 89.77 | 92.45 |
| | 40 °C-3W | Colorless, increased opalescence, no particles | 88.25 | 90.46 |
| | 40 °C-4W | Colorless, opalescent, no particles | 84.09 | 89.99 |
| F9 | T0 | Colorless, slightly opalescent, no particles | 90.45 | 93.07 |
| | Shaking for 3D | Colorless, slightly opalescent, no particles | 90.11 | 92.36 |
| | 5 freeze-thaw cycles | Colorless, slightly opalescent, no particles | 90.56 | 92.08 |
| | 40 °C-1W | Colorless, opalescent (increased opalescence), a large number of particles | 88.07 | 92.41 |
| | 40 °C-2W | Colorless, opalescent (increased opalescence), a large number of particles | 84.99 | 92.96 |
| | 40 °C-3W | Colorless, significantly opalescent, a large number of particles | 84.08 | 92.11 |
| | 40 °C-4W | Colorless, significantly opalescent, a large number of particles | 79.11 | 90.32 |
| F10 | T0 | Colorless, slightly opalescent, no particles | 90.88 | 93.06 |
| | Shaking for 3D | Colorless, slightly opalescent, no particles | 90.42 | 92.40 |
| | 5 freeze-thaw cycles | Colorless, slightly opalescent, no particles | 90.80 | 92.06 |
| | 40 °C-1W | Colorless, slightly opalescent, no particles | 90.28 | 92.86 |
| | 40 °C-2W | Colorless, slightly opalescent, no particles | 87.29 | 92.68 |
| | 40 °C-3W | Colorless, increased opalescence, no particles | 86.14 | 90.85 |
| | 40 °C-4W | Colorless, opalescent, no particles | 80.56 | 92.85 |

The appearance results (Table 21) show that after 3 days of shaking and 5 repeated freeze-thaw cycles, there was no significant change in the appearance of each formula. From the condition of standing at 40 °C for 1W onward, a large number of particles were found in formula F9 (pH 5.5), indicating that the protein has poor stability at pH 5.5.

The SEC (Table 21) results show that after 3 days of shaking and 5 repeated freeze-thaw cycles, there was no significant change in the SEC monomer purity of each formula. The NR-CE (Table 21) results show that after 3 days of shaking and 5 repeated freeze-thaw cycles, there was no significant change in the NR-CE main peak content of each formula.

**EP 4 763 867 A1**

**Example 10. Screening for Concentrations and Auxiliary Materials for Freeze-Dried Formulas of PD-1-IL2v**

To further improve the stability and protein concentration, freeze-dried formulations of PD-1-IL2v were developed. Samples were prepared according to the formulas in Table 22, and the prepared samples were passed through a 0.22 μm PES filter membrane, aseptically filled into vials, and freeze-dried.

Table 22. Information about freeze-dried formulas

| Formula No. | Buffer | pH | Protein concentration (mg/mL) | Auxiliary materials | Filling amount |
|---|---|---|---|---|---|
| F11 | 5 mM succinic acid-histidine | 5.0 | 80 | 8% sucrose + 0.015% SDS | 1.7 mL |
| F12 | | | 80 | 8% sucrose + 0.1% PS80 | 1.7 mL |
| F13 | | | 80 | 8% sucrose + 0.1% PS80 + 100 mM arginine hydrochloride | 1.7 mL |
| F14 | | | 40 | 4% sucrose + 0.1% PS80 | 3.4 mL |

The freeze-dried products were reconstituted by adding 1.5 mL of water. The appearances, reconstitution times, and appearances after reconstitution of the freeze-dried products are shown in Table 23.

Table 23. Freeze-drying results

| No. | Appearance after freeze-drying | Appearance after reconstitution |
|---|---|---|
| F11 | White cake-like porous solid | Colorless, clear, a large number of particles |
| F12 | White cake-like porous solid | Colorless, clear, a large number of fine particles |
| F13 | Shrunk cake | Colorless, clear, no particles |
| F14 | White cake-like porous solid | Colorless, clear, no particles |
| *Note: "/" means no detection. | | |

The reconstitution time results show that the reconstitution times of the high-concentration formulas F11-F13 were all relatively long, and the reconstitution time of formula F14 was relatively short. The reconstituted formulation of formula F14 contained the following components: 10 mM succinic acid-histidine, pH 5.0, 80 mg/mL PD-1-IL2v, 8% (w/v) sucrose, and 0.2% (w/v) PS80.

**Example 11. Screening for Concentrations for Freeze-Dried Formulations of PD-1-IL2v**

To study the effects of different protein concentrations on the reconstitution times of freeze-dried products, samples were prepared according to the formulas in Table 24-1, and the prepared samples were filtered, aseptically filled, and freeze-dried.

Table 24-1. Freeze-dried formulas

| Formula No. | Buffer | pH | Protein concentration (mg/mL) | Auxiliary materials | Filling amount |
|---|---|---|---|---|---|
| F15 | 5 mM succinic acid-histidine | 4.8 | 40 | 4% sucrose + 0.1% PS80 | 3.6 mL |
| F16 | | | 50 | | |
| F17 | | | 60 | | |

After freeze-drying, 1.5 mL of water was added for reconstitution. Information on the reconstituted solutions is shown in Table 24-2.

62

Table 24-2. Reconstituted solutions

| No. | Buffer | pH | Protein concentration (mg/mL) | Auxiliary materials |
|---|---|---|---|---|
| F15 | 10 mM succinic acid-histidine | 4.8 | 80 | 8% sucrose + 0.2% PS80 |
| F16 | | | 100 | |
| F17 | | | 120 | |

The reconstitution time results show that the reconstitution time of F15 was relatively short, and the reconstitution times of F16 and F17 were relatively long. The appearances of the freeze-dried products met the quality standard (white porous solid).

**Example 12. Screening for pH Values and Auxiliary Materials for Freeze-Dried Formulas of PD-1-IL2v**

The appearances of formulas with different pH values and different auxiliary materials after reconstitution were further investigated. Samples were prepared according to the formulas in Table 25, and the prepared samples were passed through a 0.22 $\mu$m PES filter membrane, aseptically filled, and freeze-dried. All the freeze-dried samples were reconstituted by adding 1.0 mL of water. The reconstitution results are shown in Table 25. The freeze-dried formulas prepared using sucrose and trehalose all had relatively good appearances after reconstitution.

Table 25. Freeze-dried formulas and appearances after reconstitution

| Formula No. | Buffer | pH | Protein concentration (mg/mL) | Auxiliary materials | Filling amount | Appearance after reconstitution |
|---|---|---|---|---|---|---|
| F21 | 10 mM suc-cinic acid-histidine | 4.0 | 40 | 8% trehalose + 0.08% PS80 + 50 mM arginine hydrochloride | 1.2 mL | No particles |
| F22 | | 4.0 | | 8% trehalose + 0.08% PS80 | | No particles |
| F23 | | 5.0 | | 8% trehalose + 0.08% PS80 | | A small number of particles |
| F24 | | 4.0 | | 5% trehalose + 0.08% PS80 | | No particles |
| F25 | | 4.0 | | 8% trehalose + 0.04% PS80 | | No particles |
| F26 | | 4.5 | | 8% trehalose + 0.08% PS80 | | No particles |

The appearance results after reconstitution in Table 25 show that a small number of particles were still present in formula F23 (pH 5.0) after reconstitution, and no particles were found in the other formulas. It can be seen that the pH of the formulas had a relatively big influence on their appearances after reconstitution, and different protective agent concentrations had little influence on the appearances of the freeze-dried products after reconstitution.

**Example 13. Investigation of Stability of Freeze-Dried Formulas of PD-1-IL-2v**

The following sample was prepared: 10 mM succinic acid-histidine, pH 4.5, 40 mg/mL PD-1-IL-2v, 4.4% (w/v) trehalose, and 0.04% (w/v) PS80. The filling amount was 3.64 mL.

The prepared sample was passed through a 0.22 $\mu$m PES filter membrane, aseptically filled, freeze-dried, and then reconstituted. The reconstitution volume was 1.6 mL. The reconstituted solution contained 20 mM succinic acid-histidine, pH 4.5, 80 mg/mL PD-1-IL-2v, 8.8% (w/v) trehalose, and 0.08% (w/v) PS80.

The results show that the freeze-dried product was relatively good in terms of appearance, reconstitution time, appearance after reconstitution, insoluble particle and water contents after reconstitution, as expected. The freeze-dried product exhibited relatively good stability after 4 W of standing at 40 °C. Neither the SEC monomer purity nor the NR-CE main peak purity significantly changed.

**Example 14. Formulas of PD-1-IL2v Formulations**

This example provides the following formulas:

1) 5 mM succinic acid-histidine, pH 4.5, 50 mg/mL PD-1-IL-2v, 4% (w/v) trehalose, and 0.04% (w/v) PS80;

2) 8 mM succinic acid-histidine, pH 4.5, 40 mg/mL PD-1-IL-2v, 4.4% (w/v) trehalose, and 0.04% (w/v) PS80;

3) 10 mM succinic acid-histidine, pH 4.5, 40 mg/mL PD-1-IL-2v, 5% (w/v) trehalose, and 0.04% (w/v) PS80;

4) 10 mM succinic acid-histidine, pH 4.0, 40 mg/mL PD-1-IL-2v, 4.4% (w/v) trehalose, and 0.04% (w/v) PS80;

5) 10 mM succinic acid-histidine, pH 4.0, 40 mg/mL PD-1-IL-2v, 2% (w/v) trehalose, and 0.05% (w/v) PS80;

6) 10 mM succinic acid-histidine, pH 4.5, 40 mg/mL PD-1-IL-2v, 4.4% (w/v) trehalose, and 0.08% (w/v) PS80;

7) 10 mM succinic acid-histidine, pH 4.5, 30 mg/mL PD-1-IL-2v, 5% (w/v) trehalose, and 0.04% (w/v) PS80;

8) 10 mM succinic acid-histidine, pH 4.5, 40 mg/mL PD-1-IL-2v, 5% (w/v) trehalose, and 0.04% (w/v) PS80;

9) 10 mM succinic acid-histidine, pH 4.5, 50 mg/mL PD-1-IL-2v, 5% (w/v) trehalose, and 0.04% (w/v) PS80;

10) 10 mM succinic acid-histidine, pH 4.5, 30 mg/mL PD-1-IL-2v, 4.4% (w/v) trehalose, and 0.04% (w/v) PS80;

11) 10 mM succinic acid-histidine, pH 4.5, 50 mg/mL PD-1-IL-2v, 4.4% (w/v) trehalose, and 0.04% (w/v) PS80;

12) 8 mM succinic acid-histidine, pH 4.5, 30 mg/mL PD-1-IL-2v, 5% (w/v) trehalose, and 0.02% (w/v) PS80; and

13) 10 mM succinic acid-histidine, pH 5.0, 40 mg/mL PD-1-IL-2v, 4.4% (w/v) trehalose, and 0.04% (w/v) PS80.

The above samples were freeze-dried and then reconstituted to obtain the following formulas:

14) 10 mM succinic acid-histidine, pH 4.5, 100 mg/mL PD-1-IL-2v, 8% (w/v) trehalose, and 0.08% (w/v) PS80;

15) 16 mM succinic acid-histidine, pH 4.5, 80 mg/mL PD-1-IL-2v, 8.8% (w/v) trehalose, and 0.08% (w/v) PS80;

16) 20 mM succinic acid-histidine, pH 4.5, 80 mg/mL PD-1-IL-2v, 10% (w/v) trehalose, and 0.08% (w/v) PS80;

17) 20 mM succinic acid-histidine, pH 4.0, 80 mg/mL PD-1-IL-2v, 8.8% (w/v) trehalose, and 0.08% (w/v) PS80;

18) 20 mM succinic acid-histidine, pH 4.0, 80 mg/mL PD-1-IL-2v, 4% (w/v) trehalose, and 0.1% (w/v) PS80;

19) 20 mM succinic acid-histidine, pH 4.5, 80 mg/mL PD-1-IL-2v, 8.8% (w/v) trehalose, and 0.08% (w/v) PS80;

20) 20 mM succinic acid-histidine, pH 4.5, 80 mg/mL PD-1-IL-2v, 10% (w/v) trehalose, and 0.08% (w/v) PS80;

21) 20 mM succinic acid-histidine, pH 4.5, 80 mg/mL PD-1-IL-2v, 10% (w/v) trehalose, and 0.08% (w/v) PS80;

22) 20 mM succinic acid-histidine, pH 4.5, 100 mg/mL PD-1-IL-2v, 10% (w/v) trehalose, and 0.08% (w/v) PS80;

23) 20 mM succinic acid-histidine, pH 4.5, 60 mg/mL PD-1-IL-2v, 8.8% (w/v) trehalose, and 0.08% (w/v) PS80;

24) 20 mM succinic acid-histidine, pH 4.5, 100 mg/mL PD-1-IL-2v, 8.8% (w/v) trehalose, and 0.08% (w/v) PS80;

25) 16 mM succinic acid-histidine, pH 4.5, 60 mg/mL PD-1-IL-2v, 10% (w/v) trehalose, and 0.04% (w/v) PS80; and

26) 20 mM succinic acid-histidine, pH 5.0, 80 mg/mL PD-1-IL-2v, 8.8% (w/v) trehalose, and 0.08% (w/v) PS80.

## Claims

1. A pharmaceutical composition, comprising an immunoconjugate and a buffer, wherein the immunoconjugate comprises a PD-1-binding domain and a cytokine, and the buffer is a histidine salt buffer;

   preferably, the buffer is one or more selected from the group consisting of acetic acid-histidine, succinic acid-histidine, and histidine-histidine hydrochloride;

   the PD-1-binding domain comprises an immunoglobulin single variable domain comprising a CDR1, a CDR2, and a CDR3 from the amino acid sequence set forth in any one of SEQ ID NOs: 2 and 8-15, or the amino acid sequences of the CDR1, the CDR2, and the CDR3 of the immunoglobulin single variable domain are set forth in SEQ ID NOs: 3, 16, and 17, respectively; the CDRs are defined according to the Kabat, IMGT, Chothia, AbM, or Contact numbering scheme; preferably, the CDR1, the CDR2, and the CDR3 of the immunoglobulin single variable domain are set forth in SEQ ID NOs: 3, 18, and 19, respectively,

   set forth in SEQ ID NOs: 3, 4, and 5, respectively, or

   SEQ ID NOs: 3, 4, and 20, respectively.

2. The pharmaceutical composition according to claim 1, wherein the buffer or the pharmaceutical composition has a pH of about 4.0 to about 6.5, preferably about 4.0 to about 5.0, and more preferably about 4.0 to about 4.5.

3. The pharmaceutical composition according to claim 1 or 2, wherein the buffer is at a concentration of about 1 mM to about 100 mM, preferably about 5 mM to about 35 mM, and more preferably about 10 mM to about 20 mM.

4. The pharmaceutical composition according to any one of claims 1 to 3, wherein the immunoconjugate is at a concentration of about 0.01 mg/mL to about 200 mg/mL, preferably about 10 mg/mL to about 120 mg/mL, and more preferably about 40 mg/mL to about 80 mg/mL.

5. The pharmaceutical composition according to any one of claims 1 to 4, further comprising a surfactant, wherein

preferably, the surfactant is sodium dodecyl sulfate (SDS), poloxamer, or polysorbate; more preferably, the surfactant is polysorbate 80.

6. The pharmaceutical composition according to claim 5, wherein the surfactant is at a concentration of about 0.001% (w/v) to about 1.0% (w/v), preferably about 0.04% (w/v) to about 0.2% (w/v), and preferably about 0.04% (w/v) to about 0.08% (w/v).

7. The pharmaceutical composition according to any one of claims 1 to 6, further comprising an osmotic pressure regulator, wherein

preferably, the osmotic pressure regulator is one or more selected from the group consisting of sucrose, trehalose, sorbitol, arginine, proline, glycine, mannitol, and sodium chloride;
preferably, the osmotic pressure regulator is selected from the group consisting of sucrose and/or trehalose; more preferably, the osmotic pressure regulator is sucrose or trehalose;
optionally, the pharmaceutical composition further comprises a stabilizer selected from the group consisting of arginine and salts thereof; preferably, the stabilizer is arginine hydrochloride.

8. The pharmaceutical composition according to claim 7, wherein the osmotic pressure regulator is at a concentration of about 1% (w/v) to about 20% (w/v), preferably about 1% (w/v) to about 10% (w/v), and preferably about 4% (w/v) to about 8.8% (w/v);
optionally, the stabilizer is at a concentration of about 5 mM to about 200 mM, preferably about 30 mM to about 100 mM.

9. The pharmaceutical composition according to any one of claims 1-8, comprising:

(a) about 10 mg/mL to about 200 mg/mL immunoconjugate,

about 1 mM to about 50 mM histidine salt buffer,
about 0.01% (w/v) to about 0.2% (w/v) surfactant,
about 1% (w/v) to about 20% (w/v) osmotic pressure regulator, and
about 10 mM to about 100 mM stabilizer, which is optionally present,
the pharmaceutical composition having a pH of about 4.0 to about 5.5; or

(b) about 10 mg/mL to about 120 mg/mL immunoconjugate,

about 5 mM to about 35 mM histidine salt buffer,
about 0.04% (w/v) to about 0.2% (w/v) polysorbate 80,
about 4% (w/v) to about 10% (w/v) sucrose or trehalose, and
about 30 mM to about 100 mM arginine or arginine hydrochloride, which is optionally present,
the pharmaceutical composition having a pH of about 4.0 to about 5.5; or

(c) about 10 mg/mL to about 120 mg/mL immunoconjugate,

about 5 mM to about 35 mM histidine-histidine hydrochloride, acetic acid-histidine, or succinic acid-histidine,
about 0.04% (w/v) to about 0.2% (w/v) polysorbate 80, and
about 4% (w/v) to about 10% (w/v) sucrose or trehalose,
the pharmaceutical composition having a pH of about 4.0 to about 5.0; or

(d) about 10 mg/mL to about 120 mg/mL immunoconjugate,

about 5 mM to about 20 mM acetic acid-histidine or succinic acid-histidine,
about 0.04% (w/v) to about 0.2% (w/v) polysorbate 80, and
about 1% (w/v) to about 10% (w/v) sucrose or trehalose,
the pharmaceutical composition having a pH of about 4.0 to about 5.0; or

(e) about 10 mg/mL to about 120 mg/mL immunoconjugate,

about 5 mM to about 20 mM succinic acid-histidine,
about 0.04% (w/v) to about 0.08% (w/v) polysorbate 80,
about 1% (w/v) to about 10% (w/v) trehalose, and
about 10 mM to about 100 mM arginine hydrochloride, which is optionally present,
the pharmaceutical composition having a pH of about 4.0 to about 4.5; or

(e-1) about 10 mg/mL to about 80 mg/mL immunoconjugate,

about 5 mM to about 10 mM succinic acid-histidine,
about 0.04% (w/v) to about 0.08% (w/v) polysorbate 80,
about 4% (w/v) to about 10% (w/v) trehalose, and
about 10 mM to about 100 mM arginine hydrochloride, which is optionally present,
the pharmaceutical composition having a pH of about 4.0 to about 4.5; or

(e-2) about 10 mg/mL to about 40 mg/mL immunoconjugate,

about 10 mM succinic acid-histidine,
about 0.04% (w/v) to about 0.08% (w/v) polysorbate 80, and
about 4% (w/v) to about 8.8% (w/v) trehalose,
the pharmaceutical composition having a pH of about 4.0 to about 4.5; or

(e-3) about 40 mg/mL to about 120 mg/mL immunoconjugate,

about 10 mM to about 20 mM succinic acid-histidine,
about 0.04% (w/v) to about 0.08% (w/v) polysorbate 80, and
about 4% (w/v) to about 10% (w/v) trehalose,
the pharmaceutical composition having a pH of about 4.0 to about 4.5; or

(e-4) about 40 mg/mL to about 80 mg/mL immunoconjugate,

about 20 mM succinic acid-histidine,
about 0.04% (w/v) to about 0.08% (w/v) polysorbate 80, and
about 4.4% (w/v) to about 8.8% (w/v) trehalose,
the pharmaceutical composition having a pH of about 4.0 to about 4.5; or

(f) about 10 mg/mL to about 120 mg/mL immunoconjugate,

about 5 mM to about 20 mM succinic acid-histidine,
about 0.1% (w/v) to about 0.2% (w/v) polysorbate 80,
about 4% (w/v) to about 8% (w/v) sucrose, and
about 10 mM to about 100 mM arginine hydrochloride, which is optionally present,
the pharmaceutical composition having a pH of about 4.5 to about 5.0; or

(f-1) about 10 mg/mL to about 80 mg/mL immunoconjugate,

about 5 mM to about 10 mM succinic acid-histidine,
about 0.1% (w/v) to about 0.2% (w/v) polysorbate 80,
about 4% (w/v) to about 8% (w/v) sucrose, and
about 10 mM to about 100 mM arginine hydrochloride, which is optionally present,
the pharmaceutical composition having a pH of about 4.5 to about 5.0; or

(f-2) about 40 mg/mL to about 80 mg/mL immunoconjugate,

about 5 mM succinic acid-histidine,
about 0.1% (w/v) to about 0.2% (w/v) polysorbate 80, and
about 4% (w/v) to about 8% (w/v) sucrose,
the pharmaceutical composition having a pH of about 4.5 to about 5.0; or

(f-3) about 40 mg/mL to about 120 mg/mL immunoconjugate,

about 10 mM to about 20 mM succinic acid-histidine,
about 0.1% (w/v) to about 0.2% (w/v) polysorbate 80, and
about 4% (w/v) to about 8% (w/v) sucrose,
the pharmaceutical composition having a pH of about 4.5 to about 5.0; or

(f-4) about 80 mg/mL to about 120 mg/mL immunoconjugate,

about 10 mM succinic acid-histidine,
about 0.1% (w/v) to about 0.2% (w/v) polysorbate 80, and
about 4% (w/v) to about 8% (w/v) sucrose,
the pharmaceutical composition having a pH of about 4.5 to about 5.0; or

(g) about 10 mg/mL to about 120 mg/mL immunoconjugate,

about 5 mM to about 20 mM acetic acid-histidine or histidine-histidine hydrochloride,
about 0.04% (w/v) to about 0.08% (w/v) polysorbate 80, and
about 4% (w/v) to about 8% (w/v) sucrose,
the pharmaceutical composition having a pH of about 4.5 to about 5.0.

10. The pharmaceutical composition according to any one of claims 1-9, comprising any one of the following:

(1) about 10 mg/mL to about 120 mg/mL immunoconjugate,

about 10 mM succinic acid-histidine,
about 0.04% (w/v) polysorbate 80, and
about 4.4% (w/v) trehalose,
the pharmaceutical composition having a pH of about 4.0 to about 4.5;

(2) about 10 mg/mL to about 80 mg/mL immunoconjugate,

about 10 mM succinic acid-histidine,
about 0.04% (w/v) polysorbate 80, and
about 4.4% (w/v) trehalose,
the pharmaceutical composition having a pH of about 4.0 to about 4.5;

(3) about 10 mg/mL to about 40 mg/mL immunoconjugate,

about 10 mM succinic acid-histidine,
about 0.04% (w/v) polysorbate 80, and
about 4.4% (w/v) trehalose,
the pharmaceutical composition having a pH of about 4.5;

(4) about 10 mg/mL to about 120 mg/mL immunoconjugate,

about 20 mM succinic acid-histidine,
about 0.08% (w/v) polysorbate 80, and
about 8.8% (w/v) trehalose,
the pharmaceutical composition having a pH of about 4.0 to about 4.5;

(5) about 40 mg/mL to about 120 mg/mL immunoconjugate,

about 20 mM succinic acid-histidine,
about 0.08% (w/v) polysorbate 80, and
about 8.8% (w/v) trehalose,
the pharmaceutical composition having a pH of about 4.0 to about 4.5; and

(6) about 40 mg/mL to about 80 mg/mL immunoconjugate,

   about 20 mM succinic acid-histidine,
   about 0.08% (w/v) polysorbate 80, and
   about 8.8% (w/v) trehalose,
   the pharmaceutical composition having a pH of about 4.5.

11. The pharmaceutical composition according to any one of claims 1-10, wherein the PD-1-binding domain comprises the amino acid sequence set forth in any one of SEQ ID NOs: 2 and 8-15 or an amino acid sequence having at least 90% sequence identity thereto; preferably, the PD-1-binding domain comprises the amino acid sequence set forth in SEQ ID NO: 13 or an amino acid sequence having at least 90% sequence identity thereto.

12. The pharmaceutical composition according to any one of claims 1-11, wherein the cytokine is wild-type IL-2 or a variant thereof; preferably, the IL-2 variant comprises the following mutations:

   42A/72G/88D,
   42A/72G/88R,
   42A/72G/20A,
   42A/72G/20N,
   42A/72G/91T,
   42A/72G/126D,
   42A/72G/16A/84S,
   42A/72G/26Q/29S/71Q,
   38E/42A/91T,
   42A/72G/88D/26Q/29S/71Q,
   42A/72G/88R/26Q/29S/71Q,
   42A/72G/20A/26Q/29S/71Q,
   42A/72G/20N/26Q/29S/71Q,
   42A/72G/91T/26Q/29S/71Q,
   42A/72G/126D/26Q/29S/71Q, or
   42A/72G/16A/84S/26Q/29S/71Q;
   preferably, the IL-2 variant comprises the following mutations:

   F42A/L72G/N88D,
   F42A/L72G/N88R,
   F42A/L72G/D20A,
   F42A/L72G/D20N,
   F42A/L72G/V91T,
   F42A/L72G/Q126D,
   F42A/L72G/H16A/D84S,
   F42A/L72G/N26Q/N29S/N71Q,
   R38E/F42A/V91T,
   F42A/L72G/N88D/N26Q/N29S/N71Q,
   F42A/L72G/N88R/N26Q/N29S/N71Q,
   F42A/L72G/D20A/N26Q/N29S/N71Q,
   F42A/L72G/D20N/N26Q/N29S/N71Q,
   F42A/L72G/V91T/N26Q/N29S/N71Q,
   F42A/L72G/Q126D/N26Q/N29S/N71Q, or
   F42A/L72G/H16A/D84S/N26Q/N29S/N71Q.

13. The pharmaceutical composition according to claim 12, wherein the IL-2 variant comprises a mutation at position 3 and/or a mutation at position 125; the mutation at position 3 is preferably T3A, and the mutation at position 125 is preferably C125A or C125S.

14. The pharmaceutical composition according to claim 12 or 13, wherein the IL-2 variant comprises or has the amino acid sequence set forth in any one of SEQ ID NOs: 23-31 or a sequence having at least 90% identity thereto.

15. The pharmaceutical composition according to any one of claims 1-14, wherein the immunoconjugate further comprises an immunoglobulin Fc region; preferably, the Fc region is an Fc region of human IgG1, IgG2, or IgG4;

preferably, the Fc region of human IgG1 comprises one or more amino acid mutations that reduce binding to Fcγ receptors (FcγRs) and/or reduce ADCC effects;
more preferably, the Fc region of human IgG1 comprises a mutation of L234, L235, or P329, or any combination thereof;
most preferably, the Fc region of human IgG1 comprises mutations L234A/L235A or L234A/L235A/P329G;
the mutations in the Fc region are numbered according to the EU numbering scheme.

16. The pharmaceutical composition according to claim 15, wherein the Fc region comprises mutations that promote association of a first subunit and a second subunit of the Fc region;

preferably, an amino acid residue in a CH3 region of the first subunit of the Fc region is replaced with an amino acid residue having a larger side-chain volume, thereby forming a protuberance within the CH3 region of the first subunit that is positionable in a cavity within a CH3 region of the second subunit, and an amino acid residue in the CH3 region of the second subunit of the Fc region is replaced with an amino acid residue having a smaller side-chain volume, thereby forming a cavity within the CH3 region of the second subunit in which the protuberance within the CH3 region of the first subunit is positionable;
preferably, the first subunit of the Fc region comprises mutation T366W, and the second subunit comprises a mutation selected from the group consisting of T366S, L368A, and Y407V, and any combination thereof; the first subunit of the Fc region comprises mutation S354C or E356C, and the second subunit comprises a mutation selected from the group consisting of Y349C; or the first subunit of the Fc region comprises mutations S354C/T366W, and the second subunit comprises mutations Y349C/T366S/L368A/Y407V;
preferably, the second subunit of the Fc region further comprises mutation(s) H435R/Y436F, H435R, or H435K.

17. The pharmaceutical composition according to any one of claims 15-16, wherein the PD-1-binding domain is located at the N-terminus of the Fc region, and the cytokine is located at the N-terminus or C-terminus of the Fc region; preferably, the cytokine is located in a polypeptide chain where the first subunit of the Fc region is present.

18. The pharmaceutical composition according to any one of claims 1-17, wherein the immunoconjugate comprises the following structure:

1) a first polypeptide chain comprises [PD-1-binding domain 1]-[the first subunit of the Fc region]-[linker 1]a-[IL-2 or the variant thereof], from the N-terminus to the C-terminus, and
2) a second polypeptide chain comprises [PD-1-binding domain 1]-[the second subunit of the Fc region], from the N-terminus to the C-terminus,

wherein a is selected from the group consisting of 0 and 1, and the linker has a structure of $(G_xS)_y$, wherein x is selected from the group consisting of integers from 1 to 5, and y is selected from the group consisting of integers from 1 to 6;
preferably, x is 4, and y is 1, 2, or 3.

19. The pharmaceutical composition according to any one of claims 1-18, wherein the immunoconjugate comprises a polypeptide chain combination of the following amino acid sequences:

SEQ ID NOs: 42 and 43,
SEQ ID NOs: 21 and 22,
SEQ ID NOs: 36 and 37,
SEQ ID NOs: 38 and 39, or
SEQ ID NOs: 40 and 41.

20. A freeze-dried formulation, wherein the freeze-dried formulation is obtained by freeze-drying the pharmaceutical composition according to any one of claims 1 to 19, or the freeze-dried formulation is reconstitutable to form the pharmaceutical composition according to any one of claims 1 to 19.

21. A freeze-dried formulation, wherein the freeze-dried formulation is obtained by freeze-drying a liquid formulation comprising a pharmaceutical composition, and the liquid formulation comprises:

(e-1) about 10 mg/mL to about 80 mg/mL immunoconjugate,

about 5 mM to about 10 mM succinic acid-histidine,
about 0.04% (w/v) to about 0.08% (w/v) polysorbate 80,
about 4% (w/v) to about 10% (w/v) trehalose, and
about 10 mM to about 100 mM arginine hydrochloride, which is optionally present,
the pharmaceutical composition having a pH of about 4.0 to about 4.5; or

(e-2) about 10 mg/mL to about 40 mg/mL immunoconjugate,

about 10 mM succinic acid-histidine,
about 0.04% (w/v) to about 0.08% (w/v) polysorbate 80, and
about 4% (w/v) to about 8.8% (w/v) trehalose,
the pharmaceutical composition having a pH of about 4.0 to about 4.5; or

(f-1) about 10 mg/mL to about 80 mg/mL immunoconjugate,

about 5 mM to about 10 mM succinic acid-histidine,
about 0.1% (w/v) to about 0.2% (w/v) polysorbate 80,
about 4% (w/v) to about 8% (w/v) sucrose, and
about 10 mM to about 100 mM arginine hydrochloride, which is optionally present,
the pharmaceutical composition having a pH of about 4.5 to about 5.0; or

(f-2) about 40 mg/mL to about 80 mg/mL immunoconjugate,

about 5 mM succinic acid-histidine,
about 0.1% (w/v) to about 0.2% (w/v) polysorbate 80, and
about 4% (w/v) to about 8% (w/v) sucrose,
the pharmaceutical composition having a pH of about 4.5 to about 5.0; or

preferably any one of the following:

(1) about 10 mg/mL to about 120 mg/mL immunoconjugate,

about 10 mM succinic acid-histidine,
about 0.04% (w/v) polysorbate 80, and
about 4.4% (w/v) trehalose,
the pharmaceutical composition having a pH of about 4.0 to about 4.5;

(2) about 10 mg/mL to about 80 mg/mL immunoconjugate,

about 10 mM succinic acid-histidine,
about 0.04% (w/v) polysorbate 80, and
about 4.4% (w/v) trehalose,
the pharmaceutical composition having a pH of about 4.0 to about 4.5; and

(3) about 10 mg/mL to about 40 mg/mL immunoconjugate,

about 10 mM succinic acid-histidine,
about 0.04% (w/v) polysorbate 80, and
about 4.4% (w/v) trehalose,
the pharmaceutical composition having a pH of about 4.5;
preferably, the immunoconjugate comprises a PD-1-binding domain and a cytokine, wherein the PD-1-binding domain comprises an immunoglobulin single variable domain comprising a CDR1, a CDR2, and a CDR3 from the amino acid sequence set forth in any one of SEQ ID NOs: 2 and 8-15, or the amino acid sequences of the CDR1, the CDR2, and the CDR3 of the immunoglobulin single variable domain are set forth in SEQ ID NOs: 3, 16, and 17, respectively; the CDRs are defined according to the Kabat, IMGT, Chothia, AbM, or Contact numbering scheme;

preferably, the CDR1, the CDR2, and the CDR3 of the immunoglobulin single variable domain are set forth in SEQ ID NOs: 3, 18, and 19, respectively,

set forth in SEQ ID NOs: 3, 4, and 5, respectively, or

SEQ ID NOs: 3, 4, and 20, respectively;

more preferably, the immunoconjugate comprises a polypeptide chain combination of the following amino acid sequences:

SEQ ID NOs: 42 and 43,

SEQ ID NOs: 21 and 22,

SEQ ID NOs: 36 and 37,

SEQ ID NOs: 38 and 39, or

SEQ ID NOs: 40 and 41.

22. A reconstituted solution, wherein the reconstituted solution is prepared by reconstituting the freeze-dried formulation according to claim 20 or 21;

preferably, a concentration of the immunoconjugate in the reconstituted solution is greater than a concentration of the immunoconjugate in a stock solution for preparing the freeze-dried formulation;

preferably, the ratio of the concentration of the immunoconjugate in the reconstituted solution to the concentration of the immunoconjugate in the stock solution is (1-5):1; more preferably, the ratio is (1-2):1.

23. The reconstituted solution according to claim 22, wherein the reconstituted solution is obtained by reconstituting the freeze-dried formulation according to claim 20 or 21 with a solvent; preferably, the solvent is preferably water, normal saline, or glucose.

24. The reconstituted solution according to claim 22 or 23, comprising:

(e-3) about 40 mg/mL to about 120 mg/mL immunoconjugate,

about 10 mM to about 20 mM succinic acid-histidine,

about 0.04% (w/v) to about 0.08% (w/v) polysorbate 80, and

about 4% (w/v) to about 10% (w/v) trehalose,

the pharmaceutical composition having a pH of about 4.0 to about 4.5; or

(e-4) about 40 mg/mL to about 80 mg/mL immunoconjugate,

about 20 mM succinic acid-histidine,

about 0.04% (w/v) to about 0.08% (w/v) polysorbate 80, and

about 4.4% (w/v) to about 8.8% (w/v) trehalose,

the pharmaceutical composition having a pH of about 4.0 to about 4.5; or

(f-3) about 40 mg/mL to about 120 mg/mL immunoconjugate,

about 10 mM to about 20 mM succinic acid-histidine,

about 0.1% (w/v) to about 0.2% (w/v) polysorbate 80, and

about 4% (w/v) to about 8% (w/v) sucrose,

the pharmaceutical composition having a pH of about 4.5 to about 5.0; or

(f-4) about 80 mg/mL to about 120 mg/mL immunoconjugate,

about 10 mM succinic acid-histidine,

about 0.1% (w/v) to about 0.2% (w/v) polysorbate 80, and

about 4% (w/v) to about 8% (w/v) sucrose,

the pharmaceutical composition having a pH of about 4.5 to about 5.0;

preferably any one of the following:

(4) about 10 mg/mL to about 120 mg/mL immunoconjugate,

about 20 mM succinic acid-histidine,
about 0.08% (w/v) polysorbate 80, and
about 8.8% (w/v) trehalose,
the pharmaceutical composition having a pH of about 4.0 to about 4.5;

(5) about 40 mg/mL to about 120 mg/mL immunoconjugate,

about 20 mM succinic acid-histidine,
about 0.08% (w/v) polysorbate 80, and
about 8.8% (w/v) trehalose,
the pharmaceutical composition having a pH of about 4.0 to about 4.5; and

(6) about 40 mg/mL to about 80 mg/mL immunoconjugate,

about 20 mM succinic acid-histidine,
about 0.08% (w/v) polysorbate 80, and
about 8.8% (w/v) trehalose,
the pharmaceutical composition having a pH of about 4.5;
wherein preferably, the PD-1-binding domain comprises at least one immunoglobulin single variable domain comprising a CDR1, a CDR2, and a CDR3 from the amino acid sequence set forth in any one of SEQ ID NOs: 2 and 8-15, or the amino acid sequences of the CDR1, the CDR2, and the CDR3 of the immunoglobulin single variable domain are set forth in SEQ ID NOs: 3, 16, and 17, respectively; the CDR1, the CDR2, and the CDR3 are defined according to the Kabat, IMGT, Chothia, AbM, or Contact numbering scheme;
preferably, the CDR1, the CDR2, and the CDR3 of the immunoglobulin single variable domain are set forth in SEQ ID NOs: 3, 18, and 19, respectively,
set forth in SEQ ID NOs: 3, 4, and 5, respectively, or
SEQ ID NOs: 3, 4, and 20, respectively;
preferably, the cytokine is IL-2 or a variant thereof; more preferably, the cytokine comprises the sequence set forth in any one of SEQ ID NOs: 23-31;
more preferably, the immunoconjugate comprises a polypeptide chain combination of the following amino acid sequences:

SEQ ID NOs: 42 and 43,
SEQ ID NOs: 21 and 22,
SEQ ID NOs: 36 and 37,
SEQ ID NOs: 38 and 39, or
SEQ ID NOs: 40 and 41.

**25.** A liquid formulation, comprising any one of the following:

(e) about 10 mg/mL to about 120 mg/mL immunoconjugate,

about 5 mM to about 20 mM succinic acid-histidine,
about 0.04% (w/v) to about 0.08% (w/v) polysorbate 80,
about 1% (w/v) to about 10% (w/v) trehalose, and
about 10 mM to about 100 mM arginine hydrochloride, which is optionally present,
the pharmaceutical composition having a pH of about 4.0 to about 4.5; or

(e-1) about 10 mg/mL to about 80 mg/mL immunoconjugate,

about 5 mM to about 10 mM succinic acid-histidine,
about 0.04% (w/v) to about 0.08% (w/v) polysorbate 80,
about 4% (w/v) to about 10% (w/v) trehalose, and
about 10 mM to about 100 mM arginine hydrochloride, which is optionally present,
the pharmaceutical composition having a pH of about 4.0 to about 4.5; or

(e-2) about 10 mg/mL to about 40 mg/mL immunoconjugate,

about 10 mM succinic acid-histidine,
about 0.04% (w/v) to about 0.08% (w/v) polysorbate 80, and
about 4% (w/v) to about 8.8% (w/v) trehalose,
the pharmaceutical composition having a pH of about 4.0 to about 4.5; or

(e-3) about 40 mg/mL to about 120 mg/mL immunoconjugate,

about 10 mM to about 20 mM succinic acid-histidine,
about 0.04% (w/v) to about 0.08% (w/v) polysorbate 80, and
about 4% (w/v) to about 10% (w/v) trehalose,
the pharmaceutical composition having a pH of about 4.0 to about 4.5; or

(e-4) about 40 mg/mL to about 80 mg/mL immunoconjugate,

about 20 mM succinic acid-histidine,
about 0.04% (w/v) to about 0.08% (w/v) polysorbate 80, and
about 4.4% (w/v) to about 8.8% (w/v) trehalose,
the pharmaceutical composition having a pH of about 4.0 to about 4.5; or

(f) about 10 mg/mL to about 120 mg/mL immunoconjugate,

about 5 mM to about 20 mM succinic acid-histidine,
about 0.1% (w/v) to about 0.2% (w/v) polysorbate 80,
about 4% (w/v) to about 8% (w/v) sucrose, and
about 10 mM to about 100 mM arginine hydrochloride, which is optionally present,
the pharmaceutical composition having a pH of about 4.5 to about 5.0; or

(f-1) about 10 mg/mL to about 80 mg/mL immunoconjugate,

about 5 mM to about 10 mM succinic acid-histidine,
about 0.1% (w/v) to about 0.2% (w/v) polysorbate 80,
about 4% (w/v) to about 8% (w/v) sucrose, and
about 10 mM to about 100 mM arginine hydrochloride, which is optionally present,
the pharmaceutical composition having a pH of about 4.5 to about 5.0; or

(f-2) about 40 mg/mL to about 80 mg/mL immunoconjugate,

about 5 mM succinic acid-histidine,
about 0.1% (w/v) to about 0.2% (w/v) polysorbate 80, and
about 4% (w/v) to about 8% (w/v) sucrose,
the pharmaceutical composition having a pH of about 4.5 to about 5.0; or

(f-3) about 40 mg/mL to about 120 mg/mL immunoconjugate,

about 10 mM to about 20 mM succinic acid-histidine,
about 0.1% (w/v) to about 0.2% (w/v) polysorbate 80, and
about 4% (w/v) to about 8% (w/v) sucrose,
the pharmaceutical composition having a pH of about 4.5 to about 5.0; or

(f-4) about 80 mg/mL to about 120 mg/mL immunoconjugate,

about 10 mM succinic acid-histidine,
about 0.1% (w/v) to about 0.2% (w/v) polysorbate 80, and
about 4% (w/v) to about 8% (w/v) sucrose,
the pharmaceutical composition having a pH of about 4.5 to about 5.0; or
preferably any one of the following:

(1) about 10 mg/mL to about 120 mg/mL immunoconjugate,

about 10 mM succinic acid-histidine,
about 0.04% (w/v) polysorbate 80, and
about 4.4% (w/v) trehalose,
the pharmaceutical composition having a pH of about 4.0 to about 4.5;

(2) about 10 mg/mL to about 80 mg/mL immunoconjugate,

about 10 mM succinic acid-histidine,
about 0.04% (w/v) polysorbate 80, and
about 4.4% (w/v) trehalose,
the pharmaceutical composition having a pH of about 4.0 to about 4.5;

(3) about 10 mg/mL to about 40 mg/mL immunoconjugate,

about 10 mM succinic acid-histidine,
about 0.04% (w/v) polysorbate 80, and
about 4.4% (w/v) trehalose,
the pharmaceutical composition having a pH of about 4.5;

(4) about 10 mg/mL to about 120 mg/mL immunoconjugate,

about 20 mM succinic acid-histidine,
about 0.08% (w/v) polysorbate 80, and
about 8.8% (w/v) trehalose,
the pharmaceutical composition having a pH of about 4.0 to about 4.5;

(5) about 40 mg/mL to about 120 mg/mL immunoconjugate,

about 20 mM succinic acid-histidine,
about 0.08% (w/v) polysorbate 80, and
about 8.8% (w/v) trehalose,
the pharmaceutical composition having a pH of about 4.0 to about 4.5;

(6) about 40 mg/mL to about 80 mg/mL immunoconjugate,

about 20 mM succinic acid-histidine,
about 0.08% (w/v) polysorbate 80, and
about 8.8% (w/v) trehalose,
the pharmaceutical composition having a pH of about 4.5;
wherein preferably, the PD-1-binding domain comprises an immunoglobulin single variable domain comprising a CDR1, a CDR2, and a CDR3 from the amino acid sequence set forth in any one of SEQ ID NOs: 2 and 8-15, or the amino acid sequences of the CDR1, the CDR2, and the CDR3 of the immunoglobulin single variable domain are set forth in SEQ ID NOs: 3, 16, and 17, respectively; the CDRs are defined according to the Kabat, IMGT, Chothia, AbM, or Contact numbering scheme; preferably, the CDR1, the CDR2, and the CDR3 of the immunoglobulin single variable domain are set forth in SEQ ID NOs: 3, 18, and 19, respectively,
set forth in SEQ ID NOs: 3, 4, and 5, respectively, or
SEQ ID NOs: 3, 4, and 20, respectively;
more preferably, the immunoconjugate comprises a polypeptide chain combination of the following amino acid sequences:

SEQ ID NOs: 42 and 43,
SEQ ID NOs: 21 and 22,
SEQ ID NOs: 36 and 37,
SEQ ID NOs: 38 and 39, or
SEQ ID NOs: 40 and 41.

26. The pharmaceutical composition according to any one of claims 1 to 19, the reconstituted solution according to any one of claims 22-24, or the liquid formulation according to claim 25, being an intravenous injection, a subcutaneous injection, an intraperitoneal injection, or an intramuscular injection, preferably an intravenous or subcutaneous injection.

27. A product, comprising a container, wherein the container contains the pharmaceutical composition according to any one of claims 1 to 19, the freeze-dried formulation according to claim 20 or 21, the reconstituted solution according to any one of claims 22-24, or the liquid formulation according to claim 25.

28. A method for treating or preventing a disease, comprising administering to a subject in need thereof a therapeutically or prophylactically effective amount of the pharmaceutical composition according to any one of claims 1 to 19, the freeze-dried formulation according to claim 20 or 21, the reconstituted solution according to any one of claims 22-24, the liquid formulation according to claim 25, or the product according to claim 27, wherein:

preferably, the disease is a cancer;
more preferably, the cancer is selected from the group consisting of lung cancer, prostate cancer, breast cancer, head and neck cancer, esophageal cancer, stomach/gastric cancer, colon cancer, colorectal cancer, bladder cancer, cervical cancer, uterine cancer, ovarian cancer, hepatocellular carcinoma, melanoma, renal cell carcinoma, squamous cell carcinoma, and hematological cancer.

29. A method for preparing a liquid formulation, comprising the following steps:

preparing a stock solution comprising the pharmaceutical composition according to any one of claims 1-19, and freeze-drying the stock solution to obtain a freeze-dried formulation, and
reconstituting the freeze-dried formulation to obtain the liquid formulation,
wherein a concentration of the immunoconjugate in the liquid formulation is greater than a concentration of the immunoconjugate in the stock solution;
preferably, the ratio of the concentration of the immunoconjugate in the liquid formulation to the concentration of the immunoconjugate in the stock solution is (1-5):1, preferably (1-2):1.

30. A method for preparing a pharmaceutical composition, comprising:

preparing a stock solution comprising the pharmaceutical composition according to any one of claims 1-19, and freeze-drying the stock solution to obtain a freeze-dried formulation, and
reconstituting the freeze-dried formulation with a solvent to obtain a liquid formulation,
wherein a concentration of the immunoconjugate in the liquid formulation is greater than a concentration of the immunoconjugate in the stock solution, and the solvent is preferably water;
preferably, the ratio of the concentration of the immunoconjugate in the liquid formulation to the concentration of the immunoconjugate in the stock solution is (1-5):1, preferably (1-2):1.

**DC cell:T cell mixed lymphocyte reaction**

FIG. 1

FIG. 2

## M07e cell proliferation

FIG. 3A

## M07e-hPD-1 cell proliferation

FIG. 3B

## M07e-hPD-1 cell proliferation

FIG. 4

FIG. 5A

FIG. 5B

**CD4+ T cell proliferation**

FIG. 6A

**CD8+ T cell proliferation**

FIG. 6B

## INTERNATIONAL SEARCH REPORT

| International application No. |
|---|
| **PCT/CN2024/112330** |

| A. | CLASSIFICATION OF SUBJECT MATTER |
|---|---|

C07K19/00(2006.01)i; C07K16/28(2006.01)i; C07K14/55(2006.01)i; C12N15/62(2006.01)i; C12N15/63(2006.01)i; A61P35/00(2006.01)i; A61K38/20(2006.01)i; A61K39/00(2006.01)i; A61K39/395(2006.01)i; C07K16/28(2006.01)i; A61K47/68(2017.01)i

According to International Patent Classification (IPC) or to both national classification and IPC

| B. | FIELDS SEARCHED |
|---|---|

Minimum documentation searched (classification system followed by classification symbols)

IPC: C07K, C12N, A61K, A61P

Documentation searched other than minimum documentation to the extent that such documents are included in the fields searched

Electronic data base consulted during the international search (name of data base and, where practicable, search terms used)

CNTXT, WPABSC, WPABS, ENTXTC, ENTXT, DPWI, VEN, CNKI, 万方数据, Wanfang Data, Web of Science, 百度学术, Baidu Scholar, HimmPat, incoPat, 读秀, DUXIU, 中国生物序列检索系统, China Biological Sequence Search System, NCBI, EBI, STNext: 申请人, 发明人, Applicants, Inventors, PD-1, 单域抗体, 纳米抗体, 重链抗体, IL-2, 突变, Fc, 组氨酸, 缓冲剂, 聚山梨酯, 蔗糖, 海藻糖, 精氨酸, single domain antibody, nanobody, heavy chain antibody, mutat+, histidine, buffers, polysorbate, sucrose, trehalose, arginine, 序列1-50, sequences 1-50

| C. | DOCUMENTS CONSIDERED TO BE RELEVANT |
|---|---|

| Category* | Citation of document, with indication, where appropriate, of the relevant passages | Relevant to claim No. |
|---|---|---|
| PY | WO 2023151661 A1 (JIANGSU HENGRUI PHARMACEUTICALS CO., LTD. et al.) 17 August 2023 (2023-08-17) description, page 3, lines 5-10, page 6, lines 14-35, page 8, lines 5-8, page 9, lines 8-20, page 10, lines 25-36, page 13, lines 17-24, page 14, lines 29-36, and page 15, lines 5-24, and sequences 3, 13-15, 17-35 and 88-92 | 1-30 |
| Y | CN 110392692 A (F. HOFFMANN-LA ROCHE AG) 29 October 2019 (2019-10-29) description, paragraphs 104, 110, 111, 127, 131, 132, 192-195, 230, 244, 296, 304, 311, 319, 320 and 336 | 1-30 |
| Y | WO 2023040945 A1 (JIANGSU HENGRUI PHARMACEUTICALS CO., LTD. et al.) 23 March 2023 (2023-03-23) description, page 3, lines 1-5 and 10-17, page 4, lines 12-34, page 15, lines 24-34, page 16, lines 24-35, and page 17, lines 20-33, and sequences 3, 13-15 and 17-35 | 1-30 |

✓ Further documents are listed in the continuation of Box C.    ✓ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **07 November 2024** | **12 November 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

# EP 4 763 867 A1

**INTERNATIONAL SEARCH REPORT**

| International application No. |
| --- |
| **PCT/CN2024/112330** |

## C. DOCUMENTS CONSIDERED TO BE RELEVANT

| Category* | Citation of document, with indication, where appropriate, of the relevant passages | Relevant to claim No. |
| --- | --- | --- |
| Y | CN 112105343 A (PFIZER INC.) 18 December 2020 (2020-12-18)<br>claims 1-3, 5-7, 11-15, 20 and 27-30, and description, paragraphs 6, 7, 10-13, 17, 20, 24, 30, 116 and 118 | 1-30 |
| Y | WO 2022174102 A1 (SYNTHORX, INC. et al.) 18 August 2022 (2022-08-18)<br>description, paragraphs 11, 12, 15, 28, 209, 213, 393-395, 401 and 422 | 1-30 |
| Y | WO 2022100686 A1 (JIANGSU HENGRUI MEDICINE CO., LTD. et al.) 19 May 2022 (2022-05-19)<br>description, page 2, lines 29-35, page 4, lines 19-29, page 5, lines 3-15, page 8, lines 5-27, and page 15, lines 1-20, and sequences 1-37 | 1-30 |
| Y | WO 2023036215 A1 (SHANGHAI QILU PHARMACEUTICAL RES AND DEVELOPMENT CENTRE LTD.) 16 March 2023 (2023-03-16)<br>description, page 1, lines 39-20, and page 8, lines 3-10 and 21-26, and sequence 32 | 15-18 |
| Y | CN 115867316 A (JIANGSU HENGRUI MEDICINE CO., LTD. et al.) 28 March 2023 (2023-03-28)<br>description, paragraphs 9, 22-25, 29-32, 63, 76-81, 90, 108 and 119 | 1-30 |
| A | CN 110505883 A (F. HOFFMANN-LA ROCHE AG) 26 November 2019 (2019-11-26)<br>claims 1-3, 9, 20, 28, 29 and 47, and description, paragraphs 14, 28, 31, 34, 37, 38 and 50 | 1-30 |

Form PCT/ISA/210 (second sheet) (July 2022)

**INTERNATIONAL SEARCH REPORT**

International application No.

**PCT/CN2024/112330**

| Box No. I | Nucleotide and/or amino acid sequence(s) (Continuation of item 1.c of the first sheet) |

1.  With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)),

          ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**

International application No.

**PCT/CN2024/112330**

**Box No. II**     **Observations where certain claims were found unsearchable (Continuation of item 2 of first sheet)**

This international search report has not been established in respect of certain claims under Article 17(2)(a) for the following reasons:

1. ☑  Claims Nos.: **28**
   because they relate to subject matter not required to be searched by this Authority, namely:

   Claim 28 relates to a method for treating diseases, and therefore does not comply with PCT Rule 39.1(iv). However, this search is made on the basis that the subject matter is amended to be a pharmaceutical use.

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

| International application No. |
|---|
| **PCT/CN2024/112330** |

| Patent document cited in search report | | | Publication date (day/month/year) | Patent family member(s) | | | Publication date (day/month/year) |
|---|---|---|---|---|---|---|---|
| WO | 2023151661 | A1 | 17 August 2023 | None | | | |
| CN | 110392692 | A | 29 October 2019 | PL | 3606946 | T3 | 28 November 2022 |
| | | | | TW | 201900220 | A | 01 January 2019 |
| | | | | TWI | 800506 | B | 01 May 2023 |
| | | | | DK | 3606946 | T3 | 24 October 2022 |
| | | | | LT | 3606946 | T | 25 October 2022 |
| | | | | MA | 49033 | A | 12 February 2020 |
| | | | | MA | 49033 | B1 | 31 October 2022 |
| | | | | CA | 3053357 | A1 | 11 October 2018 |
| | | | | JP | 2020515275 | A | 28 May 2020 |
| | | | | JP | 7426825 | B2 | 02 February 2024 |
| | | | | AR | 126987 | A2 | 06 December 2023 |
| | | | | MX | 2023003397 | A | 31 March 2023 |
| | | | | CL | 2019002346 | A1 | 10 January 2020 |
| | | | | JP | 2022062001 | A | 19 April 2022 |
| | | | | EP | 3606946 | A1 | 12 February 2020 |
| | | | | EP | 3606946 | B1 | 24 August 2022 |
| | | | | ES | 2928718 | T3 | 22 November 2022 |
| | | | | MY | 201482 | A | 26 February 2024 |
| | | | | HUE | 059885 | T2 | 28 January 2023 |
| | | | | PH | 12019502273 | A1 | 07 December 2020 |
| | | | | AU | 2018247765 | A1 | 22 August 2019 |
| | | | | AU | 2018247765 | B2 | 23 November 2023 |
| | | | | US | 2018326010 | A1 | 15 November 2018 |
| | | | | US | 11413331 | B2 | 16 August 2022 |
| | | | | KR | 20210124518 | A | 14 October 2021 |
| | | | | KR | 102461885 | B1 | 03 November 2022 |
| | | | | AR | 111400 | A1 | 10 July 2019 |
| | | | | EP | 4201953 | A1 | 28 June 2023 |
| | | | | KR | 20190121816 | A | 28 October 2019 |
| | | | | RS | 63663 | B1 | 30 November 2022 |
| | | | | PT | 3606946 | T | 17 October 2022 |
| | | | | ZA | 201905517 | B | 26 May 2021 |
| | | | | UA | 125700 | C2 | 18 May 2022 |
| | | | | RU | 2019134338 | A | 05 May 2021 |
| | | | | RU | 2019134338 | A3 | 07 December 2021 |
| | | | | CO | 2019009354 | A2 | 09 September 2019 |
| | | | | BR | 112019017329 | A2 | 14 April 2020 |
| | | | | IL | 269395 | A | 28 November 2019 |
| | | | | SG | 11201909205 | YA | 28 November 2019 |
| | | | | US | 2023134606 | A1 | 04 May 2023 |
| | | | | US | 12023368 | B2 | 02 July 2024 |
| | | | | HRP | 20221254 | T1 | 23 December 2022 |
| | | | | JP | 2024045143 | A | 02 April 2024 |
| | | | | MX | 2019011770 | A | 09 January 2020 |
| | | | | CR | 20190426 | A | 01 November 2019 |
| | | | | WO | 2018184964 | A1 | 11 October 2018 |
| | | | | PE | 20191494 | A1 | 21 October 2019 |
| WO | 2023040945 | A1 | 23 March 2023 | None | | | |
| CN | 112105343 | A | 18 December 2020 | EP | 3761954 | A1 | 13 January 2021 |

Form PCT/ISA/210 (patent family annex) (July 2022)

## INTERNATIONAL SEARCH REPORT
### Information on patent family members

International application No.

**PCT/CN2024/112330**

| Patent document cited in search report | | | Publication date (day/month/year) | Patent family member(s) | | | Publication date (day/month/year) |
|---|---|---|---|---|---|---|---|
| | | | | US | 2021002369 | A1 | 07 January 2021 |
| | | | | RU | 2020129226 | A3 | 07 April 2022 |
| | | | | CA | 3093036 | A1 | 12 September 2019 |
| | | | | JP | 2021517129 | A | 15 July 2021 |
| | | | | JP | 7312188 | B2 | 20 July 2023 |
| | | | | AU | 2019232625 | A1 | 17 September 2020 |
| | | | | KR | 20200128115 | A | 11 November 2020 |
| | | | | KR | 102594028 | B1 | 24 October 2023 |
| | | | | WO | 2019171253 | A1 | 12 September 2019 |
| | | | | WO | 2019171253 | A8 | 01 October 2020 |
| | | | | MX | 2020009275 | A | 08 January 2021 |
| | | | | IL | 277095 | A | 29 October 2020 |
| | | | | BR | 112020017935 | A2 | 09 February 2021 |
| WO | 2022174102 | A1 | 18 August 2022 | EP | 4291243 | A1 | 20 December 2023 |
| | | | | US | 2023381335 | A1 | 30 November 2023 |
| | | | | TW | 202302148 | A | 16 January 2023 |
| WO | 2022100686 | A1 | 19 May 2022 | TW | 202227122 | A | 16 July 2022 |
| WO | 2023036215 | A1 | 16 March 2023 | | None | | |
| CN | 115867316 | A | 28 March 2023 | JP | 2023535384 | A | 17 August 2023 |
| | | | | US | 2023295329 | A1 | 21 September 2023 |
| | | | | WO | 2022022660 | A1 | 03 February 2022 |
| | | | | EP | 4190353 | A1 | 07 June 2023 |
| | | | | EP | 4190353 | A4 | 14 February 2024 |
| | | | | TW | 202214298 | A | 16 April 2022 |
| | | | | KR | 20230044448 | A | 04 April 2023 |
| | | | | BR | 112023001471 | A2 | 14 March 2023 |
| | | | | MX | 2023001160 | A | 22 February 2023 |
| | | | | AU | 2021317805 | A1 | 16 March 2023 |
| | | | | CA | 3189452 | A1 | 03 February 2022 |
| CN | 110505883 | A | 26 November 2019 | WO | 2018189220 | A1 | 18 October 2018 |
| | | | | CA | 3058279 | A1 | 18 October 2018 |
| | | | | KR | 20190136076 | A | 09 December 2019 |
| | | | | AU | 2018250875 | A1 | 03 October 2019 |
| | | | | MX | 2019012187 | A | 25 November 2019 |
| | | | | BR | 112019021411 | A2 | 05 May 2020 |
| | | | | IL | 269558 | A | 28 November 2019 |
| | | | | EP | 3609537 | A1 | 19 February 2020 |
| | | | | JP | 2020516638 | A | 11 June 2020 |
| | | | | TW | 201902918 | A | 16 January 2019 |
| | | | | US | 2020188526 | A1 | 18 June 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2023110361039
- WO 2020125743 A
- WO 9627011 A
- WO 98050431 A
- EP 1870459 A
- WO 2007110205 A
- WO 2007147901 A
- WO 2009089004 A
- WO 2010129304 A
- WO 201190754 A
- WO 2011143545 A
- WO 2012058768 A
- WO 2013157954 A
- WO 2013096291 A
- WO 2012107417 A
- WO 2009040562 A
- WO 2010035012 A
- WO 2005003169 A
- WO 2005003170 A
- WO 2005003171 A
- WO 9222583 A
- WO 05113605 A
- WO 2006082515 A
- WO 2012130831 A

### Non-patent literature cited in the description

- **RILEY et al.** *Immunol. Rev.*, 2009, vol. 29, 114-25
- **CHEN et al.** *Nat. Rev. Immunol.*, 2013, vol. 13, 227-42
- **DONG et al.** *Nat. Med.*, 1999, vol. 5, 1365-9
- *J. biol. chem.*, 1968, vol. 243, 3558
- **HOLLIGER ; HUDSON.** *Nature Biotech.*, 2005, vol. 23 (9), 1126-1136
- **ADAIR ; LAWSON.** *Drug Design Reviews-Online*, 2005, vol. 2 (3), 209-217
- **VERMA et al.** *Journal of Immunological Methods*, 1998, vol. 216, 165-181
- **MCCOY et al.** *J Mol Biol*, 1997, vol. 268, 570-584
- **LEE et al.** *Protein Sci.*, 2001, vol. 10, 362-377
- **CHAU et al.** *J Comp Mol Des*, 1994, vol. 8, 51325
- **LAWRENCE et al.** *J Mol Biol*, 1993, vol. 234, 946-950
- **WALLS et al.** *J Mol Biol*, 1992, vol. 228, 277-297
- **SCHUELER-FURMAN et al.** *Proteins*, 2005, vol. 60, 187-194
- **JOHNSON ; WU.** *Nucleic Acids Res.*, 2000, vol. 28, 214-8
- **CHOTHIA et al.** *J. Mol. Biol.*, 1986, vol. 196, 901-17
- **CHOTHIA et al.** *Nature*, 1989, vol. 342, 877-83
- **MARTIN et al.** *Proc Natl Acad Sci (USA)*, 1989, vol. 86, 9268-9272
- **SAMUDRALA et al.** Ab Initio Protein Structure Prediction Using a Combined Hierarchical Approach. *PROTEINS, Structure, Function and Genetics Suppl.*, 1999, vol. 3, 194-198
- **MACCALLUM et al.** *J. Mol. Biol.*, 1996, vol. 5, 732-45
- **MAKABE et al.** *Journal of Biological Chemistry*, 2008, vol. 283, 1156-1166
- **HAMERS-CASTERMAN C ; ATARHOUCH T ; MUYLDERMANS S ; ROBINSON G ; HAMERS C ; SONGA EB ; BENDAHMAN N ; HAMERS R.** Naturally occurring antibodies devoid of light chains. *Nature*, 1993, vol. 363, 446-448
- **KABAT, E. A. et al.** Sequences of Proteins of Immunological Interest. 1991
- **MARKS et al.** *Biotechnology*, 1992, vol. 10, 779-783
- **BARBAS et al.** *Proc. Nat. Acad. Sci, USA*, 1994, vol. 91, 3809-3813
- **SHIER et al.** *Gene*, 1995, vol. 169, 147-155
- **YELTON et al.** *Immunol.*, 1995, vol. 155, 1994-2004
- **JACKSON et al.** *J. Immunol.*, 1995, vol. 154 (7), 3310-9
- **HAWKINS et al.** *J. Mol. Biol.*, 1992, vol. 226 (3), 889896
- **KS JOHNSON ; RE HAWKINS.** Affinity maturation of antibodies using phage display. Oxford University Press, 1996
- **CACECI et al.** *Byte*, 1984, vol. 9, 340-362
- **WONG ; LOHMAN.** *Proc. Natl. Acad. Sci. USA*, 1993, vol. 90, 5428-5432

- Antibodies: A Laboratory Manual and Molecular Cloning: A Laboratory Manual. Cold Spring Harbor Laboratory